# EUROPEAN PATENT APPLICATION

(11) **EP 3 839 050 A2**
(43) Date of publication of application: **23.06.2021**
(21) Application number: 20191981.8
(22) Date of filing: 15.03.2013
(51) Int. Cl.: C12N 15/85

(54) **NON-DISRUPTIVE GENE TARGETING**

(30) Priority: 18.04.2012 US 201261635203 P; 01.06.2012 US 201261654645 P
(62) Divisional of application: 13778431.0
(71) Applicant: The Board of Trustees of the Leland Stanford Junior University, Stanford, CA 94305-2038 (US); Board of Regents of the University of Texas System, Austin, TX 78701 (US)
(72) Inventor: KAY, Mark A., Los Altos, CA 94024 (US); PORTEUS, Matthew, Stanford, CA 94305 (US); BARKER, Jenny, Dallas, TX 75234 (US); CHECKETTS, Josh, Palo Alto, CA 94303 (US); VOIT, Richard, Palo Alto, CA 94306-1166 (US); BARZEL, Adi, Palo Alto, CA 94306 (US)
(74) Representative: Chapman, Desmond Mark

(57) **Abstract**

Compositions and methods are provided for integrating one or more genes of interest into cellular DNA without substantially disrupting the expression of the gene at the locus of integration, i.e., the target locus. These compositions and methods are useful in any in vitro or in vivo application in which it is desirable to express a gene of interest in the same spatially and temporally restricted pattern as that of a gene at a target locus while maintaining the expression of the gene at the target locus, for example, to treat disease, in the production of genetically modified organisms in agriculture, in the large scale production of proteins by cells for therapeutic, diagnostic, or research purposes, in the induction of iPS cells for therapeutic, diagnostic, or research purposes, in biological research, etc. Reagents, devices and kits thereof that find use in practicing the subject methods are also provided.

## Description

### FIELD OF THE INVENTION

This invention pertains to donor polynucleotide compositions for site-specific nucleic acid modification.

### CROSS REFERENCE TO RELATED APPLICATIONS

Pursuant to 35 U.S.C. § 119 (e), this application claims priority to the filing date of the United States Provisional Patent Application Serial No. 61/635,203, filed April 18, 2012 and United States Provisional Patent Application Serial No. 61/654,645, filed June 1, 2012; the disclosures of which are herein incorporated by reference.

### BACKGROUND OF THE INVENTION

Site-specific manipulation of the genome is a desirable goal for many applications in medicine, biotechnology, and biological research. In recent years much effort has been made to develop new technologies for gene targeting in mitotic and post mitotic cells. However, integration of a gene of interest into a target locus may disrupt expression of the gene at the target locus, producing unwanted effects on the cell. The present invention addresses these issues.

### SUMMARY OF THE INVENTION

Compositions and methods are provided for integrating one or more genes of interest into cellular DNA without substantially disrupting the expression of the gene at the locus of integration, i.e., the target locus. These compositions and methods are useful in any in vitro or in vivo application in which it is desirable to express a gene of interest in the same spatially and temporally restricted pattern as that of a gene at a target locus while maintaining the expression of the gene at the target locus, for example, to treat disease, in the production of genetically modified organisms in agriculture, in the large scale production of proteins by cells for therapeutic, diagnostic, or research purposes, in the induction of iPS cells for therapeutic, diagnostic, or research purposes, in biological research, etc. Reagents, devices and kits thereof that find use in practicing the subject methods are also provided.

In one aspect of the invention, a donor polynucleotide composition for expressing a gene of interest from a target locus in a cell without disrupting the expression of the gene at the target locus is provided. In some embodiments, the donor polynucleotide comprises a nucleic acid cassette comprising the gene of interest and at least one element selected from the group consisting of a 2A peptide, an internal ribosome entry site (IRES), an N-terminal intein splicing region and a C-terminal intein splicing region, a splice donor and a splice acceptor, and a coding sequence for the gene at the target locus; and sequences flanking the cassette that are homologous to sequences flanking an integration site in the target locus. In some embodiments, the cassette is configured such that the gene of interest is operably linked to the promoter at the target locus upon insertion into the target locus. In some embodiments, the cassette comprises a promoter operably linked to the gene of interest. In some embodiments, the cassette comprises two or more genes of interest.

In one aspect of the invention, a method is provided for expressing a gene of interest from a target locus in a cell without disrupting the expression of the gene at the target locus. In some embodiments, the method comprises contacting the cell with an effective amount of a donor polynucleotide, e.g., as described above or disclosed elsewhere herein. In some embodiments, the contacting occurs in the presence of one or more targeted nucleases. In some embodiments, the cell stably expresses the one or more targeted nucleases. In some embodiments, the method further comprises contacting the cell with the one or more targeted nucleases. In some embodiments, the one or more targeted nucleases is selected from the group consisting of a zinc finger nuclease, a TALEN, a homing endonuclease, or a targeted SPO11 nuclease. In some embodiments, the target locus is selected from the group consisting of actin, ADA, albumin, α-globin, β-globin, CD2, CD3, CD5, CD7, E1α, IL2RG, Ins1, Ins2, NCF1, p50, p65, PF4, PGC-γ, PTEN, TERT, UBC, and VWF. In some embodiments, the gene of interest is a therapeutic peptide or polypeptide, a selectable marker, or an imaging marker. In some embodiments, the cell is a mitotic cell. In other embodiments, the cell is a post-mitotic cell. In some embodiments, the cell is in vitro. In other embodiments, the cell is in vivo.

In one aspect of the invention, a method is provided for producing a gene modification in a cell in a subject, the gene modification comprising an insertion in a target DNA locus that does not disrupt the expression of the gene at the target locus. In some embodiments, the method comprises contacting a cell ex vivo with an effective amount of a donor polynucleotide, e.g., as described above or disclosed elsewhere herein, where the contacting occurs under conditions that are permissive for nonhomologous end joining or homologous recombination; and transplanting the cell into the subject.

In some embodiments, the method further comprises contacting the cells with a first targeted nuclease that is specific for a first nucleotide sequence within the target locus, and a second targeted nuclease that is specific for a second nucleotide sequence within the target locus. In some embodiments, the cell to be contacted is harvested from the subject. In some embodiments, the method further comprises selecting for the cells comprising the insertion prior to transplanting. In some embodiments, the method further comprises expanding the cells comprising the insertion prior to transplanting.

In one aspect of the invention, a method is provided for treating a wound in an individual. In some embodiments, the method comprises contacting a cell with an effective amount of donor polynucleotide comprising at least one wound healing growth factor gene, wherein the donor polynucleotide is configured to promote the integration of the wound healing growth factor into a target locus in the cell without disrupting the expression of the gene at the target locus. In some embodiments, the contacting occurs in vitro, and the method further comprises transplanting the cell into the individual. In other embodiments, the contacting occurs in vivo.

In some embodiments, the cell is a fibroblast. In some embodiments, the fibroblast is autologous. In some embodiments, the fibroblast is induced from a pluripotent stem cell. In some embodiments, the fibroblast is a universal fibroblast. In some embodiments, the wound healing growth factor gene is selected from the group consisting of PDGF, VEGF, EGF, TGFα, TGBβ, FGF, TNF, IL-1, IL-2, IL-6, IL-8, and endothelium derived growth factor. In certain embodiments, the target locus is the adenosine deaminase gene (ADA) locus. In some such embodiments, the donor polynucleotide promotes the integration into the ADA locus at exon 1. In certain such embodiments, the cells are contacted with a first targeted nuclease that is specific for a first nucleotide sequence within the ADA locus, and a second targeted nuclease that is specific for a second nucleotide sequence within the ADA locus.

In some embodiments, the first targeted nuclease and the second targeted nuclease are TALENs. In some embodiments, the donor polynucleotide further comprises a suicide gene. In some embodiments, the suicide gene is the TK gene, inducible caspase 9, or CD20. In some embodiments, the suicide gene is under the control of a constitutively acting promoter. In other embodiments, the suicide gene is under the control of an inducible promoter.

In one aspect of the invention, a method is provided for treating or protecting against a nervous system condition in an individual. In some embodiments, the method comprises contacting a cell with an effective amount of donor polynucleotide comprising at least one neuroprotective factor, wherein the donor polynucleotide is configured to promote the integration of the neuroprotection factor into a target locus in the cell without disrupting the expression of the gene at the target locus. In some embodiments, the contacting occurs in vitro, and the method further comprises transplanting the cell into the individual. In other embodiments, the contacting occurs in vivo. In some embodiments, the cell is an astrocyte, an oligodendrocyte, a Schwann cell, or a neuron. In some embodiments, the cell is a neuron, and the target locus is the NF locus, the NSE locus, the NeuN locus, or the MAP2 locus. In some embodiments, the cell is an astrocyte, and the target locus is the GFAP locus or S100B locus. In some embodiments, the cell is an oligodendrocyte or Schwann cell, and the target locus is the GALC locus or MBP locus. In some embodiments, the cell is autologous. In some embodiments, the cell is induced from a pluripotent stem cell. In some embodiments, the neuroprotective factor is selected from the group consisting of a neurotrophin, Kifap3, Bcl-xl, Crmp1, Chkβ, CALM2, Caly, NPG11, NPT1, Eef1a1, Dhps, Cd151, Morf412, CTGF, LDH-A, Atl1, NPT2, Ehd3, Cox5b, Tubal a, γ-actin, Rpsa, NPG3, NPG4, NPG5, NPG6, NPG7, NPG8, NPG9, and NPG10.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures.

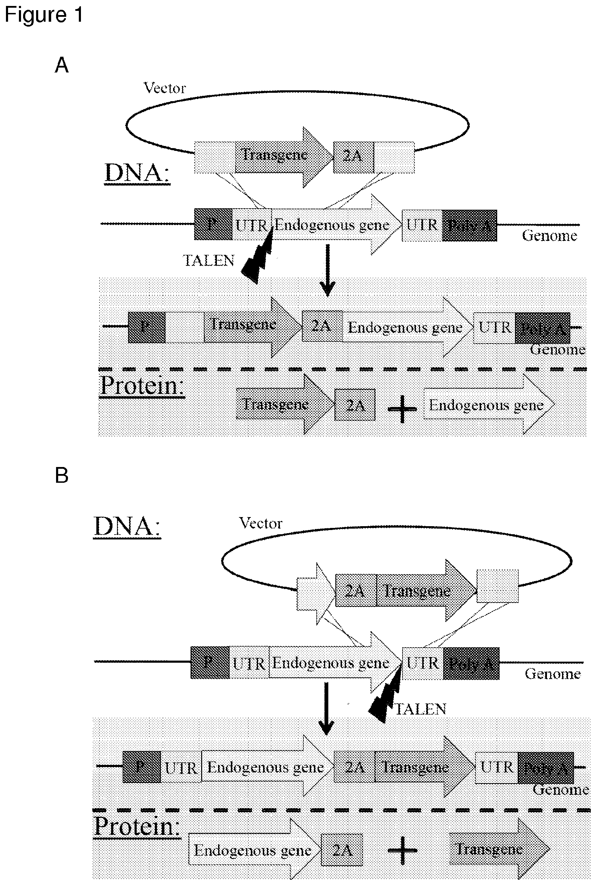

**Figure 1** depicts targeted integration without gene disruption using 2A peptides. A gene of interest ("transgene" in green) is inserted into the target locus such that it is operably linked to the promoter of the gene at the target locus ("endogenous gene" in blue). (A) The transgene cassette that is inserted comprises a 2A peptide downstream of the transgene. This configuration provides for transgene insertion immediately downstream of the 5' untranslated region (UTR) and start codon of the gene at the target locus without disrupting the transcription or translation of the endogenous gene downstream of the insertion site. (B) The transgene cassette that is inserted comprises a 2A peptide upstream of the transgene. This configuration provides for transgene insertion immediately upstream of the 3' untranslated region and stop codon of the gene at the target locus. P, endogenous gene promoter; UTR, endogenous gene untranslated region; PolyA, polyadenylation sequence; 2A, 2A peptide. The use of the targeted nuclease TALEN is optional.

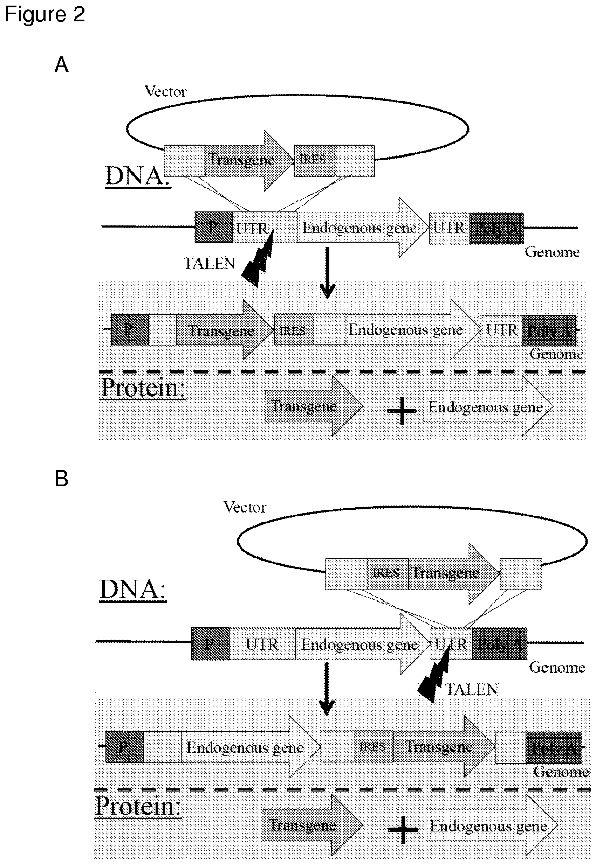

**Figure 2** depicts targeted integration without gene disruption using an IRES. (A) The transgene cassette that is inserted comprises a sequence encoding an IRES downstream of the transgene. This configuration provides for transgene insertion within the 5' untranslated region (UTR) of the gene at the target locus without disrupting the transcription or translation of the endogenous gene sequence downstream of the insertion site. (B) The transgene cassette that is inserted comprises a sequence encoding an IRES upstream of the transgene. This configuration provides for transgene insertion within the 3' UTR of the gene at the target locus without disrupting the transcription or translation of the endogenous gene sequence upstream of the insertion site. P, endogenous gene promoter; UTR, endogenous gene untranslated region; PolyA, polyadenylation sequence; IRES, internal ribosomal entry sequence. The use of the targeted nuclease TALEN is optional.

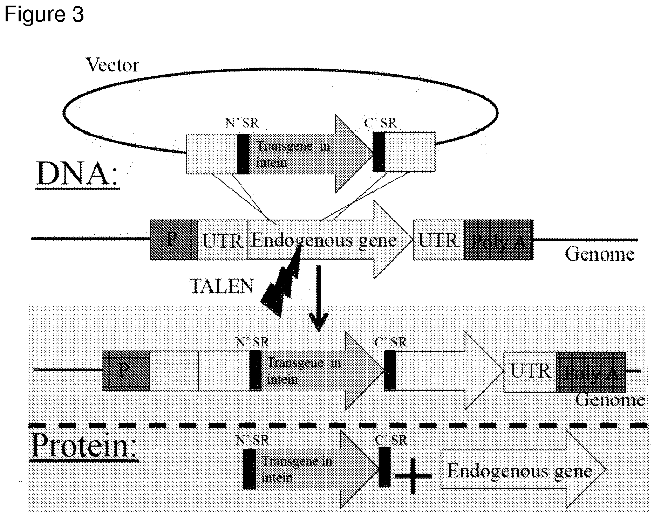

**Figure 3** depicts targeted integration without gene disruption using an intein configuration. The transgene cassette comprises an intein N-terminal splicing region and an intein C-terminal splicing region upstream and downstream, respectively, of the transgene, and is inserted into the target locus such that it is operably linked and in frame with the promoter of the gene at the target locus. After translation, the transgene polypeptide is spliced out, resulting in the production of uninterrupted protein encoded by the gene at the target locus. This configuration provides for transgene insertion into any coding exon in the gene at the target locus. P, endogenous gene promoter; UTR, endogenous gene untranslated region; PolyA, polyadenylation sequence; N' SR, N-terminal splicing region; C' SR, C-terminal splicing region. The use of the targeted nuclease TALEN is optional.

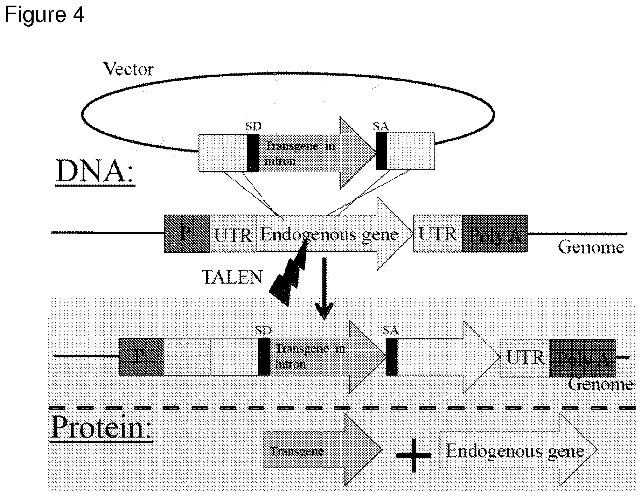

**Figure 4** depicts targeted integration without gene disruption using an intron configuration. The transgene cassette comprises a splice donor and splice acceptor upstream and downstream, respectively, of the transgene, and is inserted into the target locus such that it is operably linked and in frame with the promoter of the gene at the target locus. After transcription, the transgene pre-mRNA is spliced out, allowing for uninterrupted translation of protein encoded by the gene at the target locus. This configuration provides for transgene insertion into any transcribed region of the target locus, i.e. any region 5' of the polyadenylation sequence. P, endogenous gene promoter; UTR, endogenous gene untranslated region; PolyA, polyadenylation sequence; SD, splice donor; SA, splice acceptor. The use of the targeted nuclease TALEN is optional.

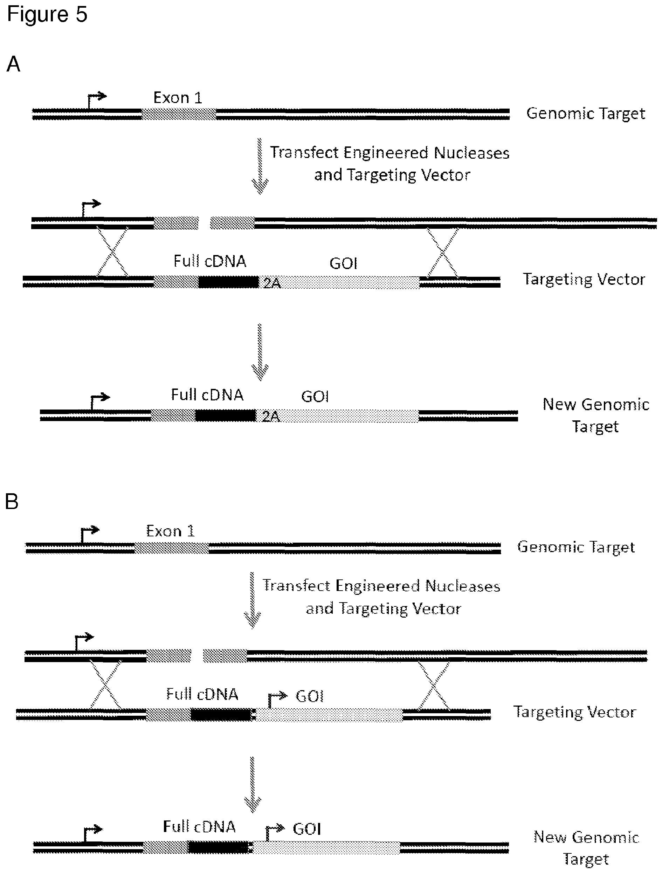

**Figure 5** depicts targeted integration without gene disruption by cDNA complementation of the gene at the target locus. The coding sequence downstream of the insertion site (with wobble mutations to prevent premature recombination if inserted in the 3' end of a coding exon, or without wobble mutations if inserted in the 5' end of the coding exon) is provided on the donor polynucleotide ("targeting vector") in addition to the gene of interest ("GOI"), and is inserted into the target locus such that it is under the control of its own promoter. (A) The gene of interest may be separated from the cDNA for the gene at the target locus by a 2A peptide, so that the gene of interest will also be under control of the promoter at the target locus. (B) Alternatively, the gene of interest may be operably linked to a separate promoter.

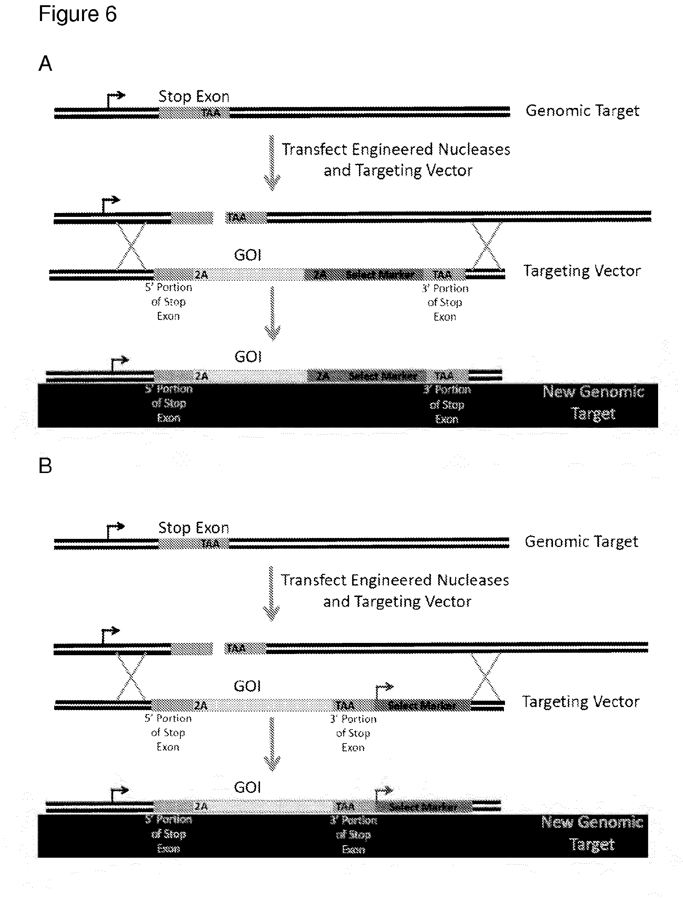

**Figure 6** depicts targeted integration of multiple genes of interest. The gene of interest ("GOI", in green) coupled to a 2A peptide is inserted into the target locus such that it is operably linked to the promoter of the gene at the target locus. In addition, a second gene of interest--in this instance, a selectable marker-- is also inserted into the locus. (A) The selectable marker is expressed from the same promoter driving expression of the gene at the target locus and the gene of interest by including a 2A peptide between the gene of interest and the selectable marker. (B) The selectable is operably linked to a promoter distinct from that driving the expression of the gene at the target locus and the first gene of interest.

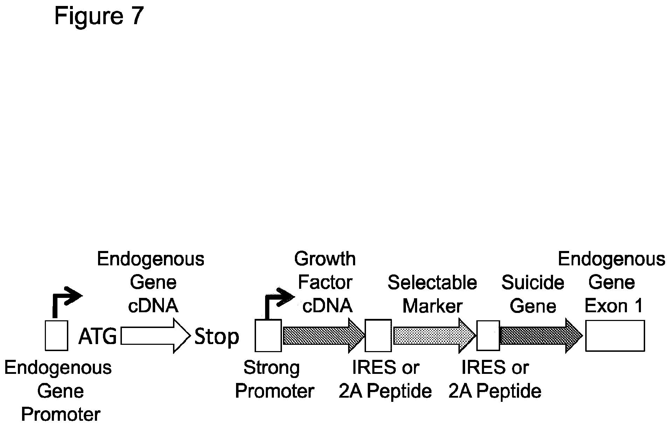

**Figure 7** provides a schematic of an engineered genomic target. In this example cells, e.g. fibroblasts, are engineered to secrete wound healing growth factors. The growth factor cDNA (e.g. PDGFbb, VEGF, FGF, etc.) is integrated into a target locus (e.g. the ADA gene) under the control of a strong promoter (e.g. CMV, CAG, UBC, EF1a, Fibronectin etc.), which promotes high expression of the therapeutic growth factor by the cells. Also integrated in this example is cDNA for the endogenous gene (to provide for gene complementation), a selectable marker (for selection and purification of the engineered cells, e.g. P140KMGMT, truncated NGFR, truncated CD4, truncated CD8, etc.), and a suicide gene under the control of an inducible promoter (to eliminate the cells from the body after they have secreted sufficient growth factors to heal the wound, e.g. inducible Caspase9, HSV-TK, CD20, etc.).

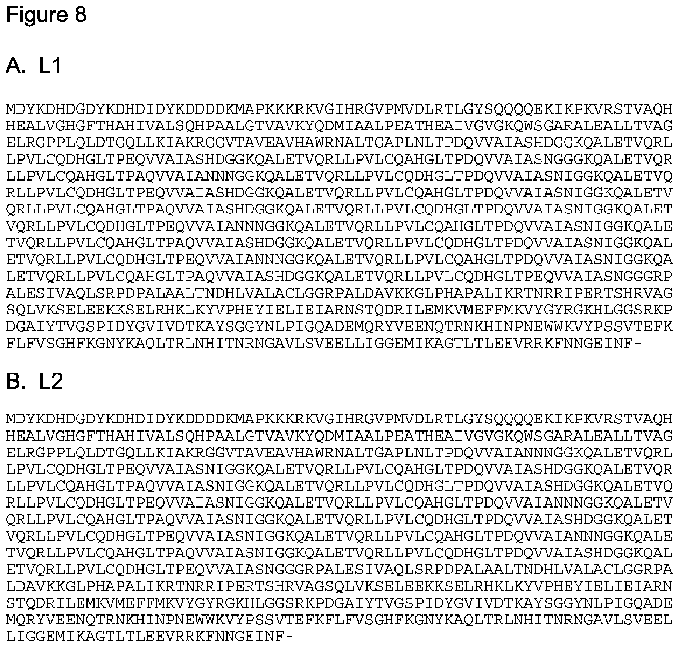

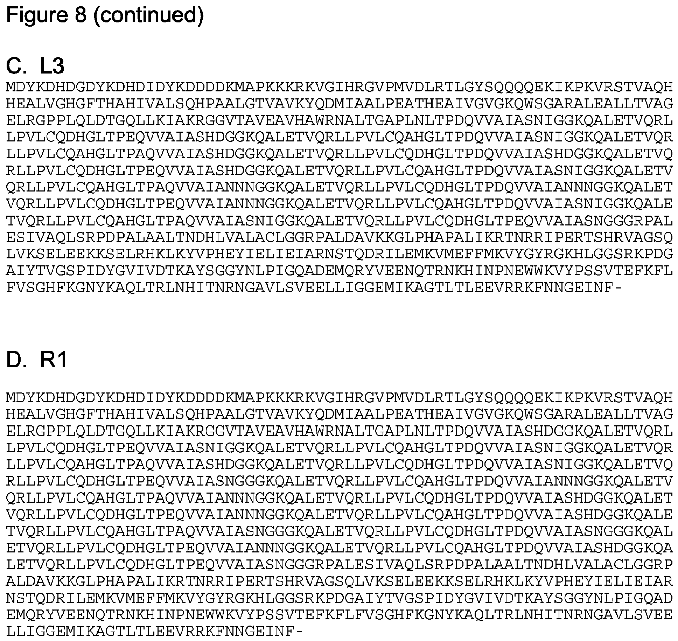

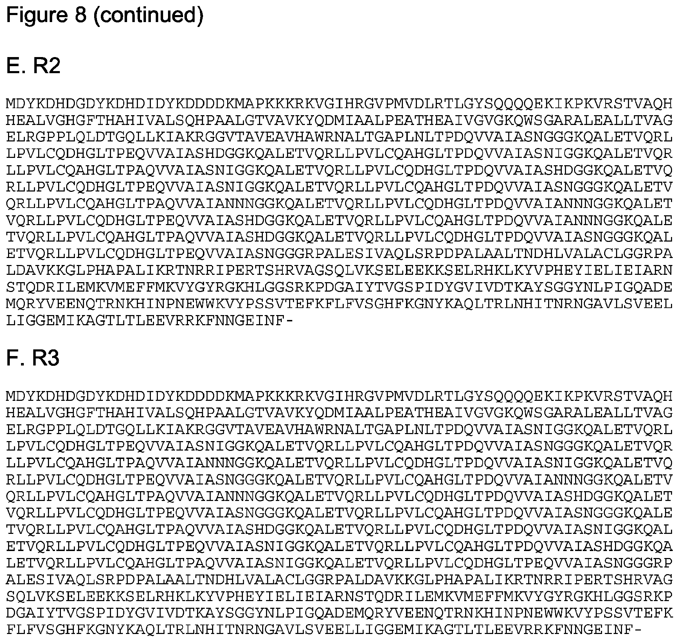

**Figure 8** provides examples of TALEN sequences that may be used to target the human IL2RG gene. (A) Left sequence L1 (SEQ ID NO:9); (B) Left sequence L2 (SEQ ID NO:10); (C) Left sequence L3 (SEQ ID NO:11); (D) Right sequence R1 (SEQ ID NO:12); (E) Right sequence R2 (SEQ ID NO:13); (F) Right sequence R3 (SEQ ID NO:14). Combinations of sequences of particular interest include L1/R1, L1/R2, L1/R3, L2/R1, L2/R2, L2/R3, and L3/R3.

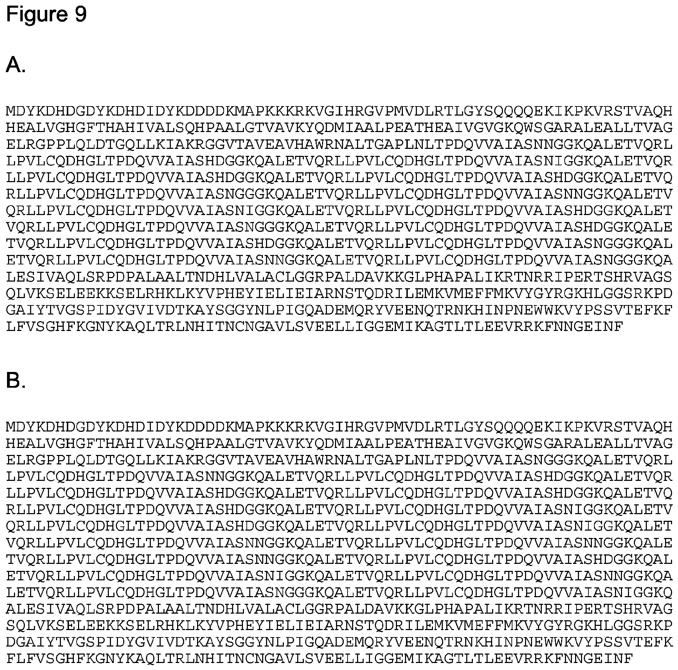

**Figure 9** provides examples of TALEN sequences that may be used together to target the human beta-globin gene. (A) Left sequence (SEQ ID NO:15); (B) Right sequence (SEQ ID NO:16).

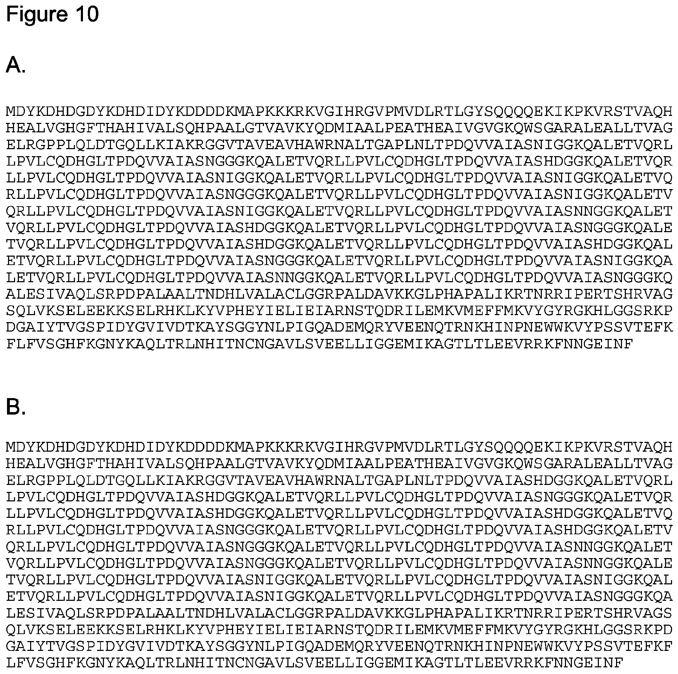

**Figure 10** provides examples of TALEN sequences that may be used together to target the human gamma-globin gene. (A) Left sequence(SEQ ID NO:17); (B) Right sequence(SEQ ID NO:18).

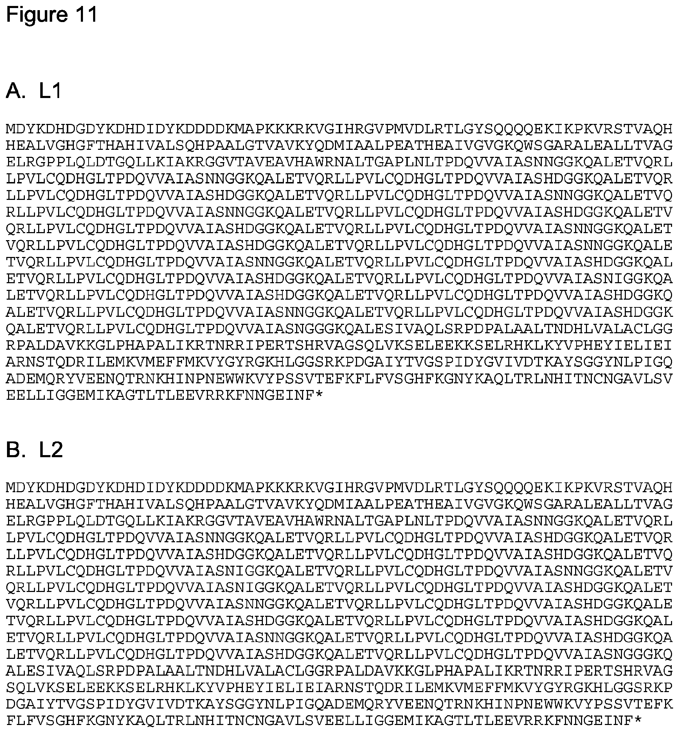

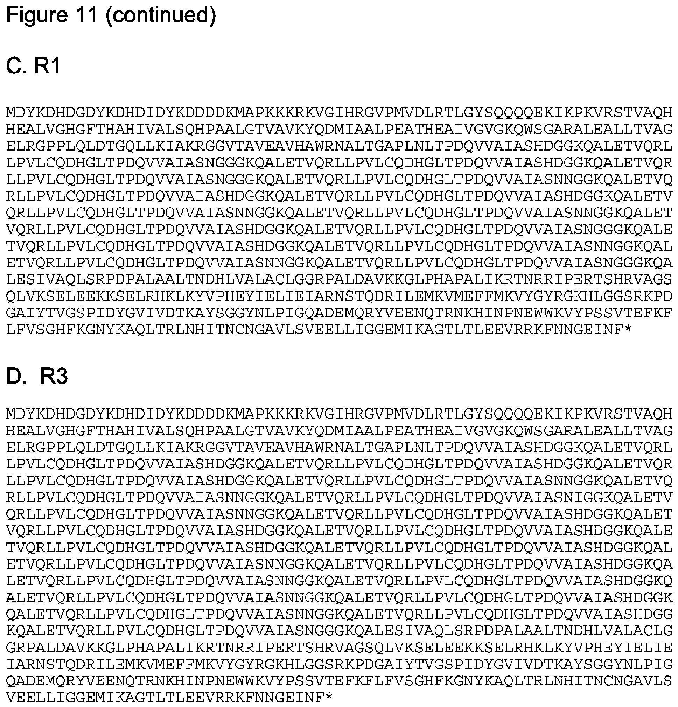

**Figure 11** provides examples of TALEN sequences that may be used to target the human ADA gene. (A) Left sequence L1 (SEQ ID NO:19); (B) Left sequence L2 (SEQ ID NO:20); (C) Right sequence R1(SEQ ID NO:21); (D) Right sequence R3 (SEQ ID NO:22). Combinations of particular interest include L1/R1 and L2/ R3.

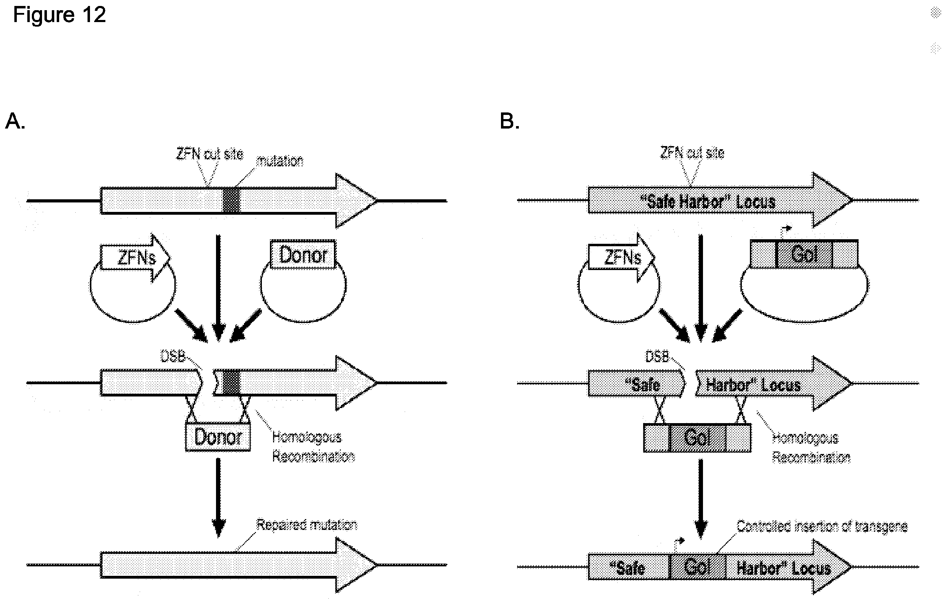

**Figure 12** is a depiction of gene correction (A), versus gene addition (B).

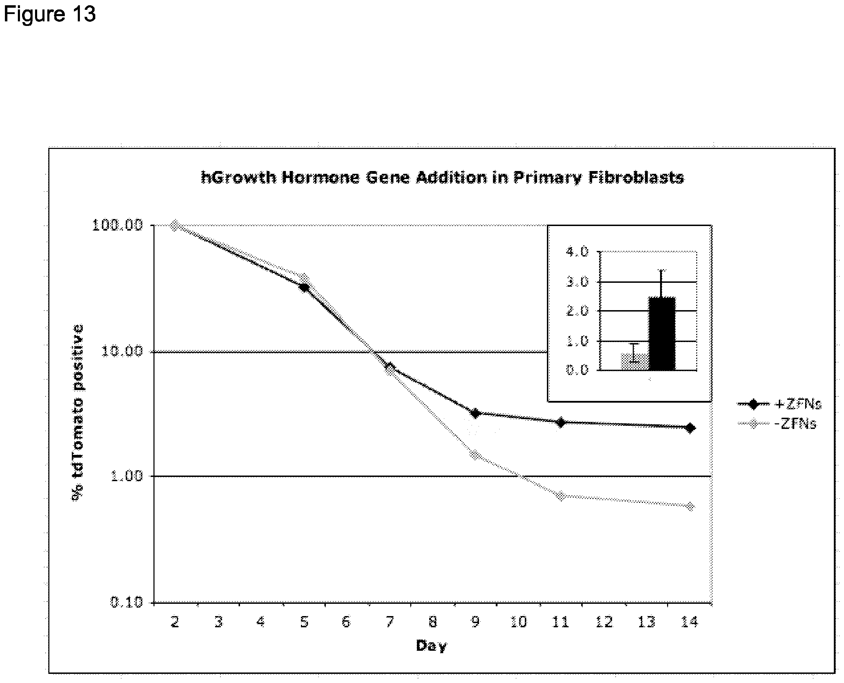

**Figure 13** depicts gene addition with a non-specific reporter.

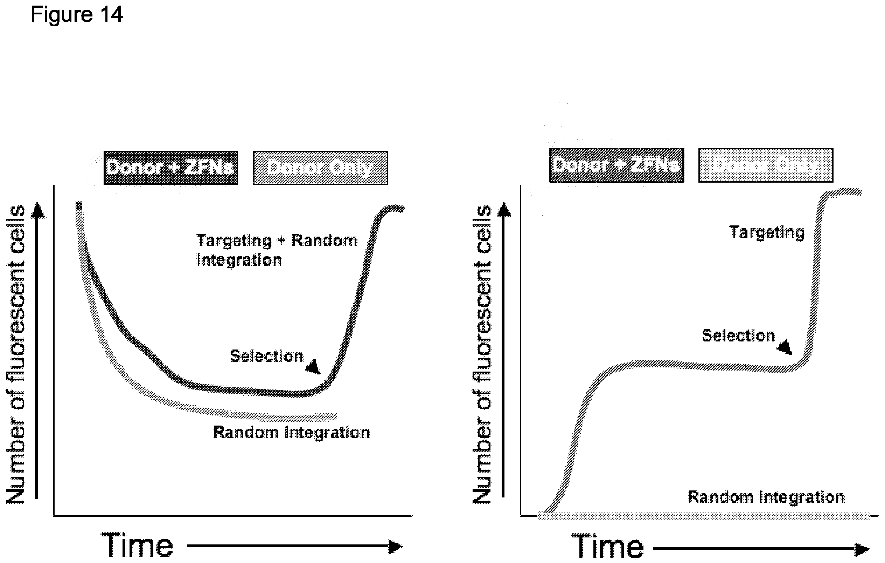

**Figure 14** depicts reporter readouts.

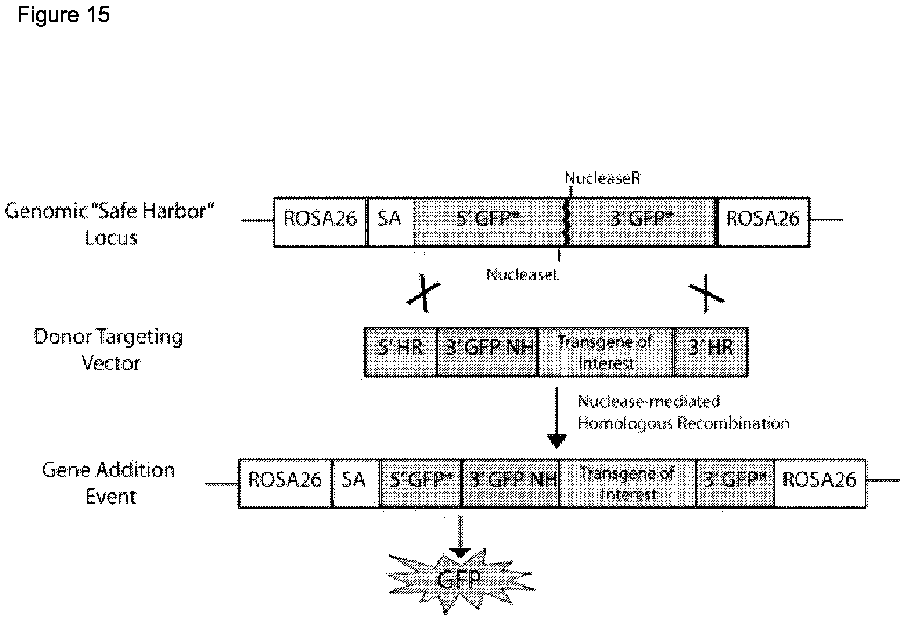

**Figure 15** illustrates the development of a gene-addition specific reporter.

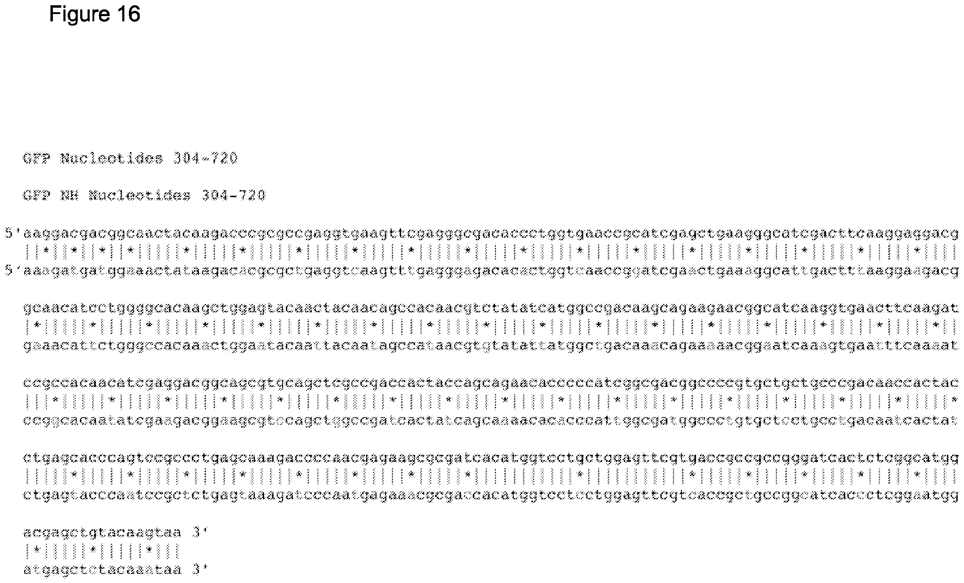

**Figure 16** illustrates the strategy for modifying GFP codons to produce the GFP NH coding sequence used in the reporter of figure 15 (top sequence: SEQ ID NO:23; bottom sequence: SEQ ID NO:24)

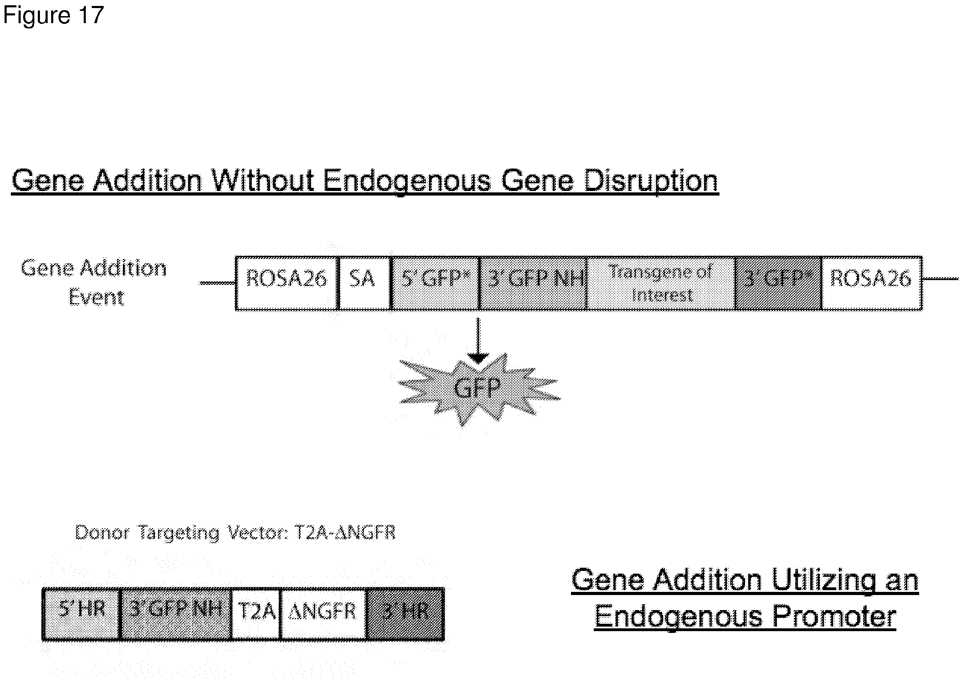

**Figure 17** depicts the implications for targeting in human cells.

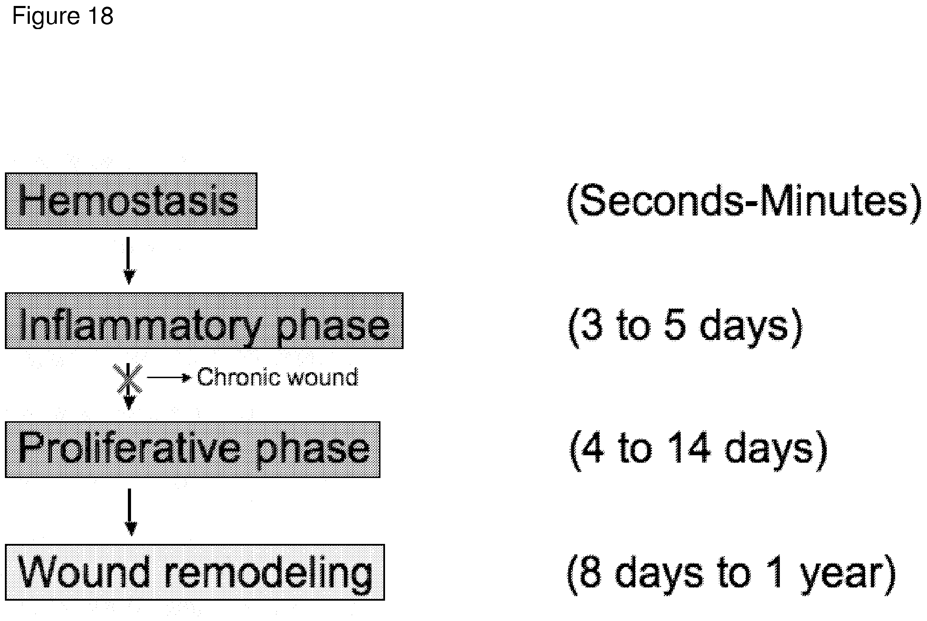

**Figure 18** provides a review of the stages of wound healing.

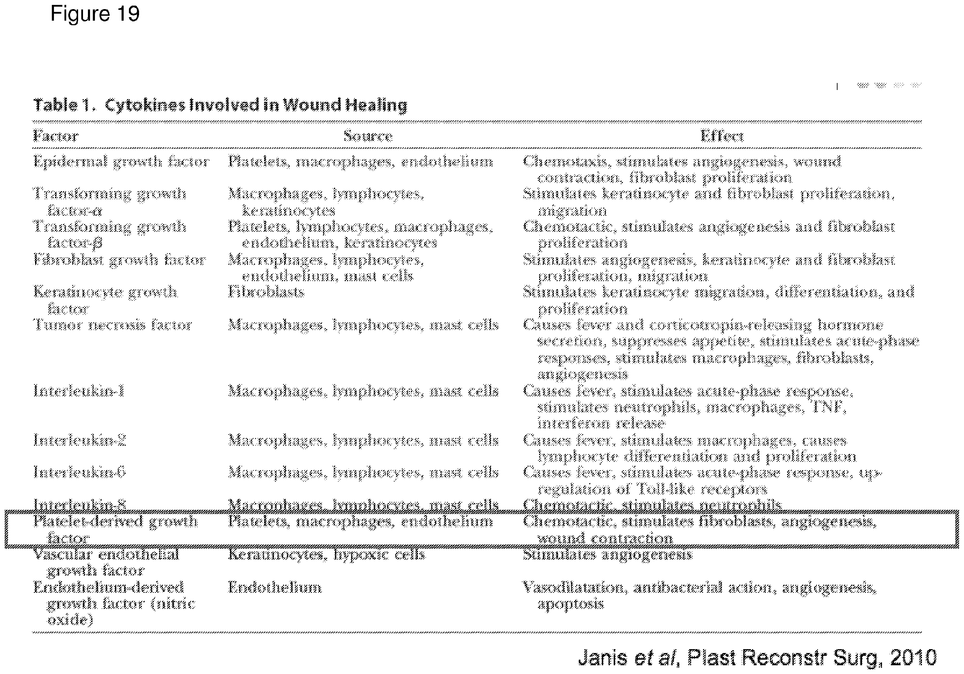

**Figure 19** provides examples of cytokines that may be expressed from a target locus by the subject methods to treat chronic wounds.

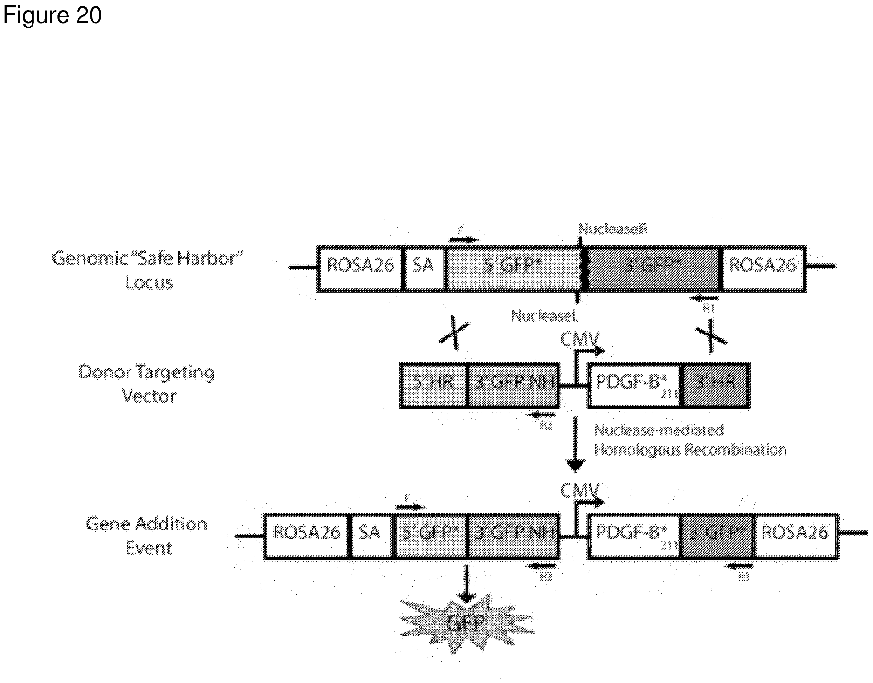

**Figure 20** depicts the application of the subject gene addition methodology to the integration of the PDGF gene at the mouse ROSA26 locus in mouse fibroblasts.

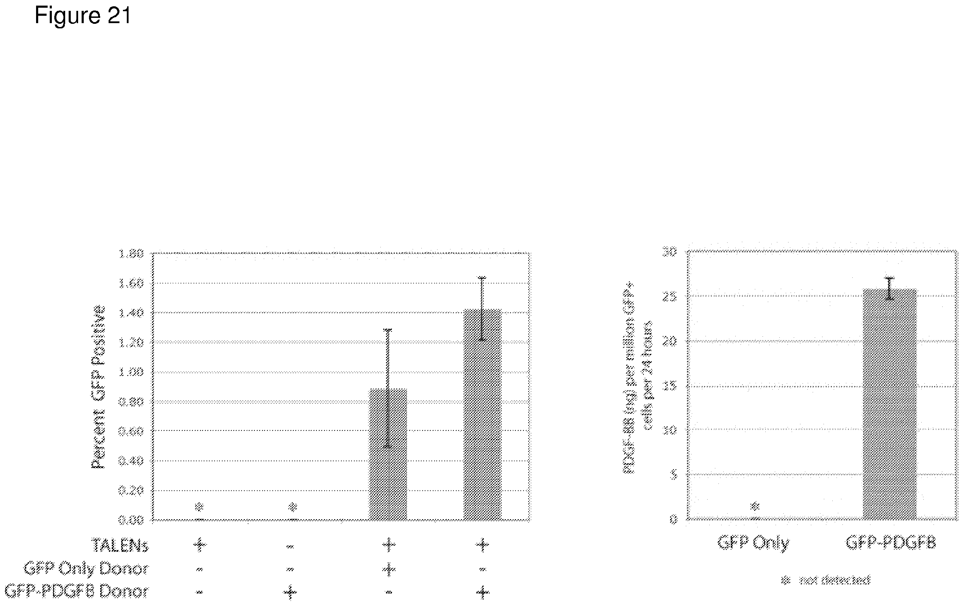

**Figure 21** demonstrates the expression of the integrated donor vector in Figure 20 in fibroblasts.

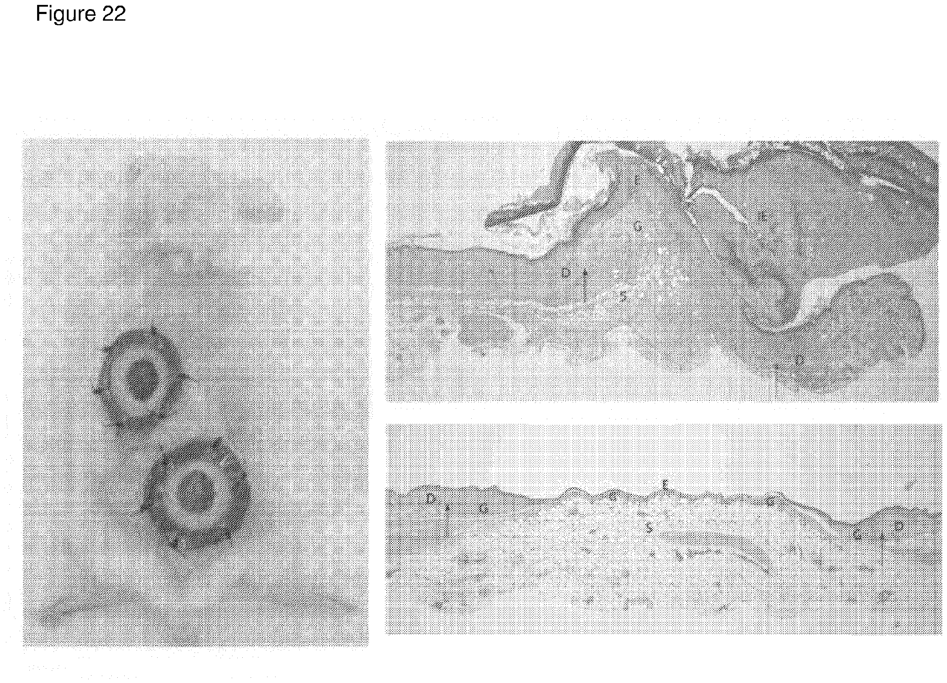

**Figure 22** depicts a mouse model of wound healing, in which splinting prevents wound contracture (Galiano et al. (2004) Quantitative and reproducible murine model of excisional wound healing. Wound Rep Regen. 12(4):485-92).

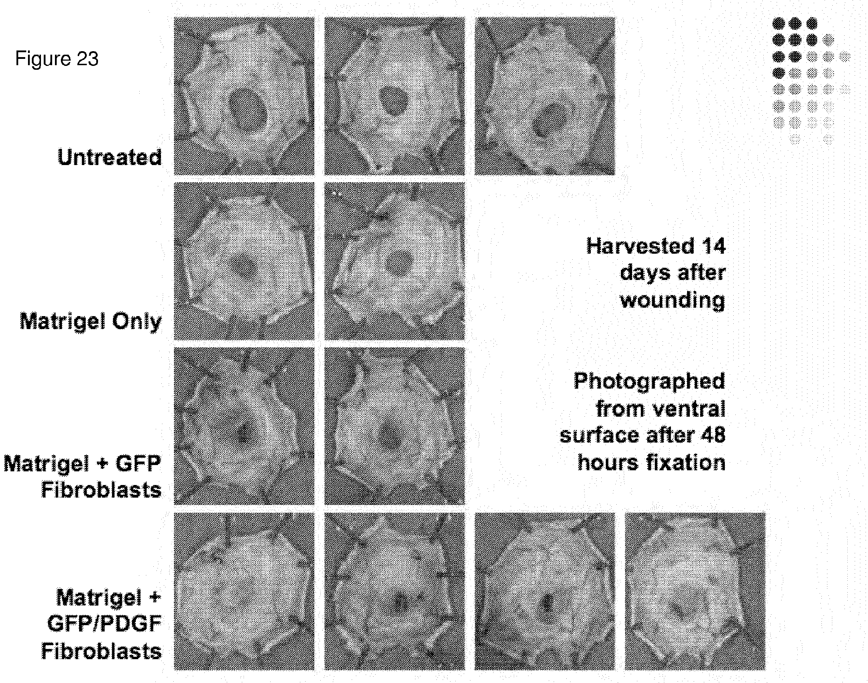

**Figure 23** demonstrates the efficacy with which fibroblasts modified by the subject methods to express PDGF promote wound healing.

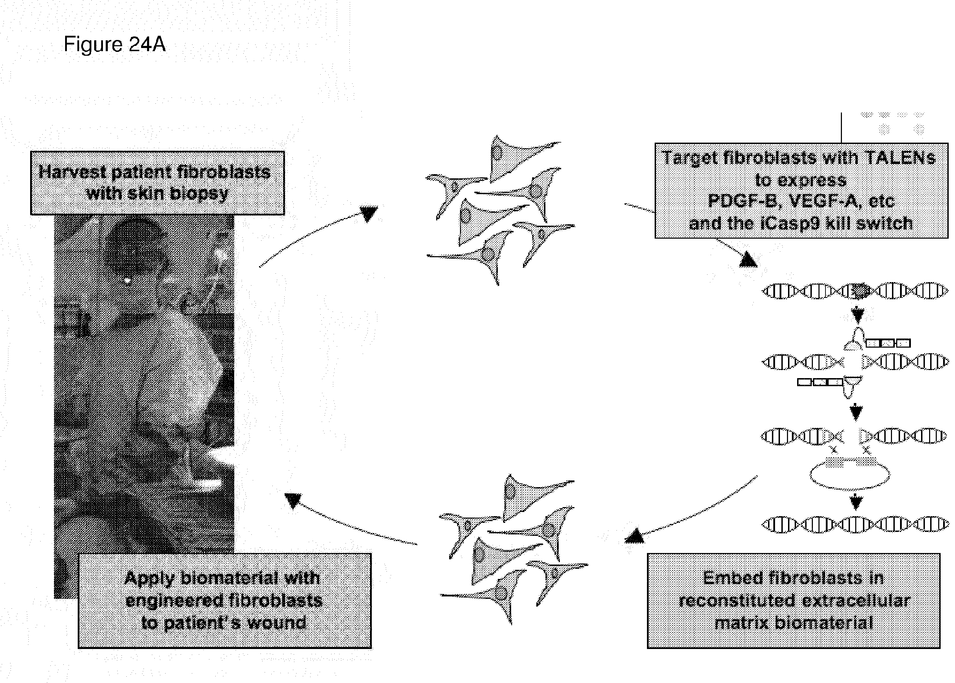

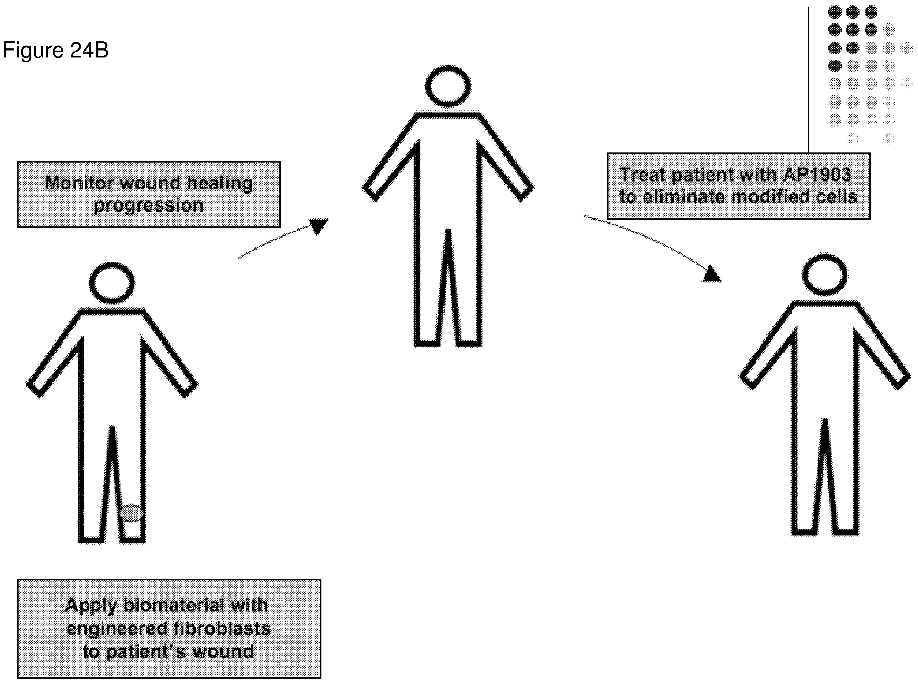

**Figure 24** depicts the application of the subject gene addition methodology to the treatment of a wound in a patient. (A) Modification of fibroblasts ex vivo and transplantation back to the individual. (B) Monitoring the fibroblast recipient, and eliminating those fibroblasts after wound healing is complete using the integrated suicide gene.

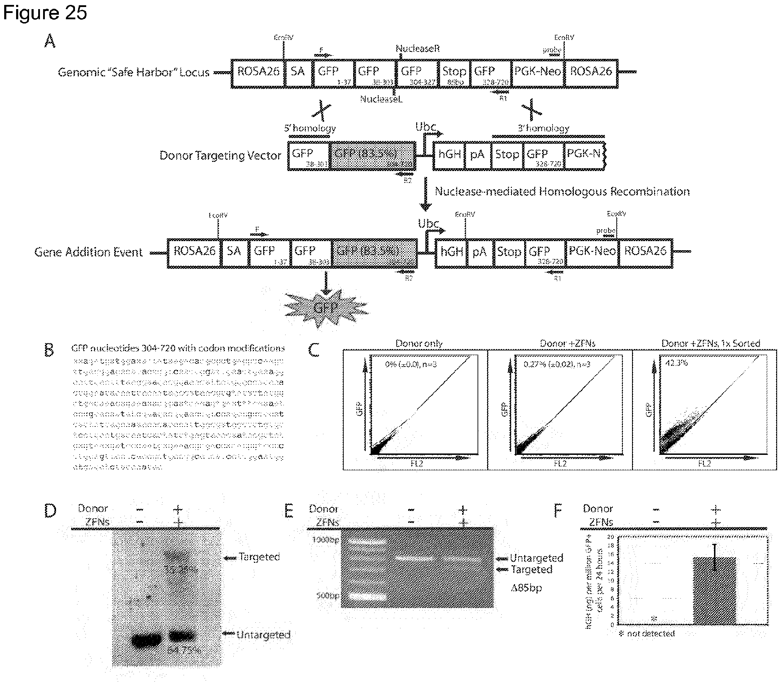

**Figure 25** depicts designing a gene addition-specific GFP reporter locus followed by human growth hormone gene addition. We designed a donor plasmid containing regions of homology to the genomic safe harbor locus. When nuclease expression plasmids were co-transfected with the donor, a site-specific gene addition event occurs (A). Critically, we included in our donor a region of DNA which can encode for the c-terminus of GFP, yet is non-homologous for wild-type GFP (B, SEQ ID NO:24). This allows for the GFP expression to serve as a specific reporter for gene addition while simultaneously allowing transgene insertion. We demonstrated that co-transfection of all 3 plasmids resulted in GFP+ cells and that these could be sorted by flow cytometry (C). Sorted cells were analyzed by DIG-Southern with an EcoRV digest, and gene addition was confirmed (D). PCR of sorted cells also confirmed gene addition (E). ELISA was performed on the sorted population of cells and confirmed growth hormone expression (F).

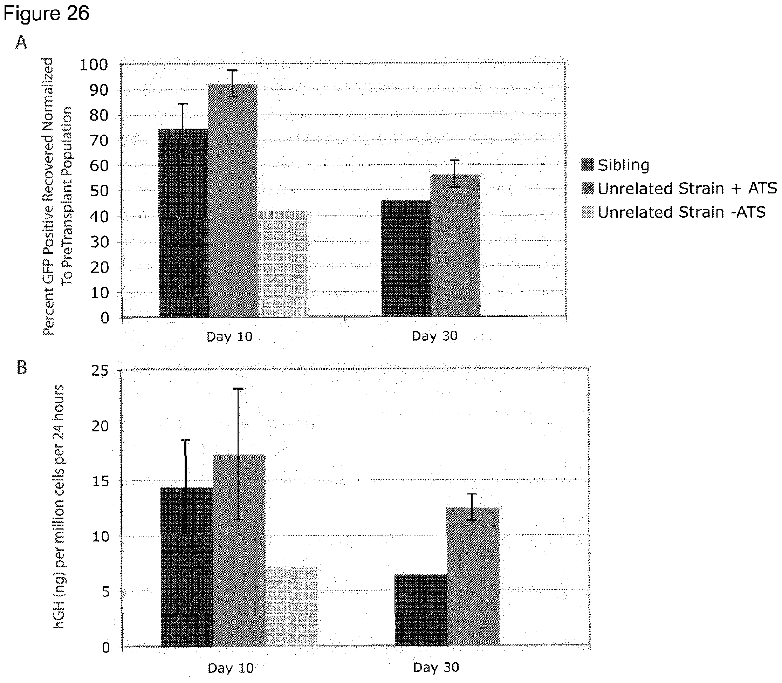

**Figure 26** demonstrates the engraftment of engineered fibroblasts into recipient mice. We transplanted fibroblasts targeted with the gene addition construct described in Figure 25 subcutaneously in Matrigel into either a sibling mouse (dark grey), an unrelated mouse pretreated with anti-mouse thymocyte serum (ATS) for immunosuppresion (intermediate grey) or an unrelated mouse without ATS treatment (light grey). We excised the matrigel plug and observed successful engraftment of the fibroblasts after 10 days in the sibling and unrelated +ATS cohorts. After 30 days however, only the unrelated +ATS cohort had substantial engraftment (A). hGH expression was analyzed with ELISA after excision of the matrigel plug and it was found that hGH expression persisted after transplantation and mirrored GFP expression (B). Error bars represent +/- 1 standard deviation.

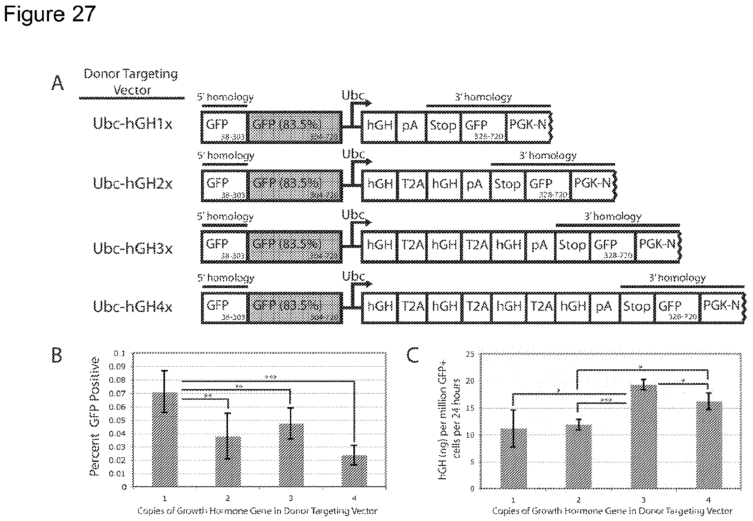

**Figure 27** illustrates that growth hormone expression increases by targeting T2A-linked cDNA tandem arrays. We designed four donor constructs, each containing an increasing number of growth hormone cDNA copies linked by a T2A peptide (A). As the size of the donor increased, the targeting efficiency decreased (B). Next, we sorted for GFP+ cells and normalized growth hormone expression (ELISA) to the GFP percentage. We found that increasing the copy number of cDNA can increase expression. However, Ubc-hGH4x did have lower expression than Ubc-hGH3x(C). Error bars represent +/- 1 standard deviation and p values were calculated with a Student's T-test assuming unequal variances. *p ≤0.05, **p ≤0.01, ***p ≤0.001

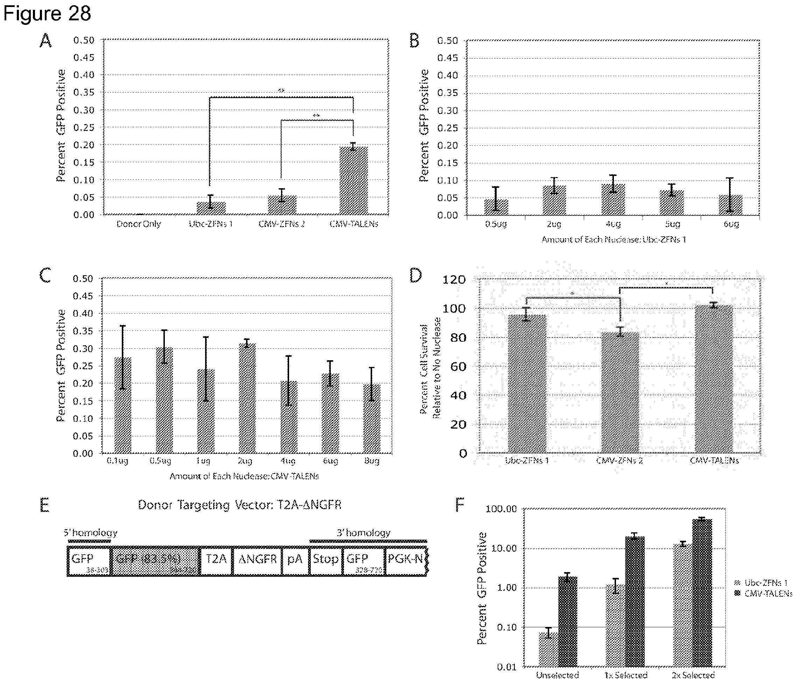

**Figure 28** illustrates that TALENs demonstrate increased targeting and decreased toxicity compared with ZFNs. We compared the ability of TALENs to stimulate gene addition compared with the ZFNs used in Figure 27.1, 27.2 and 27.3. We found that TALENs outperformed ZFNs in terms of targeting efficiency (A) and also in terms of decreased cellular toxicity (D). We titrated the amount of ZFNs (B) and TALENs (C) and found that TALENs had higher levels of gene addition at all quantities. We then designed a donor construct to test the ability to target a transgene (truncated nerve growth factor receptor) in-frame with the target locus without the use of an exogenous promoter (E). We were able to successfully target and select for the transgene using magnetic beads (F). Error bars represent +/- 1 standard deviation and p values were calculated with a Student's T-test assuming unequal variances. *p ≤0.05, **p ≤0.001

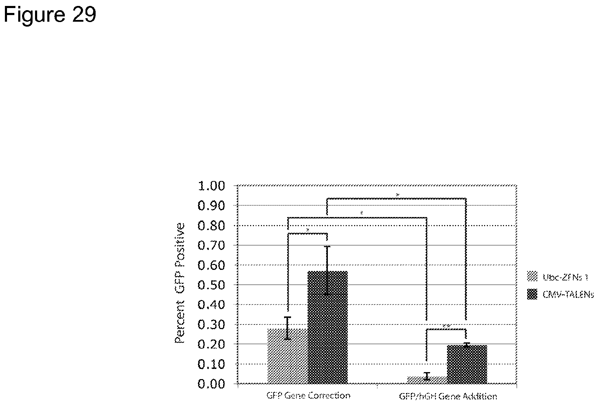

**Figure 29** illustrates GFP gene correction versus GFP-human growth hormone gene addition. We compared our previously published GFP gene correction strategy with the GFP-human growth hormone gene addition described in this study. Smaller DNA modifications associated with gene correction ("GFP Gene Correction") showed an increased frequency of targeting compared with larger gene insertions ("GFP/hGH Gene Addition"). TALENs (dark grey) demonstrated an increased frequency of targeting for both gene correction and gene addition compared with ZFNs (light grey). *p ≤0.05, **p ≤0.001

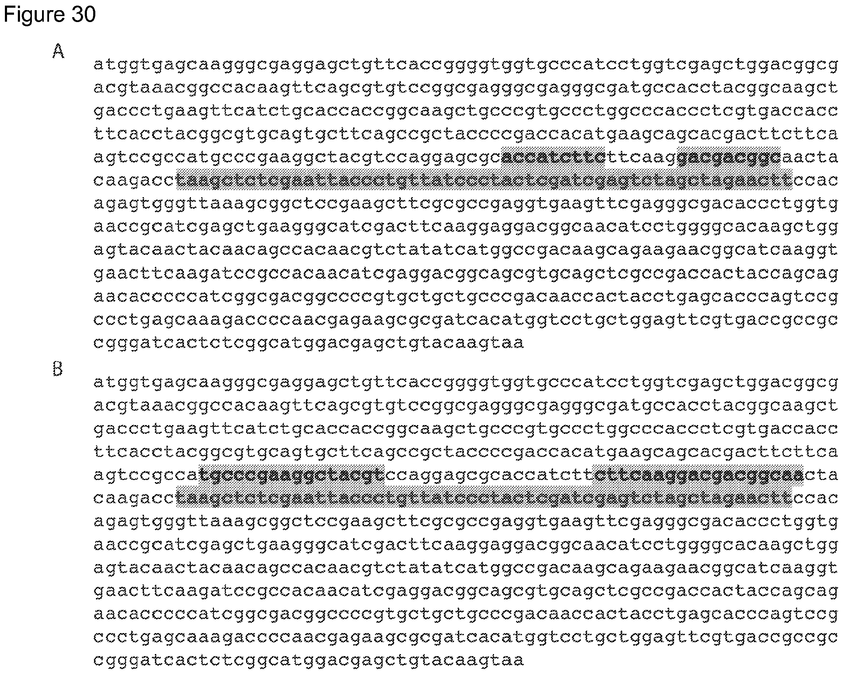

**Figure 30** provides ZFN and TALEN binding sites. Shown is the GFP target locus with an 85bp insertion (red bold) rendering the endogenous knock-in GFP gene non-functional. Left and right ZFN binding sites (A, SEQ ID NO:25, black bold) and left and right TALEN binding sites (B, SEQ ID NO:26, black bold) are depicted showing overlap and proximity.

### DETAILED DESCRIPTION OF THE INVENTION

Compositions and methods are provided for integrating one or more genes of interest into cellular DNA without substantially disrupting the expression of the gene at the locus of integration, i.e., the target locus. These compositions and methods are useful in any in vitro or in vivo application in which it is desirable to express a gene of interest in the same spatially and temporally restricted pattern as that of a gene at a target locus while maintaining the expression of the gene at the target locus, for example, to treat disease, in the production of genetically modified organisms in agriculture, in the large scale production of proteins by cells for therapeutic, diagnostic, or research purposes, in the induction of iPS cells for therapeutic, diagnostic, or research purposes, in biological research, etc. Reagents, devices and kits thereof that find use in practicing the subject methods are also provided. These and other objects, advantages, and features of the invention will become apparent to those persons skilled in the art upon reading the details of the compositions and methods as more fully described below.

Before the present methods and compositions are described, it is to be understood that this invention is not limited to particular method or composition described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described. All publications mentioned herein are incorporated herein by reference to disclose and describe the methods and/or materials in connection with which the publications are cited. It is understood that the present disclosure supercedes any disclosure of an incorporated publication to the extent there is a contradiction.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

It must be noted that as used herein and in the appended claims, the singular forms "a", "an", and "the" include plural referents unless the context clearly dictates otherwise. Thus, for example, reference to "a cell" includes a plurality of such cells and reference to "the peptide" includes reference to one or more peptides and equivalents thereof, e.g. polypeptides, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Definitions

A "DNA molecule" refers to the polymeric form of deoxyribonucleotides (adenine, guanine, thymine, or cytosine) in either single stranded form or a double-stranded helix. This term refers only to the primary and secondary structure of the molecule, and does not limit it to any particular tertiary forms. Thus, this term includes double-stranded DNA found, inter alia, in linear DNA molecules (e.g., restriction fragments), viruses, plasmids, and chromosomes.

As used herein, a "gene of interest" is a DNA sequence that is transcribed into RNA and in some instances translated into a polypeptide in vivo when placed under the control of appropriate regulatory sequences. A gene of interest can include, but is not limited to, prokaryotic sequences, cDNA from eukaryotic mRNA, genomic DNA sequences from eukaryotic (e.g., mammalian) DNA, and synthetic DNA sequences. For example, a gene of interest may encode an miRNA, an shRNA, a native polypeptide (i.e. a polypeptide found in nature) or fragment thereof; a variant polypeptide (i.e. a mutant of the native polypeptide having less than 100% sequence identity with the native polypeptide) or fragment thereof; an engineered polypeptide or peptide fragment, a therapeutic peptide or polypeptide, an imaging marker, a selectable marker, etc.

As used herein, a "target locus" is a region of DNA into which a gene of interest is integrated, e.g. a region of DNA in a vector, a region of DNA in a phage, a region of chromosomal or mitochondrial DNA in a cell, etc.

As used herein, a "target gene" or "endogenous gene" or "gene at a target locus" is a gene that naturally exists at a locus of integration, i.e. the gene that is endogenous to the target locus.

A "coding sequence", e.g. coding sequence for a gene at a target locus, is a DNA sequence which is transcribed and translated into a polypeptide in vivo when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and synthetic DNA sequences. A polyadenylation signal and transcription termination sequence may be located 3' to the coding sequence.

"DNA regulatory sequences", as used herein, are transcriptional and translational control sequences, such as promoters, enhancers, polyadenylation signals, terminators, and the like, that provide for and/or regulate expression of a coding sequence in a host cell.

As used herein, a "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains responsible for the binding of RNA polymerase. Eukaryotic promoters will often, but not always, contain "TATA" boxes and "CAT" boxes. Various promoters, including inducible promoters, may be used to drive the various vectors of the present invention.

As used herein, the term "reporter gene" refers to a coding sequence whose product may be assayed easily and quantifiably when attached to promoter and in some instances enhancer elements and introduced into tissues or cells. The promoter may be a constitutively active promoter, i.e. a promoter is active in the absence externally applied agents, or it may be an inducible promoter, i.e. a promoter whose activity is regulated upon the application of an agent to the cell, e.g. doxycycline.

A "vector" is a replicon, such as plasmid, phage, virus, or cosmid, to which another DNA segment, i.e. an "insert", may be attached so as to bring about the replication of the attached segment.

An "expression cassette" comprises a DNA coding sequence operably linked to a promoter. By "operably linked" it is meant that the promoter effectively controls expression of the coding sequence.

A "DNA construct" is a DNA molecule comprising a vector and an insert, e.g. an expression cassette.

By a "2A peptide" it is meant a small (18-22 amino acids) sequence that allows for efficient, stoichiometric production of discrete protein products within a single reading frame through a ribosomal skipping event within the 2A peptide sequence.

By an "internal ribosome entry site," or "IRES" it is meant a nucleotide sequence that allows for the initiation of protein translation in the middle of a messenger RNA (mRNA) sequence.

By an "intein" it is meant a segment of a polypeptide that is able to excise itself and rejoin the remaining portions (the "exteins") with a peptide bond.

By an "intron" it is meant a nucleotide sequence within a gene that is removed by RNA splicing to generate the final mature RNA product of a gene

A cell has been "transformed" or "transfected" by exogenous or heterologous DNA, e.g. a DNA construct, when such DNA has been introduced inside the cell. The transforming DNA may or may not be integrated (covalently linked) into the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth in vitro for many generations.

"Binding" as used herein, e.g. with reference to DNA binding domains, refers to a sequence-specific, non-covalent interaction between macromolecules (e.g., between a protein and a nucleic acid). Not all components of a binding interaction need be sequence-specific (e.g., contacts with phosphate residues in a DNA backbone), as long as the interaction as a whole is sequence-specific. Such interactions are generally characterized by a dissociation constant (K_{d}) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. "Affinity" refers to the strength of binding, increased binding affinity being correlated with a lower K_{d}.

By "binding domain" it is meant a protein domain that is able to bind non-covalently to another molecule. A binding domain can bind to, for example, a DNA molecule (a DNA-binding protein), an RNA molecule (an RNA-binding protein) and/or a protein molecule (a protein-binding protein). In the case of a protein domain-binding protein, it can bind to itself (to form homodimers, homotrimers, etc.) and/or it can bind to one or more molecules of a different protein or proteins.

By "heterologous DNA binding domain" it is meant a DNA binding domain in a protein that is not found in the native protein. For example, in a Spo11-DNA binding domain fusion protein in which the DNA binding domain is a heterologous DNA binding domain, the DNA binding domain is from a protein other than Spo11.

An "accessible region" is a site in cellular chromatin in which a target site present in the nucleic acid can be bound by an exogenous molecule comprising a DNA binding domain which recognizes the target site. A "target site" or "target sequence" is a nucleic acid sequence that defines a portion of a nucleic acid to which a DNA binding molecule will bind, provided sufficient conditions for binding exist. For example, the sequence 5'-GAATTC-3' is a target site for the Eco RI restriction endonuclease.

By "cleavage" it is meant the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In certain embodiments, fusion polypeptides are used for targeted double-stranded DNA cleavage.

"Nuclease" and "endonuclease" are used interchangeably herein to mean an enzyme which possesses catalytic activity for DNA cleavage.

By "cleavage domain" or "active domain" of a nuclease it is meant the polypeptide sequence or domain within the nuclease which possesses the catalytic activity for DNA cleavage. A cleavage domain can be contained in a single polypeptide chain or cleavage activity can result from the association of two (or more) polypeptides.

By "targeted nuclease" it is meant a nuclease that is targeted to a specific DNA sequence. Targeted nucleases are targeted to a specific DNA sequence by the DNA binding domain to which they are fused. In other words, the nuclease is guided to a DNA sequence, e.g. a chromosomal sequence or an extrachromosomal sequence, e.g. an episomal sequence, a minicircle sequence, a mitochondrial sequence, a chloroplast sequence, etc., by virtue of its fusion to a DNA binding domain with specificity for the target DNA sequence of interest.

By "recombination" it is meant a process of exchange of genetic information between two polynucleotides. As used herein, "homologous recombination (HR)" refers to the specialized form of such exchange that takes place, for example, during repair of double-strand breaks in cells. This process requires nucleotide sequence homology, uses a "donor" molecule to template repair of a "target" molecule (i.e., the one that experienced the double-strand break), and leads to the transfer of genetic information from the donor to the target. Homologous recombination may result in an alteration of the sequence of the target molecule, if the donor polynucleotide differs from the target molecule and part or all of the sequence of the donor polynucleotide is incorporated into the target polynucleotide.

General methods in molecular and cellular biochemistry can be found in such standard textbooks as Molecular Cloning: A Laboratory Manual, 3rd Ed. (Sambrook et al., HaRBor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998), the disclosures of which are incorporated herein by reference. Reagents, cloning vectors, and kits for genetic manipulation referred to in this disclosure are available from commercial vendors such as BioRad, Stratagene, Invitrogen, Sigma-Aldrich, and ClonTech.

As summarized above, compositions and methods are provided for integrating a gene of interest into cellular DNA without substantially disrupting the expression of the gene at the locus of integration, i.e. the target locus. In other words, the normal expression of the gene that resides at the target locus (the "endogenous gene", or "target gene") is maintained spatially (i.e. in cells and tissues in which it would normally be expressed), temporally (i.e. at the correct times, e.g. developmentally, during cellular response, etc.), and at levels that are substantially unchanged from normal levels, for example, at levels that differ 5-fold or less from normal levels, e.g. 4-fold or less, or 3-fold or less, more usually 2-fold or less from normal levels, following targeted integration of the gene of interest into the target locus. By "integration" it is meant that the gene of interest is stably inserted into the cellular genome, i.e. covalently linked to the nucleic acid sequence within the cell's chromosomal or mitochondrial DNA. By "targeted integration" it is meant that the gene of interest is inserted into the cell's chromosomal or mitochondrial DNA at a specific site, or "integration site". These compositions and methods are particularly beneficial because they provide for genetic modification of cellular DNA and the expression of one or more genes of interest, e.g. a gene encoding a therapeutic polypeptide or peptide thereof, a gene encoding an imaging marker, a gene encoding a selectable marker, etc., from that cellular DNA without affecting cellular functions promoted by the gene that is expressed from that cellular DNA.

In describing aspects of the invention, compositions will be described first, followed by methods for their use.

### COMPOSITIONS

In performing the subject methods, a gene of interest is provided to cells on a donor polynucleotide, also referred to herein as a "targeting polynucleotide" or "targeting vector". In other words, cells are contacted with a donor polynucleotide that comprises the nucleic acid sequence to be integrated into the cellular genome by targeted integration. To promote targeted integration, the donor polynucleotide may comprise nucleic acid sequences that promote homologous recombination at the site of integration. Homologous recombination refers to the exchange of nucleic acid material that takes place, for example, during repair of double-strand breaks in cells, for example, double strand breaks caused by a targeted nuclease. This process requires nucleotide sequence homology, using the "donor" molecule, e.g. the donor polynucleotide, to template repair of a "target" molecule, i.e., the nucleic acid that experienced the double-strand break, e.g. a target locus in the cellular genome, and leads to the transfer of genetic information from the donor to the target. As such, in donor polynucleotides of the subject compositions, the gene of interest may be flanked by sequences that contain sufficient homology to a genomic sequence at the cleavage site, e.g. 70%, 80%, 85%, 90%, 95%, or 100% homology with the nucleotide sequences flanking the cleavage site, e.g. within about 50 bases or less of the cleavage site, e.g. within about 30 bases, within about 15 bases, within about 10 bases, within about 5 bases, or immediately flanking the cleavage site, to support homologous recombination between it and the genomic sequence to which it bears homology. Approximately 25, 50 100 or 200 nucleotides or more of sequence homology between a donor and a genomic sequence will support homologous recombination therebetween.

The flanking recombination sequences can be of any length, e.g. 10 nucleotides or more, 50 nucleotides or more, 100 nucleotides or more, 250 nucleotides or more, 500 nucleotides or more, 1000 nucleotides (1 kb) or more, 5000 nucleotides (5 kb) or more, 10000 nucleotides (10kb) or more etc. Generally, the homologous region(s) of a donor sequence will have at least 50% sequence identity to a genomic sequence with which recombination is desired. In certain embodiments, 60%, 70%, 80%, 90%, 95%, 98%, 99%, or 99.9% sequence identity is present. Any value between 1% and 100% sequence identity can be present, depending upon the length of the donor polynucleotide.

In some instances, the flanking sequences may be substantially equal in length to one another, e.g. one may be 30% shorter or less than the other flanking sequence, 20% shorter or less than the other flanking sequence, 10% shorter or less than the other flanking sequence, 5% shorter or less than the other flanking sequence, 2% shorter or less than the other flanking sequence, or only a few nucleotides less than the other. In other instances, the flanking sequences may be substantially different in length from one another, e.g. one may be 40% shorter or more, 50% shorter or more, sometimes 60% shorter or more, 70% shorter or more, 80% shorter or more, 90% shorter or more, or 95% shorter or more than the other flanking sequence.

In some instances, the genomic sequences to which the flanking homologous sequences on the donor polynucleotide have homology are sequences that are used by nucleases or site-specific recombinases, e.g. integrases, resolvases, and the like, to promote site-specific recombination, e.g. as known in the art and as discussed in greater detail below.

The donor polynucleotide will typically also comprise one or more additional elements that provide for the expression of the gene of interest without substantially disrupting the expression of the gene at the target locus. For example, the donor polynucleotide may comprise a nucleic acid sequence encoding a 2A peptide positioned adjacent to the gene of interest. See, for example, Figure 1. By a "2A peptide" it is meant a small (18-22 amino acids) peptide sequence that allows for efficient, stoichiometric, concordant expression of discrete protein products within a single vector, regardless of the order of placement of the genes within the vector, through ribosomal skipping. 2A peptides are readily identifiable by their consensus motif (DVEXNPGP) and their ability to promote protein cleavage. Any convenient 2A peptide may be used in the donor polynucleotide, e.g. the 2A peptide from a virus such as foot-and-mouth disease virus (F2A), equine Rhinitis A virus, porcine teschovirus-1 (P2A) or Thosea asigna virus (T2A), or any of the 2A peptides described in Szymczak-Workman, A. et al. "Design and Construction of 2A Peptide-Linked Multicistronic Vectors". Adapted from: Gene Transfer: Delivery and Expression of DNA and RNA (ed. Friedmann and Rossi). CSHL Press, Cold Spring Harbor, NY, USA, 2007, the disclosure of which is incorporated herein by reference.

Typically, the gene of interest and 2A peptide will be positioned on the donor polynucleotide so as to provide for uninterrupted expression of the gene at the target locus upon insertion of the gene of interest. For example, it may be desirable to insert the gene of interest into an integration site that is 3', or "downstream" of the initiation codon of the gene at the target locus, for example, within the first 50 nucleotides 3' of the initiation codon (i.e. the start ATG) for the gene at the target locus, e.g. within the first 25 nucleotides 3' of initiation codon, within the first 10 nucleotides 3' of the initiation codon, within the first 5 nucleotides 3' of the initiation codon, or in some instances, immediately 3' of the initiation codon, i.e. adjacent to the initiation codon. In such instances, the 2A peptide would be positioned within the donor polynucleotide such that it is immediately 3' to the gene of interest, and flanking recombination sequences selected that will guide homologous recombination and integration of the gene of interest to the integration site that is 3' of the initiation codon at the target locus. See, for example, Figure 1A. As another example, it may be desirable to insert the gene of interest into an integration site that is 5', or "upstream" of the termination codon of the gene at the target locus, for example, within the first 50 nucleotides 5' of the termination codon (i.e. the stop codon, e.g. TAA, TAG, or TGA), e.g. within the first 25 nucleotides 5' of termination codon, within the first 10 nucleotides 5' of the termination codon, within the first 5 nucleotides of the termination codon, or in some embodiments, immediately 5' of the termination codon, i.e. adjacent to the termination codon. In such instances, the 2A peptide would be positioned within the donor polynucleotide such that it is immediately 5' to the gene of interest, and flanking recombination sequences selected that will guide homologous recombination and integration of the gene of interest to the integration site that is 5' of the termination codon at the target locus. See, for example, Figure 1B.

As another example, the donor polynucleotide may comprise a nucleic acid sequence encoding an internal ribosome entry site positioned adjacent to the gene of interest. See Figure 2. By an "internal ribosome entry site," or "IRES" it is meant a nucleotide sequence that allows for the initiation of protein translation in the middle of a messenger RNA (mRNA) sequence. For example, when an IRES segment is located between two open reading frames in a bicistronic eukaryotic mRNA molecule, it can drive translation of the downstream protein-coding region independently of the 5'-cap structure bound to the 5' end of the mRNA molecule, i.e. in front of the upstream protein coding region. In such a setup both proteins are produced in the cell. The protein located in the first cistron is synthesized by the cap-dependent initiation approach, while translation initiation of the second protein is directed by the IRES segment located in the intercistronic spacer region between the two protein coding regions. IRESs have been isolated from viral genomes and cellular genomes. Artificially engineered IRESs are also known in the art. Any convenient IRES may be employed in the donor polynucleotide.

Typically, as with the 2A peptide, the gene of interest and IRES will be positioned on the donor polynucleotide so as to provide for uninterrupted expression of the gene at the target locus upon insertion of the gene of interest. For example, it may be desirable to insert the gene of interest into an integration site within the 5' untranslated region (UTR) of the gene at the target locus. In such instances, the IRES would be positioned within the donor polynucleotide such that it is immediately 3' to the gene of interest, and flanking recombination sequences selected that will guide homologous recombination and integration of the gene of interest-IRES cassette to the integration site within the 5' UTR.. See, for example, Figure 2A. As another example, it may be desirable to insert the gene of interest into an integration site within the 3' UTR of the gene at the target locus, i.e. downstream of the stop codon, but upstream of the polyadenylation sequence. In such instances, the IRES would be positioned within the donor polynucleotide such that it is immediately 5' to the gene of interest, and flanking recombination sequences selected that will guide homologous recombination and integration of the IRES-gene of interest cassette to the integration site within the 3' UTR of the gene at the target locus. See, for example, Figure 2B.

As another example, the donor polynucleotide may comprise nucleic acid sequences that configure the gene of interest into an intein-like structure. See Figure 3. By an "intein" it is meant a segment of a polypeptide that is able to excise itself and rejoin the remaining portions of the translated polypeptide sequence (the "exteins") with a peptide bond. In other words, the donor polynucleotide comprises nucleic acid sequences that, when translated, promote excision of the protein encoded by the gene of interest from the polypeptide that is translated from the modified target locus. Inteins may be naturally occurring, i.e. inteins that spontaneously catalyze a protein splicing reaction to excise their own sequences and join the flanking extein sequences, or artificial, i.e. inteins that have been engineered to undergo controllable splicing. Inteins typically comprise an N-terminal splicing region comprising a Cys (C), Ser (S), Ala (A), Gin (Q) or Pro (P) at the most N-terminal position and a downstream TXXH sequence; and a C-terminal splicing region comprising an Asn (N), Gin (Q) or Asp (D) at the most C-terminal position and a His (H) at the penultimate C-terminal position. In addition, a Cys (C), Ser (S), or Thr (T) is located in the +1 position of the extein from which the intein is spliced (-1 and +1 of the extein being defined as the positions immediately N-terminal and C-terminal, respectively, to the intein insertion site). See, for example, the diagram below: 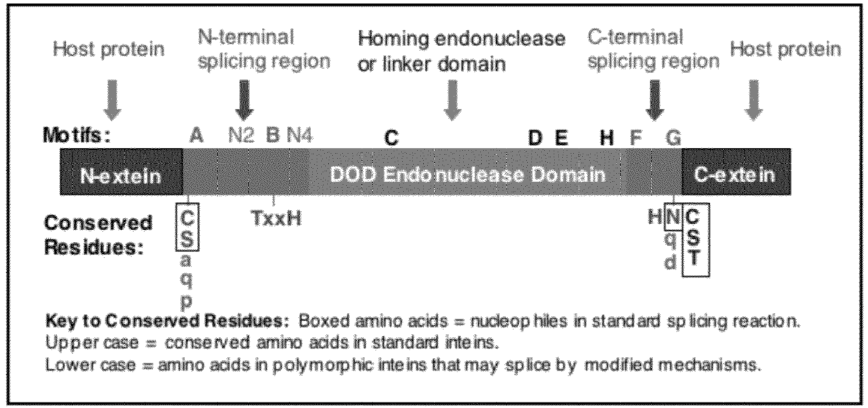 Mechanism by which inteins promote protein splicing and the requirements for intein splicing may be found in Liu, X-Q, "Protein Splicing Intein: Genetic Mobility, Origin, and Evolution" Annual Review of Genetics 2000, 34: 61-76 and in publicly available databases such as, for example, the InBase database on the New England Biolabs website, found on the world wide web at "tools(dot)neb(dot)com/inbase/mech(dot)php", the disclosures of which are incorporated herein by reference. Any sequences, e.g. N-terminal splicing regions and C-terminal splicing regions, known to confer intein-associated excision, be it
18 spontaneous or controlled excision, on a donor polynucleotide, find use in the subject compositions. Genes of interest that are configured as inteins may be inserted at an integration site in any exon of a target locus, i.e. between the start codon and the stop codon of the gene at the target locus. See, e.g. Figure 3.

As another example, the donor polynucleotide may comprise nucleic acid sequences that configure the gene of interest into an intron structure. See Figure 4. By an "intron" it is meant any nucleotide sequence within a gene that is removed by RNA splicing to generate the final mature RNA product of a gene. In other words, the donor polynucleotide comprises nucleic acid sequences that, when transcribed, promote excision of the pre-RNA encoded by the gene of interest from the pre-RNA that is transcribed from the modified target locus, allowing the gene of interest to be translated separately from the mRNA of the target locus. Introns typically comprise a 5' splice site (splice donor), a 3' splice site (spice acceptor) and a branch site. The splice donor includes an almost invariant sequence GU at the 5' end of the intron. The splice acceptor terminates the intron with an almost invariant AG sequence. Upstream (5'-ward) from the splice acceptor is a region high in pyrimidines (C and U) or a polypyrimidine tract. Upstream from the polypyrimidine tract is the branch point, which includes an adenine nucleotide. In addition to comprising these elements, the donor polynucleotide may comprise one or more additional sequences that promote the translation of the mRNA transcribed from the gene of interest, e.g. a Kozak consensus sequence, a ribosomal binding site, an internal ribosome entry site, etc. Genes of interest that are configured as introns may be inserted at an integration site within the transcribed sequence of a target locus anywhere 5' of the nucleic acid sequence that encodes the polyadenylation sequence, e.g. the 3' untranslated region, the coding sequence, or the 5' untranslated region of the gene at the target locus. See, e.g. Figure 4.

As another example, the donor polynucleotide may comprise coding sequence, e.g. cDNA, for the gene at the target locus. Integrating coding sequence for the gene at the target locus into the target locus finds many uses. For example, integrating coding sequence for the gene at the target locus that is downstream, or 3', of the insertion site will ensure that the expression of the gene is not substantially disrupted by the integration of the gene of interest. As another example, it may be desirable to integrate coding sequence for the gene at the target locus so as to express a gene sequence that is a variant from that at the cell's target locus, e.g. if the gene at the cell's target locus is mutant, e.g. to complement a mutant target locus with wild-type gene sequence to treat a genetic disorder. If expression of both the cDNA for the gene at the target locus and the gene of interest are to be regulated by the promoter at the target locus, endogenous gene cDNA sequence and the gene of interest may be provided on the donor polynucleotide as a cassette with a 2A peptide separating the sequences. See, for example, Figure 5A. Alternatively, it may be desirable to express the gene of interest from a separate promoter, e.g. an inducible promoter, or a promoter that is expressed in cells other than those in which the promoter at the target locus is active. In such cases, the gene of interest may be operably linked to a different promoter, and the cDNA sequence placed 5' of the gene of interest on the donor polynucleotide such that it will be operably linked to the promoter at the locus. See, e.g. Figure 5B.

As illustrated by the above example, in some instances, it may be desirable to insert two or more genes of interest, e.g. three or more, 4 or more, or 5 or more genes of interest into a target locus. In such instances, multiple 2A peptides or IRESs may be used to create a bicistronic or multicistronic donor polynucleotide. See, for example, Figure 6A, in which a gene of interest and a selectable marker are integrated into the 3' region of the gene at the target locus, with 2A peptides being used to promote their cleavage from the target polypeptide and from one another. Alternatively, as depicted in Figure 6B, additional coding sequences of interest may be provided on the donor polynucleotide under the control of a promoter distinct from that of the gene at the target locus.

The donor polynucleotide may also comprise sequences, e.g. restriction sites, nucleotide polymorphisms, selectable markers etc., which may be used to assess for successful insertion of the gene of interest at the cleavage site. In addition, the donor polynucleotide may also comprise a vector backbone containing sequences that are not homologous to the DNA region of interest and that are not intended for insertion into the DNA region of interest.

### METHODS

The donor polynucleotides described herein may be used to genetically modify a cell's chromosomal or mitochondrial DNA at any convenient site. Examples of target loci of particular interest for integrating a gene of interest include, without limitation, actin, ADA, albumin, α-globin, β-globin, γ-globin, CD2, CD3, CD5, CD7, E1α, IL2RG, Ins1, Ins2, NCF1, p50, p65, PF4, PGC-γ, PTEN, TERT, UBC, and VWF. Any convenient location within a target locus may be targeted, the donor polynucleotide being configured as described above and the attached figures to provide for targeted integration without disrupting the aforementioned gene.

Donor polynucleotide may be provided to the cells as single-stranded DNA or double-stranded DNA. It may be introduced into a cell in linear or circular form. If introduced in linear form, the ends of the donor polynucleotide may be protected (e.g.from exonucleolytic degradation) by methods known to those of skill in the art. For example, one or more dideoxynucleotide residues are added to the 3' terminus of a linear molecule and/or self-complementary oligonucleotides are ligated to one or both ends. See, for example, Chang et al. (1987) Proc. Natl. Acad Sci USA 84:4959-4963; Nehls et al. (1996) Science 272:886-889. Additional methods for protecting exogenous polynucleotides from degradation include, but are not limited to, addition of terminal amino group(s) and the use of modified internucleotide linkages such as, for example, phosphorothioates, phosphoramidates, and O-methyl ribose or deoxyribose residues. As an alternative to protecting the termini of a linear donor sequence, additional lengths of sequence may be included outside of the regions of homology that can be degraded without impacting recombination.

Donor polynucleotide can be introduced into a cell as part of a vector molecule. Many vectors, e.g. plasmids, cosmids, minicircles, phage, viruses, etc., useful for transferring nucleic acids into target cells are available. The vectors comprising the nucleic acid(s) may be maintained episomally, *e.g.* as plasmids, minicircle DNAs, viruses such cytomegalovirus, adenovirus, *etc.,* or they may be integrated into the target cell genome, through homologous recombination or random integration, *e.g.* retrovirus-derived vectors such as MMLV, HIV-1, ALV, *etc.* The vector molecule may have additional sequences such as, for example, replication origins, promoters and genes encoding antibiotic resistance. Vectors may be provided directly to the subject cells. In other words, the cells are contacted with vectors comprising the donor polynucleotide such that the vectors are taken up by the cells. Methods for contacting cells with nucleic acid vectors that are plasmids, such as electroporation, calcium chloride transfection, and lipofection, are well known in the art. DNA can be introduced as naked nucleic acid, as nucleic acid complexed with an agent such as a liposome or poloxamer, or can be delivered by viruses (e.g., adenovirus, AAV).

For viral vector delivery, the cells may be contacted with viral particles comprising the donor polynucleotide. Retroviruses, for example, lentiviruses, are particularly suitable to the method of the invention. Commonly used retroviral vectors are "defective", *i.e.* unable to produce viral proteins required for productive infection. Rather, replication of the vector requires growth in a packaging cell line. To generate viral particles comprising genes of interest, the retroviral nucleic acids comprising the nucleic acid are packaged into viral capsids by a packaging cell line. Different packaging cell lines provide a different envelope protein (ecotropic, amphotropic or xenotropic) to be incorporated into the capsid, this envelope protein determining the specificity of the viral particle for the cells (ecotropic for murine and rat; amphotropic for most mammalian cell types including human, dog and mouse; and xenotropic for most mammalian cell types except murine cells). The appropriate packaging cell line may be used to ensure that the cells are targeted by the packaged viral particles. Methods of introducing the retroviral vectors comprising the donor polynucleotide into packaging cell lines and of collecting the viral particles that are generated by the packaging lines are well known in the art.

In some embodiments, targeted integration is promoted by the presence of sequences on the donor polynucleotide that are homologous to sequences flanking the integration site. For example, targeted integration using the donor polynucleotides described herein may be achieved following conventional transfection techniques, e.g. techniques used to create gene knockouts or knockins by homologous recombination.

In other embodiments, targeted integration is promoted both by the presence of sequences on the donor polynucleotide that are homologous to sequences flanking the integration site, and by contacting the cells with donor polynucleotide in the presence of a site-specific recombinase. By a site-specific recombinase, or simply a recombinase, it is meant is a polypeptide that catalyzes conservative site-specific recombination between its compatible recombination sites. As used herein, a site-specific recombinase includes native polypeptides as well as derivatives, variants and/or fragments that retain activity, and native polynucleotides, derivatives, variants, and/or fragments that encode a recombinase that retains activity.

For example, a recombinase may be from the Integrase or Resolvase families. The Integrase family of recombinases has over one hundred members and includes, for example, FLP, Cre, lambda integrase, and R. The Integrase family, also referred to as the tyrosine family or the lambda (λ) integrase family, uses the catalytic tyrosine's hydroxyl group for a nucleophilic attack on the phosphodiester bond of the DNA. Typically, members of the tyrosine family initially nick the DNA, which later forms a double strand break. Examples of tyrosine family integrases include Cre, FLP, SSV1, and lambda (λ) integrase. In the resolvase family, also known as the serine recombinase family, a conserved serine residue forms a covalent link to the DNA target site (Grindley, et al., (2006) Ann Rev Biochem 16:16). Examples of resolvases include ϕC31 Int, R4, TP901 -1, A118, ϕFC1, TnpX, and CisA. Other recombination systems include, for example, the SSV1 site-specific recombination system from Sulfolobus shibatae (Maskhelishvili, et al., (1993) Mol Gen Genet 237:334-42); and a retroviral integrase-based integration system (Tanaka, et al., (1998) Gene 17:67-76).

Sometimes the recombinase is one that does not require cofactors or a supercoiled substrate, including but not limited to Cre, FLP, and active derivatives, variants or fragments thereof. FLP recombinase catalyzes a site-specific reaction during DNA replication and amplification of the two-micron plasmid of S. cerevisiae. FLP recombinase catalyzes site-specific recombination between two FRT sites. The FLP protein has been cloned and expressed (Cox, (1993) Proc Natl Acad Sci USA 80:4223-7). Functional derivatives, variants, and fragments of FLP are known (Buchholz, et al., (1998) Nat Biotechnol 16:617-8, Hartung, et al., (1998) J Biol Chem 273:22884-91, Saxena, et al., (1997) Biochim Biophys Acta 1340:187-204, and Hartley, et al., (1980) Nature 286:860-4). The bacteriophage recombinase Cre catalyzes site-specific recombination between two lox sites (Guo, et al., (1997) Nature 389:40-6; Abremski, et al., (1984) J Biol Chem 259:1509-14; Chen, et al., (1996) Somat Cell Mol Genet 22:477-88; Shaikh, et al., (1977) J Biol Chem 272:5695-702; and, Buchholz, et al., (1998) Nat Biotechnol 16:617-8.

Methods for modifying the kinetics, cofactor interaction and requirements, expression, optimal conditions, and/or recognition site specificity, and screening for activity of recombinases and variants are known, see for example Miller, et al., (1980) Cell 20:721-9; Lange-Gustafson and Nash, (1984) J Biol Chem 259:12724-32; Christ, et al., (1998) J Mol Biol 288:825-36; Lorbach, et al., (2000) J Mol Biol 296:1175-81; Vergunst, et al., (2000) Science 290:979-82; Dorgai, et al., (1995) J Mol Biol 252:178-88; Dorgai, et al., (1998) J Mol Biol 277:1059-70; Yagu, et al., (1995) J Mol Biol 252:163-7; Sclimente, et al., (2001) Nucleic Acids Res 29:5044-51; Santoro and Schultze, (2002) Proc Natl Acad Sci USA 99:4185-90; Buchholz and Stewart, (2001) Nat Biotechnol 19:1047-52; Voziyanov, et al., (2002) Nucleic Acids Res 30:1656-63; Voziyanov, et al., (2003) J Mol Biol 326:65-76; Klippel, et al., (1988) EMBO J 7:3983-9; Arnold, et al., (1999) EMBO J 18:1407-14; WO03/08045; WO99/25840; and WO99/25841, the disclosures of which are incorporated herein by reference

A recombinase can be provided via a polynucleotide that encodes the recombinase or it can be stably expressed by the cell. Any recognition site for a recombinase can be used at the integration site and on the donor polynucleotide, including naturally occurring sites and variants. Recognition sites range from about 30 nucleotide minimal sites to a few hundred nucleotides. In some embodiments, the presence of the recombinase will improve the efficiency of integration, for example 2-fold or more, e.g. 3-fold, 4-fold, 5-fold or more, in some instances 10-fold, 20-fold, 50-fold or 100-fold or more over that observed in the absence of the enzyme. For reviews of site-specific recombinases and their recognition sites, see Sauer (1994) Curr Op Biotechnol 5:521-7; and Sadowski, (1993) FASEB 7:760-7.

In other embodiments, targeted integration is promoted both by the presence of sequences on the donor polynucleotide that are homologous to sequences flanking the integration site, and by contacting the cells with donor polynucleotide in the presence of a targeted nuclease. By a "targeted nuclease" it is meant a nuclease that cleaves a specific DNA sequence to produce a double strand break at that sequence. In these aspects of the method, this cleavage site becomes the site of integration for the one or more genes of interest. As used herein, a nuclease includes naturally occurring nucleases as well as recombinant, i.e. engineered, nucleases.

One example of a targeted nuclease that may be used in the subject methods is a zinc finger nuclease or "ZFN". ZFNs are targeted nucleases comprising a nuclease fused to a zinc finger DNA binding domain. By a "zinc finger DNA binding domain" or "ZFBD" it is meant a polypeptide domain that binds DNA in a sequence-specific manner through one or more zinc fingers. A zinc finger is a domain of about 30 amino acids within the zinc finger binding domain whose structure is stabilized through coordination of a zinc ion. Examples of zinc fingers include C₂H₂ zinc fingers, C₃H zinc fingers, and C₄ zinc fingers. A "designed" zinc finger domain is a domain not occurring in nature whose design/composition results principally from rational criteria, e.g. application of substitution rules and computerized algorithms for processing information in a database storing information of existing ZFP designs and binding data. See, for example, U.S. Pat. Nos. 6,140,081; 6,453,242; and 6,534,261; see also WO 98/53058; WO 98/53059; WO 98/53060; WO 02/016536 and WO 03/016496. A "selected" zinc finger domain is a domain not found in nature whose production results primarily from an empirical process such as phage display, interaction trap or hybrid selection. ZFNs are described in greater detail in US Patent No. 7,888,121 and US Patent No. 7,972,854, the complete disclosures of which are incorporated herein by reference. The most recognized example of a ZFN in the art is a fusion of the Fokl nuclease with a zinc finger DNA binding domain.

Another example of a targeted nuclease that finds use in the subject methods is a TAL Nuclease ("TALN", TAL effector nuclease, or "TALEN"). A TALN is a targeted nuclease comprising a nuclease fused to a TAL effector DNA binding domain. By "transcription activator-like effector DNA binding domain", "TAL effector DNA binding domain", or "TALE DNA binding domain" it is meant the polypeptide domain of TAL effector proteins that is responsible for binding of the TAL effector protein to DNA. TAL effector proteins are secreted by plant pathogens of the genus Xanthomonas during infection. These proteins enter the nucleus of the plant cell, bind effector-specific DNA sequences via their DNA binding domain, and activate gene transcription at these sequences via their transactivation domains. TAL effector DNA binding domain specificity depends on an effector-variable number of imperfect 34 amino acid repeats, which comprise polymorphisms at select repeat positions called repeat variable-diresidues (RVD). TALENs are described in greater detail in US Patent Application No. 2011/0145940; in Christian, M et al. (2010) Targeting DNA Double-Strand Breaks with Tal Effector Nucleases. Genetics 186:757-761; and in Li, T. et al. (2010) TAL nucleases (TALNs): hybrid proteins composed of TAL effectors and Fokl DNA-cleavage domain. Nucleic Acids Res. 39(1):359-372; the complete disclosures of which are incorporated herein by reference. The most recognized example of a TALEN in the art is a fusion polypeptide of the Fokl nuclease to a TAL effector DNA binding domain.

Another example of a targeted nuclease that finds use in the subject methods is a targeted Spo11 nuclease, a polypeptide comprising a Spo11 polypeptide having nuclease activity fused to a DNA binding domain, e.g. a zinc finger DNA binding domain, a TAL effector DNA binding domain, etc. that has specificity for a DNA sequence of interest. See, for example, US Application No. 61/555,857, the disclosure of which is incorporated herein by reference.

Other nonlimiting examples of targeted nucleases include naturally occurring and recombinant nucleases, e.g. restriction endonucleases, meganucleases homing endonucleases, and the like.

Typically, targeted nucleases are used in pairs, with one targeted nuclease specific for one sequence of an integration site and the second targeted nuclease specific for a second sequence of an integration site. In the present case, any targeted nuclease(s) that are specific for the integration site of interest and promote the cleavage of an integration site may be used. The targeted nuclease(s) may be stably expressed by the cells. Alternatively, the targeted nuclease(s) may be transiently expressed by the cells, e.g. it may be provided to the cells prior to, simultaneously with, or subsequent to contacting the cells with donor polynucleotide. If transiently expressed by the cells, the targeted nuclease(s) may be provided to cells as DNA, e.g. as described above for the donor polynucleotide. Alternatively, targeted nuclease(s) may be provided to cells as mRNA encoding the targeted nuclease(s), e.g. using well-developed transfection techniques; see, e.g. Angel and Yanik (2010) PLoS ONE 5(7): e11756; Beumer et al. (2008) PNAS 105(50):19821-19826, and the commercially available TransMessenge® reagents from Qiagen, Stemfect™ RNA Transfection Kit from Stemgent, and TransIT®-mRNA Transfection Kit from Mirus Bio LLC. Alternatively, the targeted nuclease(s) may be provided to cells as a polypeptide. Such polypeptides may optionally be fused to a polypeptide domain that increases solubility of the product, and/or fused to a polypeptide permeant domain to promote uptake by the cell. The targeted nuclease(s) may be produced by eukaryotic cells or by prokaryotic cells, it may be further processed by unfolding, e.g. heat denaturation, DTT reduction, etc. and may be further refolded, using methods known in the art. It may be modified, e.g. by chemical derivatization or by molecular biology techniques and synthetic chemistry, e.g. to so as to improve resistance to proteolytic degradation or to optimize solubility properties or to render the polypeptide more suitable as a therapeutic agent.

Any cell's genome may be modified by the compositions and methods described herein. For example, the cell may be a meiotic cell, a mitotic cell, or a post-mitotic cell. Mitotic and post-mitotic cells of interest in these embodiments include pluripotent stem cells, e.g. ES cells, iPS cells, and embryonic germ cells; and somatic cells, e.g. fibroblasts, hematopoietic cells, neurons, muscle cells, bone cells, vascular endothelial cells, gut cells, and the like, and their lineage-restricted progenitors and precursors. Cells may be from any mammalian species, e.g. murine, rodent, canine, feline, equine, bovine, ovine, primate, human, etc.

Cells may be modified in vitro or in vivo. If modified in vitro, cells may be from established cell lines or they may be primary cells, where "primary cells", "primary cell lines", and "primary cultures" are used interchangeably herein to refer to cells and cells cultures that have been derived from a subject and either modified without significant additional culturing, i.e. modified "ex vivo", e.g. for return to the subject, or allowed to grow *in vitro* for a limited number of passages, i.e. splittings, of the culture. For example, primary cultures are cultures that may have been passaged 0 times, 1 time, 2 times, 4 times, 5 times, 10 times, or 15 times, but not enough times go through the crisis stage. Typically, the primary cell lines of the present invention are maintained for fewer than 10 passages in vitro.

If the cells are primary cells, they may be harvest from an individual by any convenient method. For example, leukocytes may be conveniently harvested by apheresis, leukocytapheresis, density gradient separation, etc., while cells from tissues such as skin, muscle, bone marrow, spleen, liver, pancreas, lung, intestine, stomach, etc. are most conveniently harvested by biopsy. An appropriate solution may be used for dispersion or suspension of the harvested cells. Such solution will generally be a balanced salt solution, *e.g.* normal saline, PBS, Hank's balanced salt solution, *etc.,* conveniently supplemented with fetal calf serum or other naturally occurring factors, in conjunction with an acceptable buffer at low concentration, generally from 5-25 mM. Convenient buffers include HEPES, phosphate buffers, lactate buffers, etc. The cells may be used immediately, or they may be stored, frozen, for long periods of time, being thawed and capable of being reused. In such cases, the cells will usually be frozen in 10% DMSO, 50% serum, 40% buffered medium, or some other such solution as is commonly used in the art to preserve cells at such freezing temperatures, and thawed in a manner as commonly known in the art for thawing frozen cultured cells.

To induced DNA integration in vitro, the donor polynucleotide is provided to the cells for about 30 minutes to about 24 hours, e.g., 1 hour, 1.5 hours, 2 hours, 2.5 hours, 3 hours, 3.5 hours 4 hours, 5 hours, 6 hours, 7 hours, 8 hours, 12 hours, 16 hours, 18 hours, 20 hours, or any other period from about 30 minutes to about 24 hours, which may be repeated with a frequency of about every day to about every 4 days, e.g., every 1.5 days, every 2 days, every 3 days, or any other frequency from about every day to about every four days. The donor polynucleotide may be provided to the subject cells one or more times, e.g. one time, twice, three times, or more than three times, and the cells allowed to incubate with the donor polynucleotide for some amount of time following each contacting event e.g. 16-24 hours, after which time the media is replaced with fresh media and the cells are cultured further.

In cases in which both the donor polynucleotide and a targeted nuclease(s) are provided to the cell, the donor polynucleotide and targeted nuclease(s) may be provided simultaneously, e.g. as two nucleic acid vectors delivered simultaneously, or as a single nucleic acid vector comprising the nucleic acid sequences for both the targeted nuclease(s), e.g. under control of a promoter, and the donor polynucleotide. Alternatively, the donor polynucleotide and targeted nuclease(s) may be provided consecutively, e.g. the donor polynucleotide being provided first, followed by the targeted nuclease(s), etc. or vice versa.

Contacting the cells with the donor polynucleotide may occur in any culture media and under any culture conditions that promote the survival of the cells. For example, cells may be suspended in any appropriate nutrient medium that is convenient, such as Iscove's modified DMEM or RPMI 1640, supplemented with fetal calf serum or heat inactivated goat serum (about 5-10%), L-glutamine, a thiol, particularly 2-mercaptoethanol, and antibiotics, e.g. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors. Conditions that promote the survival of cells are typically permissive of nonhomologous end joining and homologous recombination.

Typically, an effective amount of donor polynucleotide is provided to the cells to promote recombination and integration. An effective amount of donor polynucleotide is the amount to induce a 2-fold increase or more in the number of cells in which integration of the gene of interest in the presence of targeted nuclease(s) is observed relative to a negative control, e.g. a cell contacted with an empty vector. The amount of integration may be measured by any convenient method. For example, the presence of the gene of interest in the locus may be detected by, e.g., flow cytometry. PCR or Southern hybridization may be performed using primers that will amplify the target locus to detect the presence of the insertion. The expression or activity of the integrated gene of interest may be determined by Western, ELISA, testing for protein activity, etc. e.g. 2 hours, 4 hours, 8 hours, 12 hours, 24 hours, 36 hours, 48 hours, 72 hours or more after contact with the donor polynucleotide. As another example, integration may be measured by co-integrating an imaging marker or a selectable marker, and detecting the presence of the imaging or selectable marker in the cells.

Typically, genetic modification of the cell using the subject compositions and methods will *not* be accompanied by disruption of the expression of the gene at the modified locus, i.e. the target locus. In other words, the normal expression of the gene at the target locus is maintained spatially, temporally, and at levels that are substantially unchanged from normal levels, for example, at levels that differ 5-fold or less from normal levels, e.g. 4-fold or less, or 3-fold or less, more usually 2-fold or less from normal levels, following targeted integration of the gene of interest into the target locus.

In some instances, the population of cells may be enriched for those comprising the genetic modification by separating the genetically modified cells from the remaining population. Separation of genetically modified cells typically relies upon the expression of a selectable marker that is co-integrated into the target locus. By a "selectable marker" it is meant an agent that can be used to select cells, e.g. cells that have been targeted by compositions of the subject application. In some instances, the selection may be positive selection; that is, the cells are isolated from a population, e.g. to create an enriched population of cells comprising the genetic modification. In other instances, the selection may be negative selection; that is, the population is isolated away from the cells, e.g. to create an enriched population of cells that do not comprise the genetic modification. Separation may be by any convenient separation technique appropriate for the selectable marker used. For example, if a fluorescent marker has been inserted, cells may be separated by fluorescence activated cell sorting, whereas if a cell surface marker has been inserted, cells may be separated from the heterogeneous population by affinity separation techniques, e.g. magnetic separation, affinity chromatography, "panning" with an affinity reagent attached to a solid matrix, or other convenient technique. Techniques providing accurate separation include fluorescence activated cell sorters, which can have varying degrees of sophistication, such as multiple color channels, low angle and obtuse light scattering detecting channels, impedance channels, etc. The cells may be selected against dead cells by employing dyes associated with dead cells (e.g. propidium iodide). Any technique may be employed which is not unduly detrimental to the viability of the genetically modified cells.

Cell compositions that are highly enriched for cells comprising modified DNA are achieved in this manner. By "highly enriched", it is meant that the genetically modified cells will be 70% or more, 75% or more, 80% or more, 85% or more, 90% or more of the cell composition, for example, about 95% or more, or 98% or more of the cell composition. In other words, the composition may be a substantially pure composition of genetically modified cells.

Genetically modified cells produced by the methods described herein may be used immediately. Alternatively, the cells may be frozen at liquid nitrogen temperatures and stored for long periods of time, being thawed and capable of being reused. In such cases, the cells will usually be frozen in 10% DMSO, 50% serum, 40% buffered medium, or some other such solution as is commonly used in the art to preserve cells at such freezing temperatures, and thawed in a manner as commonly known in the art for thawing frozen cultured cells.

The genetically modified cells may be cultured in vitro under various culture conditions. The cells may be expanded in culture, i.e. grown under conditions that promote their proliferation. Culture medium may be liquid or semi-solid, e.g. containing agar, methylcellulose, etc. The cell population may be suspended in an appropriate nutrient medium, such as Iscove's modified DMEM or RPMI 1640, normally supplemented with fetal calf serum (about 5-10%), L-glutamine, a thiol, particularly 2-mercaptoethanol, and antibiotics, e.g. penicillin and streptomycin. The culture may contain growth factors to which the cells are responsive. Growth factors, as defined herein, are molecules capable of promoting survival, growth and/or differentiation of cells, either in culture or in the intact tissue, through specific effects on a transmembrane receptor. Growth factors include polypeptides and non-polypeptide factors.

Cells that have been genetically modified in this way may be transplanted to a subject for purposes such as gene therapy, e.g. to treat a disease or as an antiviral, antipathogenic, or anticancer therapeutic, for the production of genetically modified organisms in agriculture, or for biological research. The subject may be a neonate, a juvenile, or an adult. Of particular interest are mammalian subjects. Mammalian species that may be treated with the present methods include canines and felines; equines; bovines; ovines; etc. and primates, particularly humans. Animal models, particularly small mammals, e.g. murine, lagomorpha, etc. may be used for experimental investigations.

Cells may be provided to the subject alone or with a suitable substrate or matrix, e.g. to support their growth and/or organization in the tissue to which they are being transplanted. Usually, at least 1x10³ cells will be administered, for example 5x10³ cells, 1x10⁴ cells, 5x10⁴ cells, 1x10⁵ cells, 1 x 10⁶ cells or more. The cells may be introduced to the subject via any of the following routes: parenteral, subcutaneous, intravenous, intracranial, intraspinal, intraocular, or into spinal fluid. The cells may be introduced by injection, catheter, or the like. Examples of methods for local delivery, that is, delivery to the site of injury, include, e.g. through an Ommaya reservoir, e.g. for intrathecal delivery (see e.g. US Patent Nos. 5,222,982 and 5385582, incorporated herein by reference); by bolus injection, e.g. by a syringe, e.g. into a joint; by continuous infusion, e.g. by cannulation, e.g. with convection (see e.g. US Application No. 20070254842, incorporated here by reference); or by implanting a device upon which the cells have been reversably affixed (see e.g. US Application Nos. 20080081064 and 20090196903, incorporated herein by reference).

The number of administrations of treatment to a subject may vary. Introducing the genetically modified cells into the subject may be a one-time event; but in certain situations, such treatment may elicit improvement for a limited period of time and require an on-going series of repeated treatments. In other situations, multiple administrations of the genetically modified cells may be required before an effect is observed. The exact protocols depend upon the disease or condition, the stage of the disease and parameters of the individual subject being treated.

In other aspects of the invention, the donor polynucleotide is employed to modify cellular DNA in vivo. In these in vivo embodiments, the donor polynucleotide is administered directly to the individual. Donor polynucleotide may be administered by any of a number of well-known methods in the art for the administration of nucleic acids to a subject. The donor polynucleotide can be incorporated into a variety of formulations. More particularly, donor polynucleotide of the present invention can be formulated into pharmaceutical compositions by combination with appropriate pharmaceutically acceptable carriers or diluents.

Pharmaceutical preparations are compositions that include one or more donor polynucleotides present in a pharmaceutically acceptable vehicle. "Pharmaceutically acceptable vehicles" may be vehicles approved by a regulatory agency of the Federal or a state government or listed in the U.S. Pharmacopeia or other generally recognized pharmacopeia for use in mammals, such as humans. The term "vehicle" refers to a diluent, adjuvant, excipient, or carrier with which a compound of the invention is formulated for administration to a mammal. Such pharmaceutical vehicles can be lipids, e.g. liposomes, e.g. liposome dendrimers; liquids, such as water and oils, including those of petroleum, animal, vegetable or synthetic origin, such as peanut oil, soybean oil, mineral oil, sesame oil and the like, saline; gum acacia, gelatin, starch paste, talc, keratin, colloidal silica, urea, and the like. In addition, auxiliary, stabilizing, thickening, lubricating and coloring agents may be used. Pharmaceutical compositions may be formulated into preparations in solid, semi-solid, liquid or gaseous forms, such as tablets, capsules, powders, granules, ointments, solutions, suppositories, injections, inhalants, gels, microspheres, and aerosols. As such, administration of the donor polynucleotide can be achieved in various ways, including oral, buccal, rectal, parenteral, intraperitoneal, intradermal, transdermal, intracheal, etc., administration. The active agent may be systemic after administration or may be localized by the use of regional administration, intramural administration, or use of an implant that acts to retain the active dose at the site of implantation. The active agent may be formulated for immediate activity or it may be formulated for sustained release.

For some conditions, particularly central nervous system conditions, it may be necessary to formulate agents to cross the blood-brain barrier (BBB). One strategy for drug delivery through the blood-brain barrier (BBB) entails disruption of the BBB, either by osmotic means such as mannitol or leukotrienes, or biochemically by the use of vasoactive substances such as bradykinin. The potential for using BBB opening to target specific agents to brain tumors is also an option. A BBB disrupting agent can be co-administered with the therapeutic compositions of the invention when the compositions are administered by intravascular injection. Other strategies to go through the BBB may entail the use of endogenous transport systems, including Caveolin-1 mediated transcytosis, carrier-mediated transporters such as glucose and amino acid carriers, receptor-mediated transcytosis for insulin or transferrin, and active efflux transporters such as p-glycoprotein. Active transport moieties may also be conjugated to the therapeutic compounds for use in the invention to facilitate transport across the endothelial wall of the blood vessel. Alternatively, drug delivery of therapeutics agents behind the BBB may be by local delivery, for example by intrathecal delivery, e.g. through an Ommaya reservoir (see e.g. US Patent Nos. 5,222,982 and 5385582, incorporated herein by reference); by bolus injection, e.g. by a syringe, e.g. intravitreally or intracranially; by continuous infusion, e.g. by cannulation, e.g. with convection (see e.g. US Application No. 20070254842, incorporated here by reference); or by implanting a device upon which the agent has been reversably affixed (see e.g. US Application Nos. 20080081064 and 20090196903, incorporated herein by reference).

Typically, an effective amount of donor polynucleotide is provided. As discussed above with regard to ex vivo methods, an effective amount or effective dose of a donor polynucleotide in vivo is the amount to induce a 2-fold increase or more in the number of cells in which recombination between the donor polynucleotide and the target locus can be observed relative to a negative control, e.g. a cell contacted with an empty vector or irrelevant polypeptide. The amount of recombination may be measured by any convenient method, e.g. as described above and known in the art. The calculation of the effective amount or effective dose of a donor polynucleotide to be administered is within the skill of one of ordinary skill in the art, and will be routine to those persons skilled in the art. Needless to say, the final amount to be administered will be dependent upon the route of administration and upon the nature of the disorder or condition that is to be treated.

The effective amount given to a particular patient will depend on a variety of factors, several of which will differ from patient to patient. A competent clinician will be able to determine an effective amount of a therapeutic agent to administer to a patient to halt or reverse the progression the disease condition as required. Utilizing LD₅₀ animal data, and other information available for the agent, a clinician can determine the maximum safe dose for an individual, depending on the route of administration. For instance, an intravenously administered dose may be more than an intrathecally administered dose, given the greater body of fluid into which the therapeutic composition is being administered. Similarly, compositions which are rapidly cleared from the body may be administered at higher doses, or in repeated doses, in order to maintain a therapeutic concentration. Utilizing ordinary skill, the competent clinician will be able to optimize the dosage of a particular therapeutic in the course of routine clinical trials.

For inclusion in a medicament, the donor polynucleotide may be obtained from a suitable commercial source. As a general proposition, the total pharmaceutically effective amount of the donor polynucleotide administered parenterally per dose will be in a range that can be measured by a dose response curve.

Donor polynucleotide-based therapies, i.e. preparations of donor polynucleotide to be used for therapeutic administration, must be sterile. Sterility is readily accomplished by filtration through sterile filtration membranes (e.g., 0.2 µm membranes). Therapeutic compositions generally are placed into a container having a sterile access port, for example, an intravenous solution bag or vial having a stopper pierceable by a hypodermic injection needle. The donor polynucleotide-based therapies may be stored in unit or multi-dose containers, for example, sealed ampules or vials, as an aqueous solution or as a lyophilized formulation for reconstitution. As an example of a lyophilized formulation, 10-mL vials are filled with 5 ml of sterile-filtered 1% (w/v) aqueous solution of compound, and the resulting mixture is lyophilized. The infusion solution is prepared by reconstituting the lyophilized compound using bacteriostatic Water-for-Injection.

Pharmaceutical compositions can include, depending on the formulation desired, pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation can include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

The composition can also include any of a variety of stabilizing agents, such as an antioxidant for example. When the pharmaceutical composition includes a polypeptide, the polypeptide can be complexed with various well-known compounds that enhance the in vivo stability of the polypeptide, or otherwise enhance its pharmacological properties (e.g., increase the half-life of the polypeptide, reduce its toxicity, enhance solubility or uptake). Examples of such modifications or complexing agents include sulfate, gluconate, citrate and phosphate. The nucleic acids or polypeptides of a composition can also be complexed with molecules that enhance their in vivo attributes. Such molecules include, for example, carbohydrates, polyamines, amino acids, other peptides, ions (e.g., sodium, potassium, calcium, magnesium, manganese), and lipids.

Further guidance regarding formulations that are suitable for various types of administration can be found in Remington's Pharmaceutical Sciences, Mace Publishing Company, Philadelphia, Pa., 17th ed. (1985). For a brief review of methods for drug delivery, see, Langer, Science 249:1527-1533 (1990).

The pharmaceutical compositions can be administered for prophylactic and/or therapeutic treatments. Toxicity and therapeutic efficacy of the active ingredient can be determined according to standard pharmaceutical procedures in cell cultures and/or experimental animals, including, for example, determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapies that exhibit large therapeutic indices are preferred.

The data obtained from cell culture and/or animal studies can be used in formulating a range of dosages for humans. The dosage of the active ingredient typically lines within a range of circulating concentrations that include the ED50 with low toxicity. The dosage can vary within this range depending upon the dosage form employed and the route of administration utilized.

The components used to formulate the pharmaceutical compositions are preferably of high purity and are substantially free of potentially harmful contaminants (e.g., at least National Food (NF) grade, generally at least analytical grade, and more typically at least pharmaceutical grade). Moreover, compositions intended for in vivo use are usually sterile. To the extent that a given compound must be synthesized prior to use, the resulting product is typically substantially free of any potentially toxic agents, particularly any endotoxins, which may be present during the synthesis or purification process. Compositions for parental administration are also sterile, substantially isotonic and made under GMP conditions.

The effective amount of a therapeutic composition to be given to a particular patient will depend on a variety of factors, several of which will differ from patient to patient. A competent clinician will be able to determine an effective amount of a therapeutic agent to administer to a patient to halt or reverse the progression the disease condition as required. Utilizing LD50 animal data, and other information available for the agent, a clinician can determine the maximum safe dose for an individual, depending on the route of administration. For instance, an intravenously administered dose may be more than an intrathecally administered dose, given the greater body of fluid into which the therapeutic composition is being administered. Similarly, compositions which are rapidly cleared from the body may be administered at higher doses, or in repeated doses, in order to maintain a therapeutic concentration. Utilizing ordinary skill, the competent clinician will be able to optimize the dosage of a particular therapeutic in the course of routine clinical trials.

### UTILITY

The compositions and methods disclosed herein find use in any in vitro or in vivo application in which it is desirable to express one or more genes of interest in a cell in the same spatially and temporally restricted pattern as that of a gene at a target locus while maintaining the expression of the endogenous gene at that target locus.

For example, the subject methods and compositions may be used to treat a disorder, a disease, or medical condition in a subject. Towards this end, the one or more genes of interest to be integrated into a cellular genome may include a gene that encodes for a therapeutic agent. By a "therapeutic agent" it is meant an agent, e.g. siRNA, shRNA, miRNA, CRISPRi agents, peptide, polypeptide, suicide gene, etc. that has a therapeutic effect upon a cell or an individual, for example, that promotes a biological process to treat a medical condition, e.g. a disease or disorder. The terms "individual," "subject," "host," and "patient," are used interchangeably herein and refer to any mammalian subject for whom diagnosis, treatment, or therapy is desired, particularly humans. The terms "treatment", "treating" and the like are used herein to generally mean obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment" as used herein covers any treatment of a disease in a mammal, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease.

Examples of therapeutic agents that may be integrated into a cellular genome using the subject methods and compositions include agents, i.e. siRNAs, shRNAs, miRNAs, CRISPRi agents, peptides, or polypeptides, which alter cellular activity. Other examples of therapeutic agents that may be integrated using the subject methods and compositions include suicide genes, i.e. genes that promote the death of cells in which the gene is expressed. Non-limiting examples of suicide genes include genes that encode a peptide or polypeptide that is cytotoxic either alone or in the presence of a cofactor, e.g. a toxin such as abrin, ricin A, pseudomonas exotoxin, cholera toxin, diphtheria toxin, Herpes Simplex Thymidine Kinase (HSV-TK); genes that promote apoptosis in cells, e.g. Fas, caspases (e.g. inducible Caspase9) etc.; and genes that target a cell for ADCC or CDC-dependent death, e.g. CD20.

In some instances, the therapeutic agent alters the activity of the cell in which the agent is expressed. In other words, the agent has a cell-intrinsic effect. For example, the agent may be an intracellular protein, transmembrane protein or secreted protein that, when expressed in a cell, will substitute for, or "complement", a mutant protein in the cell. In other instances, the therapeutic agent alters the activity of cells other than cells in which the agent is expressed. In other words, the agent has a cell-extrinsic effect. For example, the integrated gene of interest may encode a cytokine, chemokine, growth factor, hormone, antibody, or cell surface receptor that modulates the activity of other cells.

The subject methods and compositions may be applied to any disease, disorder, or natural cellular process that would benefit from modulating cell activity by integrating a gene of interest. For example, the subject agents and methods find use in treating genetic disorders. Any genetic disorder that results from a single gene defect may be treated by the subject compositions and methods, including, for example, hemophilia, adenosine deaminase deficiency, sickle cell disease, X-Linked Severe Combined Immunodeficiency (SCID-X1), thalassemia, cystic fibrosis, alpha-1 anti-trypsin deficiency, diamond-blackfan anemia, Gaucher's disease, growth hormone deficiency, and the like. As another for example, the subject methods may be used to in medical conditions and diseases in which it is desirable to ectopically express a therapeutic agent, e.g. siRNA, shRNA, miRNA, CRISPRi agent, peptide, polypeptide, suicide gene, etc., to promote tissue repair, tissue regeneration, or protect against further tissue insult, e.g. to promote wound healing; promote the survival of the cell and/or neighboring cells, e.g. in degenerative disease, e.g. neurodegenerative disease, kidney disease, liver disease, etc.; prevent or treat infection, etc.

As one non-limiting example, the subject methods may be used to integrate a gene encoding a neuroprotective factor, e.g. a neurotrophin (e.g. NGF, BDNF, NT-3, NT-4, CNTF), Kifap3, Bcl-xl, Crmp1, Chkβ, CALM2, Caly, NPG11, NPT1, Eef1a1, Dhps, Cd151, Morf412, CTGF, LDH-A, Atl1, NPT2, Ehd3, Cox5b, Tuba1a, γ-actin, Rpsa, NPG3, NPG4, NPG5, NPG6, NPG7, NPG8, NPG9, NPG10, etc., into the genome of neurons, astrocytes, oligodendrocytes, or Schwann cells at a locus that is active in those particular cell types (for example, for neurons, the neurofilament (NF), neuro-specific enolase (NSE), NeuN, or Map2 locus; for astrocytes, the GFAP or S100B locus; for oligodendrocytes and Schwann cells, the GALC or MBP locus). Such methods may be used to treat nervous system conditions and to protect the CNS against nervous system conditions, e.g. neurodegenerative diseases, including, for example, e.g. Parkinson's Disease, Alzheimer's Disease, Huntington's Disease, Amyotrophic Lateral Sclerosis (ALS), Spielmeyer-Vogt-Sjögren-Batten disease (Batten Disease), Frontotemporal Dementia with Parkinsonism, Progressive Supranuclear Palsy, Pick Disease, prion diseases (e.g. Creutzfeldt-Jakob disease), Amyloidosis, glaucoma, diabetic retinopathy, age related macular degeneration (AMD), and the like); neuropsychiatric disorders (e.g. anxiety disorders (e.g. obsessive compulsive disorder), mood disorders (e.g. depression), childhood disorders (e.g. attention deficit disorder, autistic disorders), cognitive disorders (e.g. delirium, dementia), schizophrenia, substance related disorders (e.g. addiction), eating disorders, and the like); channelopathies (e.g. epilepsy, migraine, and the like); lysosomal storage disorders (e.g. Tay-Sachs disease, Gaucher disease, Fabry disease, Pompe disease, Niemann-Pick disease, Mucopolysaccharidosis (MPS) & related diseases, and the like); autoimmune diseases of the CNS (e.g. Multiple Sclerosis, encephalomyelitis, paraneoplastic syndromes (e.g. cerebellar degeneration), autoimmune inner ear disease, opsoclonus myoclonus syndrome, and the like); cerebral infarction, stroke, traumatic brain injury, and spinal cord injury. Other examples of how the subject methods may be used to treat medical conditions are disclosed elsewhere herein, or would be readily apparent to the ordinarily skilled artisan.

As another example, the subject methods and compositions may be used to follow cells of interest, e.g. cells comprising an integrated gene of interest. As such, the gene of interest (or one of the genes of interest) to be integrated may encode for a imaging marker. By an "imaging marker" it is meant a non-cytotoxic agent that can be used to locate and, optionally, visualize cells, e.g. cells that have been targeted by compositions of the subject application. An imaging moiety may require the addition of a substrate for detection, e.g. horseradish peroxidase (HRP), β-galactosidase, luciferase, and the like. Alternatively, an imaging moiety may provide a detectable signal that does not require the addition of a substrate for detection, e.g. a fluorophore or chromophore dye, e.g. Alexa Fluor 488® or Alexa Fluor 647®, or a protein that comprises a fluorophore or chromophore, e.g. a fluorescent protein. As used herein, a fluorescent protein (FP) refers to a protein that possesses the ability to fluoresce (i.e., to absorb energy at one wavelength and emit it at another wavelength). For example, a green fluorescent protein (GFP) refers to a polypeptide that has a peak in the emission spectrum at 510 nm or about 510 nm. A variety of FPs that emit at various wavelengths are known in the art. FPs of interest include, but are not limited to, a green fluorescent protein (GFP), yellow fluorescent protein (YFP), orange fluorescent protein (OFP), cyan fluorescent protein (CFP), blue fluorescent protein (BFP), red fluorescent protein (RFP), far-red fluorescent protein, or near-infrared fluorescent protein and variants thereof.

As another example, the subject methods and compositions may be used to isolate cells of interest, e.g. cells comprising an integrated gene of interest. Towards this end, the gene of interest (or one of the genes of interest) to be integrated may encode for a selectable marker. By a "selectable marker" it is meant an agent that can be used to select cells, e.g. cells that have been targeted by compositions of the subject application. In some instances, the selection may be positive selection; that is, the cells are isolated from a population, e.g. to create an enriched population of cells comprising the genetic modification. In other instances, the selection may be negative selection; that is, the population is isolated away from the cells, e.g. to create an enriched population of cells that do not comprise the genetic modification. Any convenient selectable marker may be employed, for example, a drug selectable marker, e.g. a marker that prevents cell death in the presence of drug, a marker that promotes cell death in the presence of drug, an imaging marker, etc.; an imaging marker that may be selected for using imaging technology, e.g. fluorescence activated cell sorting; a polypeptide or peptide that may be selected for using affinity separation techniques, e.g. fluorescence activated cell sorting, magnetic separation, affinity chromatography, "panning" with an affinity reagent attached to a solid matrix, etc.; and the like.

In some instances, the gene of interest may be conjugated to a coding domain that modulates the stability of the encoded protein, e.g. in the absence/presence of an agent, e.g. a cofactor or drug. Non-limiting examples of destabilizing domains that may be used include a mutant FRB domain that is unstable in the absence of rapamycin-derivative C20-MaRap (Stankunas K, et al. (2003) Conditional protein alleles using knockin mice and a chemical inducer of dimerization. Mol Cell. 12(6):1615-24); an FKBP12 mutant polypeptide that is metabolically unstable in the absence of its ligand Shield-1 (Banaszynski LA, et al. (2006) A rapid, reversible, and tunable method to regulate protein function in living cells using synthetic small molecules. Cell.126(5):995-1004); a mutant E. coli dihydrofolate reductase (DHFR) polypeptide that is metabolically unstable in the absence of trimethoprim (TMP) (Mari Iwamoto, et al. (2010) A general chemical method to regulate protein stability in the mammalian central nervous system. Chem Biol. 2010 September 24; 17(9): 981-988); and the like.

As discussed above, any gene of interest may be integrated into a target locus, for example, any gene encoding an siRNA, shRNA, miRNA, CRISPRi element, peptide, or polypeptide may be integrated. Additionally, as discussed above, more than one gene of interest may be integrated, for example, two or more genes of interest may be integrated, three or more genes may be integrated, four or more genes may be integrated, e.g. five or more genes may be integrated. Thus, for example, a therapeutic gene and an imaging marker may be integrated; a therapeutic gene and a selectable marker may be integrated, an imaging marker and a selectable marker may be integrated, a therapeutic gene, an imaging marker and a selectable marker may be integrated, and so forth.

Integrating one or more genes of interest into cellular DNA such that it is expressed in a spatially and temporally restricted pattern without disrupting other cellular activities finds use in many fields, including, for example, gene therapy, agriculture, biotechnology, and research. For example, such modifications are therapeutically useful, e.g. to treat a genetic disorder by complementing a genetic mutation in a subject with a wild-type copy of the gene; to promote naturally occurring processes, by promoting/augmenting cellular activities (e.g. promoting wound healing for the treatment of chronic wounds or prevention of acute wound or flap failure, by augmenting cellular activities associated with wound healing); to modulate cellular response (e.g. to treat diabetes mellitus, by providing insulin); to express antiviral, antipathogenic, or anticancer therapeutics in subjects, e.g. in specific cell populations or under specific conditions, etc. Other uses for such genetic modifications include in the induction of induced pluripotent stem cells (iPSCs), e.g. to produce iPSCs from an individual for diagnostic, therapeutic, or research purposes; in the production of genetically modified organisms, for example in manufacturing for the large scale production of proteins by cells for therapeutic, diagnostic, or research purposes; in agriculture, e.g. for the production of improved crops; or in research, e.g. for the study of animal models of disease.

### REAGENTS, DEVICES AND KITS

Also provided are reagents, devices and kits thereof for practicing one or more of the above-described methods. The subject reagents, devices and kits thereof may vary greatly. Reagents and devices of interest may include donor polynucleotide compositions, e.g. a vector comprising a nucleic acid sequence of interest to be inserted at a target locus and elements, e.g. 2A peptide(s), IRES(s), intein or intronic sequences, and/or flanking recombination sequences that will promote integration without disrupting expression of the target locus, or, e.g. a vector comprising a cloning site, e.g. a multiple cloning site, and elements, e.g. 2A peptide(s), IRES(s), intein or intronic sequences, and/or flanking recombination sequences into which a nucleic acid sequence to be integrated into a target locus may be cloned to generate a donor polynucleotide. Other non-limiting examples of reagents include targeted nuclease compositions, e.g. a target nuclease or pair of targeted nucleases specific for the integration site of interest; reagents for selecting cells genetically modified with the integrated gene of interest; and positive and negative control vectors or cells comprising integrated positive and/or negative control sequences for use in assessing the efficacy donor polynucleotide compositions in cells, etc.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### EXAMPLE 1

### Targeting 2A-fusions to endogenous genes.

2A-peptides allow the translation of multiple proteins from a single mRNA by inducing ribosomal skipping. TALENs were used to induce the targeting of transgenes fused to 2A peptides just 3' to endogenous reading frames (Figure 1C). This approach has several advantages over the common use of expression cassettes including promoter and terminator. First, as the transgene does not bring with it any promoter, the chance of off-target oncogene activation is diminished. The transgene is not expressed from the vector but only if and when integrated in-frame downstream to an endogenous promoter. This happens essentially only if integration by homologous recombination is induced at the intended target. Importantly, once integrated, the expression of the transgene is co-regulated with that of the endogenous gene at the levels of transcription, splicing, nuclear export, RNA silencing and translation. While the endogenous gene product ends up having approximately 20 additional C-terminal amino (the 2A peptide) acids, expression and activity are otherwise preserved.

2A-fusion targeting in various domains may be used in a number of applications, including: 1) Cancer immunotherapy, for example, targeting of a chimeric antigen receptor 2A-fusion to the CD2 T-cell specific cell adhesion molecule for the treatment of CLL; 2) Hemophilia gene therapy, for example, targeting of a coagulation factor 9 2A-fusion to the highly expressed Alb gene; and 3) Generation of animal models, for example, the design of a transgenic mouse carrying fluorescent and luminescent markers 2A-fused to the telomerase gene to allow the monitoring of differentiation, oncogenesis, metastasis, aging and more.

### EXAMPLE 2

### Zinc-Finger Nuclease and TAL Effector Nuclease Mediated Safe Harbor Gene Addition without Safe Harbor Gene Disruption in Mouse Primary Fibroblasts.

Nuclease-mediated safe harbor gene addition strategies are promising as next generation gene therapy technology. Heretofore, "safe harbors" have been defined as loci that can be disrupted without physiologic consequence and which carry no oncogenic potential when disrupted. In this study, homologous recombination-mediated safe harbor targeting does not require disruption of the endogenous gene product. In short, DNA which results in the same amino acid sequence as the target locus, but is non-homologous to the target locus by modification of the wobble position within multiple codons, can be targeted in-frame to result in no protein deficiency from the safe harbor.

To demonstrate the feasibility of this strategy, a previously described GFP reporter assay was used (Connelly et al Mol Ther 2010). In this assay, a GFP gene which carries an insertional mutation that renders the protein non-functional was knocked-into the mouse ROSA26 locus. For gene addition, a donor plasmid containing arms of homology to the GFP gene surround the desired "gene of interest" to be added to the genome. Importantly, 5' to the "gene of interest", we include a non-homologous sequence of DNA which codes for the completion of the C-terminus of GFP. Either Zinc-finger nucleases or TAL effector nucleases specific for the GFP locus were co-transfected with this donor resulting in a gene addition event that restores GFP expression.

We designed multiple donor plasmids with these GFP elements and included as our "gene of interest" the Ubc promoter driving human growth hormone (hGH) cDNA an array of multiple hGH genes linked by 2A peptides, or ΔNGFR, a surface selectable marker that was targeted in-frame with GFP by a 2A peptide without the use of an exogenous promoter. Targeting frequencies ranged from 0.04-1.9% in primary fibroblasts depending on the donor construct or nucleases used, and targeting events were selectable by sorting for GFP or the surface marker ΔNGFR. Transgene (hGH) expression was quantitated by ELISA (6.5-19.3 ng per million cells per 24 hours). We directly compared the ability of zinc-finger nucleases or TAL effector nucleases to stimulate targeting at the same site, and found that TALENs markedly improved the efficiency of targeting over ZFNs (5 fold) with a simultaneous decrease in associated cellular toxicity.We also observed that targeting multiple copies of a transgene linked with the 2A peptide increases expression after targeting and that targeted fibroblasts could be re-introduced subcutaneously into either an isogenic recipient mouse or mouse model of growth hormone deficiency for at least 10 days.

The impact of the targeting system described here is two-fold. First, gene addition in a safe harbor locus can now be studied with virtually any gene of interest in any primary cell type with an easily assayable and quantifiable GFP reporter. Importantly, the restoration of GFP is specific for targeting events only. This is not the case with any other reporter for gene addition described to date. Secondly, the system described here provides proof of principle for an evolution in safe harbor gene addition technology where the disruption of the target locus gene product is no longer required.

### EXAMPLE 3

### Integrating multiple genes at the CCR5 locus to stack genetic resistance to HIV.

One of the major challenges in developing therapeutics for HIV is the virus's ability to mutate and thereby evade therapy. The recent demonstration that zinc finger nucleases (ZFNs) can be used to mutate the CCR5 gene to create a population of HIV resistant T-cells or hematopoietic stem cells, phenotypically mimicking the CCR5 D32 allele, raises the possibility that precision genome engineering can be used to modify the course of HIV infection. The potential weakness of this approach is that in a patient infected with both CXCR4 and CCR5 tropic virus, simply mutating CCR5 in a fraction of T-cells probably will not be sufficient to alter the course of the disease. Instead, cells that are multiply genetically resistant to HIV need to be created. One method to safely and robustly stack genetic resistance to infection is by using ZFN-mediated homologous recombination to target a cocktail of anti-HIV factors to the CCR5 locus.

First, we targeted a GFP cassette to the CCR5 locus, using ZFNs delivered either by DNA or mRNA and achieved a targeting frequency of up to 27% without selection. Next, we chose three restriction factors that inhibit the replication cycle of HIV at three different stages and targeted combinations of these factors to the CCR5 locus in a T-cell reporter line. Using a fluorescence-based, quantitative readout of HIV infection, we identified combinations of factors that provide robust resistance to infection by CCR5-tropic and CXCR4-tropic HIV *in vitro.* Against an R5-tropic lab strain virus, CCR5 disruption alone confers 15-fold protection, but has no effect against an X4-tropic lab strain virus. Chimeric human-rhesus TRIM5a, APOBEC3G D128K, or rev M10 alone targeted to CCR5 provides effective resistance to both lab strain variants (between 2- and 260-fold protection). The combination of all three factors targeted to CCR5 confers 250-fold resistance to R4 tropic virus and 450-fold resistance to R5 tropic virus.

In summary, by using gene targeting we can create cells that are highly resistant to both CXCR4 and CCR5 tropic virus. This strategy may be the foundation for the next generation of gene therapy clinical trials to cure patients of AIDS.

### EXAMPLE 4

### Homologous-recombination mediated genome editing at the adenosine deaminase (ADA) locus in patient-derived fibroblasts using TAL effector nucleases.

Gene therapy, or the ability to correct diseases at the DNA level, has long been a goal of science and medicine. Unfortunately, early gene therapy trials using retroviral vectors to insert genes of interest resulted in insertional oncogenesis. Targeted insertion of the gene of interest through homologous recombination is a safer alternative to viral insertion of a gene.

To insert a gene of interest into the adenosine deaminase (ADA) locus, we developed 2 pairs of TAL effector nucleases (TALENs) specific to sites in exon 1 of the adenosine deaminase (ADA) locus. One cut-site is centered 77bp upstream of the ADA translational start ATG, while the other is centered 27bp downstream of the ATG. These TALENs can stimulate mutagenic repair at their target sites at a rate of 15-25% of alleles in K562 cells. We created donor templates that contained arms of homology centered at the ATG start site, with a variety of DNA fragments inserted in-frame between the arms, including the full cDNA of GFP and ADA, each connected by the t2A ribosomal skip peptide (a 2A peptide sequence from Thosea asigna virus) to cDNA for P140K MGMT (allowing for subsequent selection either *in vitro* or *in vivo*)*.* The in-frame targeted cDNA insertions allow these genes to be regulated by the endogenous ADA promoter.

Flow cytometry was used to demonstrate integration of the desired DNA fragment into the genome when donor templates were transfected along with expression plasmids encoding our TALENs, as opposed to those cells transfected with the donor alone. PCR was then used to show that site-specific insertion of these DNA fragments occur in the presence of the donor plasmid and TALENs, but are undetectable in the cells transfected with the donor plasmid alone. Treatment with O6BG and BCNU enriched for our targeted cells, demonstrating that our targeted cells express the complete construct from the endogenous promoter.

The same experiments were then carried out in patient ADA-deficient patient-derived fibroblasts. Using flow cytometry, we observed increased integration of our constructs when cells were transfected with both the donor plasmid and TALENs, as opposed to the donor alone. Targeted integration of our constructs to exon 1 of the ADA locus was confirmed in these patient-derived cells by PCR. It is expected that ADA enzymatic activity in those cells where ADA cDNA was inserted into exon 1 of ADA-deficient cells will be rescued to substantially wild-type levels.

We have demonstrated that we can achieve targeted insertion by homologous recombination of our constructs in both K562 and patient-derived fibroblast cells. We are also able to enrich for our targeted events through the use of a selectable marker. Furthermore, we have demonstrated site-specific integration of ADA cDNA into exon 1 in patient-derived cells, which allows the full-length ADA protein to be expressed under the endogenous promoter, thereby correcting the phenotype of any ADA mutation.

### EXAMPLE 5

### Gene Targeting of the Human Globin Loci using Engineered Nucleases.

Sickle cell disease is caused by a point mutation in beta-globin, resulting in the substitution of a hydrophobic valine for the hydrophilic glutamic acid at position 6, leading to the pathologic polymerization of mutated hemoglobin molecules. Much of the current pharmacological treatment for patients with sickle cell disease seeks to increase the production of gamma-globin, which can replace mutated beta-globin subunits to form nondefective fetal hemoglobin. Nuclease-mediated homologous recombination was used to target therapeutic beta-globin cDNA to the endogenous beta-globin locus. Gene targeting of the beta-globin and gamma-globin locus was also used to create a cell line that reports on the activity of each of these genes.

Tal-effector nucleases (TALENs) are designed proteins that induce DNA double-strand breaks in a sequence specific manner. Using a Golden Gate synthesis strategy, we engineered a pair of TALENs that cleave the human beta-globin locus just 3' to the site of the sickle mutation. As evidenced by a Cel-I assay, these nucleases created mutations at their target site in HEK-293T cells in 27% of alleles. These TALENs stimulated targeted integration of a GFP cassette into the beta-globin locus by homologous recombination in 23% of K562 cells without selection. Using a similar approach, we designed TALENs to target the human gamma-globin gene. The gamma-globin TALENs created mutations in ∼44% of their target sites as determined by the Cel-I assay, and stimulated targeted gene addition of the tdTomato gene to the gamma-globin site in 35% of cells. Using these nucleases we created cell lines that contain both GFP under the control of the endogenous beta-globin promoter and tdTomato under the control of the endogenous gamma-globin promoter. We are using this doubly tagged cell line to quantify the differential effect of small molecules on the activity of the two genes.

In addition to targeting GFP to the initiation ATG of the beta-globin gene, we have used a novel strategy to target the full beta-globin cDNA in-frame to the beta-globin start site followed by a P140K MGMT selection cassette. We have used this strategy to enrich for targeted cells with the drug combination 6-benzylguanine and carmustine *in vitro;* this selection system can also be used to select for targeted cells *in vivo.* After four rounds of *in vitro* selection, >80% of cells were targeted as determined by a novel deep sequencing approach to measuring targeting efficiency. This combination of nucleases and targeting vector could be used as a potential therapeutic for the treatment of both sickle cell disease and beta-thalassemia.

### EXAMPLE 6

### Engineered Nuclease Mediated Gene Targeting of the Human IL2Rγ Gene.

X-Linked Severe Combined Immunodeficiency (X-SCID) is a genetic disorder caused by mutations in the interleukin 2 receptor gamma chain (IL2Rγ) gene, which forms part of the receptor for interleukins IL-2, IL-4, IL-7, IL-9, IL-15, & IL-21. A non-functional IL2Ry gene product results in extensive defects in interleukin signaling that cripple the ability of lymphocytes to differentiate into functional T-cells, B-cells, and natural killer cells, resulting in a devastating lack of an adaptive immune system. Without successful bone marrow transplantation patients usually die in the first year of life as a result of severe infections.

Our goal is to use transcription activator-like effector nucleases (TALENs) to stimulate gene addition of IL2Ry cDNA in X-SCID patient-derived cells. TALENs create site-specific double-strand breaks (DSBs) in DNA that can be repaired via homologous recombination with a donor DNA template, resulting in correction of the endogenous gene or addition of new genetic sequences. For a specific patient the simplest form of gene therapy would be the direct correction of their disease-causing mutation. A significant drawback of this approach is that treatment of X-SCID patients with diverse mutations spread throughout the gene would necessitate development of many different pairs of nucleases and donor DNA templates, each of which could have different efficacy and toxicity profiles. Targeting of full IL2Rγ cDNA to Exon 1 could potentially bypass this problem and allow for a single gene targeting strategy that would be therapeutic for almost all X-SCID patients.

We developed pairs of TALENs targeting sequences immediately upstream of the IL2Ry start codon. All TALEN pairs designed with an optimal spacer length were highly active at creating DSBs at the endogenous target, generating mutations in 30-40% of alleles in a K562 cell line. Interestingly, the effect of varying spacer length is clearly seen with these highly active TALENs as every combination with sub-optimal or non-optimal spacer lengths showed decreased activity or no activity, respectively. When a donor DNA template containing a Ubc-eGFP insert was transfected with the most active TALEN pair, integration of Ubc-eGFP was seen in 22% of cells, compared to a background level of 1-2% integration with the Ubc-eGFP donor alone.

Preliminary data in X-SCID patient-derived lymphoblastoid cell lines from multiple patients show TALEN-mediated integration of Ubc-eGFP, and experiments targeting full IL2Ry cDNA to IL2Ry Exon 1 are ongoing. The results of these experiments illustrate the potential of using a single gene targeting strategy to produce endogenously regulated, wild-type levels of functional protein in patient cells with diverse disease-causing mutations. Using TALENs to stimulate gene addition in an *ex vivo* population of patient-derived cells could represent a treatment strategy for X-SCID and other monogenic diseases that restores wild-type gene function at the endogenous locus without stimulating oncogenic transformation.

### EXAMPLE 7

### Targeted integration of growth factors in fibroblasts to promote wound healing.

The gene encoding platelet derived growth factor (PDGF-B) was targeted to the ROSA26 locus in mouse fibroblasts (see Example 2, above, and Fig. 20). Fibroblasts modified to comprise an integrated PDGF gene were assayed for their ability to promote wound healing in the mouse model of wound healing by Galiano et al. ((2004) Quantitative and reproducible murine model of excisional wound healing. Wound Rep Regen. 12(4):485-92) (Fig. 22). Lesions transplanted with PDGF-modified fibroblasts demonstrated significantly more healing 14 days after transplantation as compared to lesions transplanted with unmodified fibroblasts.

Thus, genome editing without target gene disruption can be used to engineer cells ex vivo to secrete wound healing growth factors, e.g. PDGF, VEGF, EGF, TGFa, TGBβ, FGF, TNF, IL-1, IL-2, IL-6, IL-8, endothelium derived growth factor, etc. (see, e.g., Fig. 19), which can then be transplanted into an individual to facilitate the healing of an acute or chronic wound. These cells may be autologous, i.e. derived from the individual into which they are being transplanted, or they may be universal, i.e. cells not from the recipient individual. For example, the cells may be fibroblasts, e.g. fibroblasts isolated from an individual, universal fibroblasts, fibroblasts induced from a stem cell, e.g. iPSC. They may be transplanted to the site of a lesion, or to a site elsewhere in the body and allowed to migrate to the lesion site. In addition to the wound healing growth factor, the nucleic acid that is integrated into the target locus may comprise cDNA for the gene at the endogenous locus; and/or a selectable marker, e.g. to select and enrich for the engineered cells; and/or a suicide gene, e.g. to eliminate the engineered cells ones . See, for example, Fig. 7. It will be recognized by the ordinarily skilled artisan that any combination of elements as described herein may be used to achieve healing of the wound.

Fibroblast cell-based therapy may be used in any of a variety of conditions. For example, fibroblast cell-based therapy may be used in the treatment of genetic diseases, e.g. epidermolysis bullosa; as a vehicle for systemic protein delivery, e.g. to deliver clotting factors; as a vehicle for local protein delivery, e.g. to deliver cytokines for wound healing, tissue ischemia, etc. Other applications will be recognized by the ordinarily skilled artisan.

One example for a utility of fibroblast cell-based therapy is to treat chronic wounds, e.g. in diabetes. In 2007, there were 24 million people with diabetes and 54 million with pre-diabetes. In 2001, 6% of patients developed non-healing diabetic ulcers. Currently, 1-3 million people developed new pressure ulcers per year. The contributing factors for such ulcers include ischemia, neuropathy, immobility, poor, nutrition, and infection. Treatment options currently include infection control, surgical debridement and/or soft tissue coverage, re-vascularization, correct nutrition, prevent immobility, negative pressure dressings, and other advanced dressing modalities. As demonstrated in Figures 20-23, expression of cytokines such as PDGF from fibroblasts modified using the methodologies disclosed herein promote wound healing in a mouse model of chronic wound healing. These results demonstrate the utility of fibroblast cell-based therapy in the treatment of diabetic ulcers.

### EXAMPLE 8

Gene therapy is the modification of the nucleic acid content of cells for therapeutic purposes. While early clinical gene therapy successes were limited, in the last five years there have been a number of successful clinical gene therapy trials. These include the restoration of vision to patients with Leber's Congenital Amaurosis (LCA) with an AAV vector (Maguire, A.M., etal., Safety and efficacy of gene transfer for Leber's congenital amaurosis. N Engl J Med, 2008. 358(21): p. 2240-8), the generation of therapeutic factor IX levels from in vivo AAV transduction of liver for hemophilia B (Kay, M.A., et al., Evidence for gene transfer and expression of factor IX in haemophilia B patients treated with an AAV vector. Nat Genet, 2000. 24(3): p. 257-61; Manno, C.S., et al., Successful transduction of liver in hemophilia by AAV-Factor IX and limitations imposed by the host immune response. Nat Med, 2006. 12(3): p. 342-7; Nathwani, A.C., etal., Adenovirus-associated virus vector-mediated gene transfer in hemophilia B. N Engl J Med, 2011. 365(25): p. 2357-65), the remission of leukemia through the lentiviral transduction of T-cells with a chimeric antigen receptor against CD19 (Porter, D.L., etal., Chimeric antigen receptor-modified T cells in chronic lymphoid leukemia. N Engl J Med, 2011. 365(8): p. 725-33), the restoration of a functional immune system by *ex vivo* retroviral transduction of hematopoetic stem and progenitor cells for the primary immunodeficiencies SCID-X1, ADA-SCID, and Wiskott-Aldrich syndrome (WAS) (Aiuti, A., etal., Correction of ADA-SCID by stem cell gene therapy combined with nonmyeloablative conditioning. Science, 2002. 296(5577): p. 2410-3; Blaese, R.M., et al., T lymphocyte-directed gene therapy for ADA- SCID: initial trial results after 4 years. Science, 1995. 270(5235): p. 475-80; Boztug, K., etal., Stem-cell gene therapy for the Wiskott-Aldrich syndrome. N Engl J Med, 2010. 363(20): p. 1918-27; Cavazzana-Calvo, M., etal., Gene therapy of human severe combined immunodeficiency (SCID)-X1 disease. Science, 2000. 288(5466): p. 669-72), and the establishment of transfusion independence of a β-thalassemia patient after the *ex vivo* transduction of hematopoietic stem and progenitor cells with a lentiviral vector (Cavazzana-Calvo, M., et al., Transfusion independence and HMGA2 activation after gene therapy of human beta-thalassaemia. Nature, 2010. 467(7313): p. 318-22).

Serious adverse events have unfortunately occurred, however, in some patients from the activation of a proto-oncogene by the uncontrolled retroviral insertion of the transgene. In the SCID-X1 and WAS trials this was usually the result of the activation of the LMO2 gene (Boztug, K., etal., Stem-cell gene therapy for the Wiskott-Aldrich syndrome. N Engl J Med, 2010. 363(20): p. 1918-27; Hacein-Bey-Abina, S., etal., Insertional oncogenesis in 4 patients after retrovirusmediated gene therapy of SCID-X1. J Clin Invest, 2008. 118(9): p. 3132-42), while in the chronic granulomatous disease trials this resulted from the activation of the ecotropic viral integration site 1 (EVI1) gene (Stein, S., et al., Genomic instability and myelodysplasia with monosomy 7 consequent to EVI1 activation after gene therapy for chronic granulomatous disease. Nat Med, 2010. 16(2): p. 198-204). While frank leukemia or myelodysplasia has not resulted in the β-thalassemia trial, the single reported patient developed a non-malignant clonal expansion from insertional dysregulation of the HMGA2 gene (Cavazzana-Calvo, M., etal., Transfusion independence and HMGA2 activation after gene therapy of human beta-thalassaemia. Nature, 2010. 467(7313): p. 318-22). Currently, genomically safer retroviral and lentiviral vectors are now being tested, it remains unclear whether the therapeutic window between clinical efficacy and risk of insertional dysregulation of oncogenes is wide enough for the approach to be useful as a general approach when the integration of the transgene is necessary.

An alternative approach would be to avoid uncontrolled integrations entirely and instead target the new genetic material precisely to a specified genomic location by homologous recombination. Homologous recombination is a major mechanism that cells use to repair double strand breaks (DSBs). In genome editing, the homologous recombination machinery can be high-jacked by providing a donor template for the cell to use to repair an engineered nuclease-induced DSB. In this way the sequences in the provided donor are integrated in a precise fashion into the genome. In contrast to genome editing mediated by non-homologous end-joining in which random insertions and/or deletions are inserted at a specific genomic location by the repair of a nuclease-induced DSB, an added level of precision is gained in homologous recombination mediated genome editing as defined DNA changes (both large and small) are introduced at a precise location.

The use of homologous recombination for genome editing can be classified into two basic categories. The first is to use homologous recombination to modify directly the therapeutic gene of interest. An example of this approach is to modify the IL2RG locus as an approach to curing SCID-X1 (Lombardo, A., etal., Gene editing in human stem cells using zinc finger nucleases and integrase-defective lentiviral vector delivery. Nat Biotechnol, 2007. 25(11): p. 1298-306; Urnov, F.D., et al., Highly efficient endogenous human gene correction using designed zinc-finger nucleases. Nature, 2005. 435(7042): p. 646-51). This method has the advantage of the transgene being expressed through the endogenous regulatory elements and thus maintaining precise spatial and temporal control of transgene expression. The second is to use homologous recombination to target a transgene to a specific genomic location unrelated to the transgene itself (Benabdallah, B.F., et al., Targeted gene addition to human mesenchymal stromal cells as a cell-based plasma-soluble protein delivery platform. Cytotherapy, 2010. 12(3): p. 394-9; Hockemeyer, D., et al., Efficient targeting of expressed and silent genes in human ESCs and iPSCs using zinc-finger nucleases. Nat Biotechnol, 2009. 27(9): p. 851-7). Ideally the genomic target would be a "safe harbor" defined as a genomic site that when a transgene integrates there would be no change in cellular behavior except that determined by the new transgene. This is a strict functional rather than a bio-informatic or surmised definition of a safe harbor. Given the functional complexity of the genome that contains not only protein coding genes but also an abundance of non-coding RNAs and a plethora of dispersed regulatory elements, it is very difficult to confidently assign any genomic location as a safe harbor, although the ROSA26 locus in mice does seem to qualify. The AAVS1 locus, for example, has been proposed as a safe harbor (Hockemeyer, D., etal., Efficient targeting of expressed and silent genes in human ESCs and iPSCs using zinc-finger nucleases. Nat Biotechnol, 2009. 27(9): p. 851-7) but the disruption of even one allele of the protein phosphatase 1 regulatory subunit 12C gene within which AAVS1 resides may have subtle but important effects on cellular behavior. Safe harbor loci that can be disrupted without physiologic consequence may be, by definition, disconnected from active biologic processes in a manner that limits transgene expression and therapeutic efficacy. The closed chromatin state of an inactive locus may also inhibit optimal nuclease access.

This example describes gene targeting by homologous recombination. In this approach an engineered nuclease, either a zinc finger nuclease (ZFN) or TAL effector nuclease (TALEN), is used to induce a DSB in a safe harbor but the targeting vector is designed such that the modification of the target will not be disrupted after integration. In our proof-of-principle studies we actually simultaneously correct the target locus and insert a transgene. The correction aspect is a convenient but not essential aspect of the targeting strategy. Using this method, virtually any locus in the genome could be used as a safe harbor or be used to drive the expression of a transgene in a temporally and spatially specific manner.

### MATERIALS AND METHODS

**Generation of Gene Addition Constructs.** We constructed the gene addition vector in Figure 27.1 by synthesizing the GFP nucleotides 38-720 (Genscript). Nucleotides 38-303 consist of the published nucleotides, while 304-720 are modified as described in Figure 27.1B. We then subcloned this construct into a pUB6 expression vector (Life Technologies, Grand Island, NY). Using the same plasmid from which we derived the knock-in mouse (Connelly, J.P., etal., Gene correction by homologous recombination with zinc finger nucleases in primary cells from a mouse model of a generic recessive genetic disease. Mol Ther, 2010. 18(6): p. 1103-10), we PCR amplified the 3' homology region with 5'AAGGACGACGGCAACTAC3' (SEQ ID NO:1) and 5'GACGTGCGCTTTTGAAGCGT3' (SEQ ID NO:2) and also subcloned in the pUB6 expression vector. We next PCR amplified the hGH gene (SC300088 Origene, Rockville, MD), and subcloned this into the vector along with a PolyA region. For the multicopy hGH constructs, we performed two PCRs for cloning-the first eliminated the stop codon within hGH and the second fused a Furin-SGSG-T2A sequence (5'CGCAAGCGCCGCAGCGGCAGCGGCGAGGGCCGCGGCAGCCTGCTG ACCTGCGGCGACGTGGAGGAGAACCCCGGCCCC3' (SEQ ID NO:3)) in front of hGH so that when cloned together, the two constructs would be in the same ORF. Serial cloning of these two constructs allowed for generation of multicopy donor vectors. For the ΔNGFR vector, the synthesized construct of GFP 38-720 described above was PCR amplified to eliminate the stop codon and the Furin-SGSG-T2A was fused by PCR to the ΔNGFR construct. Subcloning of these two in-frame resulted in the donor plasmid described in Figure 27.4. All restriction enzymes were ordered from New England Biolabs Inc.

**Generation of ZFNs and TALENs.** The ZFNs described are the same two pairs we have previously published (Connelly, J.P., etal., Gene correction by homologous recombination with zinc finger nucleases in primary cells from a mouse model of a generic recessive genetic disease. Mol Ther, 2010. 18(6): p. 1103-10). The TALENs were designed to recognize TGCCCGAAGGCTACGT (SEQ ID NO:4) on the sense strand and TTGCCGTCGTCCTTGAAG (SEQ ID NO:5) on the anti-sense strand. The spacer between the TALENs is 18 basepairs. Within the TALEN repeats, NN recognizes G, HD recognizes C, NI recognizes A and NG recognizes T. These were cloned into a CMV expression vector along with the wild-type, codon optimized Fokl nuclease domain and contain a 3x FLAG tag.

**Primary Fibroblasts Culture, Transfection, and Gene Addition Analysis.** Primary fibroblasts were isolated from the ears of 3-6 month old mice by 1 hour of digest in collagenase/dispase (4mg/ml) (Roche) and then 1 ml MAF media was then added and cells incubated overnight at 37 degrees. The next morning, cells were triturated, filtered with a 70uM cell strainer (BD Biosciences, San Jose, CA) and then cultured in DMEM, 16% FBS, Pen/Strep, L-Glut, Fungizone and 1X non-essential amino acids. Critically, all cultures were maintained in low oxygen conditions (5%) which drastically improves the survival of the cells and minimizes early senescence. 1x10⁶ cells per sample were nucleofected per sample using the Basic Fibroblast kit (Lonza, Switzerland, Cat. VPI-1002) with program U-23 and analyzed for GFP fluorescence by flow cytometry. Gene addition was confirmed by DIG-Southern (Roche) using an EcoRV digest and a probe designed against the PGK-Neo region at the 3' end of our knockin locus (described in Connelly, J.P., etal., Gene correction by homologous recombination with zinc finger nucleases in primary cells from a mouse model of a generic recessive genetic disease. Mol Ther, 2010. 18(6): p. 1103-10). PCR for gene addition was performed using the following 3 primers:
F: 5'ATGGTGAGCAAGGGCGAGGA3' (SEQ ID NO:6)
R1: 5TTACTTGTACAGCTCGTCCATGCCG3' (SEQ ID NO:7)
R2: 5'TTATTTGTAGAGCTCATCCATTCCGAGGG3' (SEQ ID NO:8)
Growth hormone expression was quantitated by ELISA (ELH-GH-001 RayBiotech, Norcross, GA) by culturing 2 x 10⁴ fibroblasts in 1 ml of media for 24 hours. NFGR selection was performed by staining with magnetic bead conjugated antibodies (130-092-283 MACS kit, Miltenyi Biotec). Cells were resuspended in 2.5ml MACs buffer, then incubated in an Easy Sep (18000 Stemcell Technologies) magnet for 10 minutes in a 5 ml tube. Liquid was briskly poured out of the tube, and then the resuspension and magnetic incubation was repeated. After 3-4 days, selection was repeated.

**Transplantation of Primary Fibroblasts.** For transplantation experiments, fibroblasts underwent gene addition by nucleofection as described above. Cells were analyzed by flow cytometry prior to transplantation and were then injected subcutaneously in a Matrigel (BD Biosciences, San Jose, CA) matrix on the dorsum of either a sibling mouse or an anti-thymocyte serum (Fitzgerald industries) treated unrelated mouse. Mice who received ATS treatment were given 120 mg/kg intraperitoneally over the course of 4 days prior to transplantation for a total of 480 mg/kg. Successful lymphocyte knock down was confirmed with CBC analysis using a HemaVet system (Drew Scientific Waterbury, CT). Of note, we found that in our mice the dose needed for this lot of serum was higher than required by previous studies, suggesting that individual lots should be tested on a per strain basis for efficacy. After 10 or 30 days post-transplantation, the Matrigel plug was excised and then processed in the same manner as the initial fibroblast derivation above. Post-transplant fluorescence was quantitated by flow cytometry, and the percent survival was calculated as percent post-transplant GFP positive normalized to pre-transplant GFP positive. Post-transplant hGH expression was quantitated with ELISA from tissue culture medium 24 hours after harvested Matrigel plugs were plated in tissue culture, as described above.

### RESULTS

**Targeting Growth Hormone cDNA to a Safe Harbor Without Disruption.** A disadvantage to current safe harbor gene addition strategies is that a safe harbor must be identified where targeted insertion and disruption of the locus results in no physiologic perturbation. We sought to design a gene addition strategy that preserved the gene product of the safe harbor. For this purpose, we utilized a knock-in mouse model we have previously described (Connelly, J.P., etal., Gene correction by homologous recombination with zinc finger nucleases in primary cells from a mouse model of a generic recessive genetic disease. Mol Ther, 2010. 18(6): p. 1103-10), to serve as a reporter for gene addition events. Briefly, a mutated, non-fluorescent GFP gene was inserted in the mouse ROSA26 locus in mouse embryonic stem (ES) cells by homologous recombination. We then generated transgenic mice from these targeted mouse ES cells. We chose this model because restoration of the endogenous gene product (GFP) provides a reporter that is entirely specific for a gene addition event.

Current safe harbor gene addition reporter models rely on the integration of a transgene capable of independent expression regardless of the site of insertion. In this strategy, site-specific nucleases along with a donor plasmid containing a full-length transgene and promoter are transfected. After transfection, either targeted or random integration can occur. The efficiency of gene targeting determines the ratio of targeted to random integration. Because expression of the transgene is not dependent on site-specific integration, random integration cannot be conveniently (by flow cytometry for example) distinguished from targeted events. In our model, only site-specific gene addition restores the expression of our reporter and is a more convenient system to study gene addition events.

We designed a donor plasmid which contained a 5' region of homology to the target locus, followed by a non-homologous sequence capable of completing the C terminus of GFP, followed by a transgene, and lastly a 3' region of homology to the target locus (Figure 25A). Critically, we designed the C-terminus of the GFP gene to have multiple wobble mutations which create significant differences at the DNA level but no differences at the protein level. This strategy of creating non-homology serves to prevent cross-over by the homologous recombination machinery prior to the integration of the transgene cassette (Figure 25B). We generated two constructs, one with approximately every 3rd nucleotide modified (64.5% identity) and one with approximately every 6th nucleotide modified (83.5% identity). We found that both were sufficiently different not to be recognized as homology by the homologous recombination machinery and both were capable of restoring GFP expression. In 293T cells, the resultant GFP from both constructs was expressed well enough to be assayed by flow cytometry, however, in primary fibroblasts derived from our mouse model, the 64.5% construct was too dim to reliably distinguish GFP positive cells. We believe the dimness of GFP is the result of having to change multiple codons optimized for expression to codons that are non-optimal for expression in mammalian cells. We did observe a decrease in gene targeting frequency as compared to direct gene correction (Figure 29) but in contrast to a standard gene addition experiment where positive cells reflect both random and targeted integrations, in this system we could easily identify and purify targeted integrants without random integrants. As a result, we proceeded with constructs containing 83.5% GFP identity for the remaining experiments (Figure 25B).

We observed that a construct consisting of two homology arms and our GFP 83.5% construct followed by the Ubiquitin C (Ubc) promoter driving expression of human growth hormone (hGH) cDNA could be targeted in primary fibroblasts derived from our mouse model at a frequency of 0.27% (Figure 25C). The GFP positive cells were purified by FACS (Figure 25C), and analyzed by both Southern blotting and PCR to confirm targeting (Figure 25D and E).

Expression of hGH was confirmed by ELISA to be 15 ng per million GFP positive cells per 24 hours (Figure 25F). This data confirmed that we could generate a donor construct for gene addition that, through modification of the nucleotide sequence to prevent recognition as homology, could maintain (or in this case, restore) safe harbor gene expression after a gene addition event. Further, we established an easily assayable reporter specific for gene addition through GFP restoration that allows for rapid quantification of gene addition frequencies. Maintaining expression of the endogenous gene product (GFP) at the targeting locus provides proof of principle that gene addition can occur by the strategy described without the need for identifying a safe harbor locus that can tolerate disruption.

**Transplantation of Targeted, Growth Hormone Expressing Fibroblasts.** In an ex vivo approach to genetically modifying cells for gene therapy, the stable engraftment of genome-modified cells after transplantation is critical. Thus, we determined whether the engineered fibroblasts generated in Figure 1 could be implanted into a recipient mouse. Fibroblasts were injected subcutaneously in a Matrigel matrix and harvested 10 or 30 days after transplantation. After recovery the populations of cells were analyzed for both GFP expression by flow cytometry and hGH expression by ELISA. In a sibling mouse, 75% of the cells recovered at 10 days after transplantation were GFP positive, normalized to the pre-transplant population. However, after 30 days, 45% remained. These populations secreted 14.4 and 6.5 ng hGH per million cells per 24 hours, respectively. We hypothesized this decrease may be immune-mediated, either because of a response to the human growth hormone peptide or because our knock-in mouse reporter strain is not an isogenic strain and the transplanted cells are not immunologically identical to the recipient mouse. To test the immune mediated clearance hypothesis, fibroblasts were transplanted into an unrelated strain in the presence or absence of anti-mouse thymocyte serum (ATS) (injected intraperitoneally for 4 days prior to transplantation). It was observed that in the absence of ATS, 42% of transplanted cells remained after 10 days, and after 30 days, only 0.04%. These populations secreted 7 and 0.02 ng hGH per million cells per 24 hours, respectively. However, after only one ATS treatment course, 92% of cells remained after 10 days and 56% after 30 days. These cells secreted 17.3 and 12.5 ng hGH per million cells per 24 hours, respectively (Figure 26). These results demonstrate successful re-introduction of gene-modified cells that are capable of persisting in a recipient for at least 30 days. From this data, it could also be demonstrated that GFP expression and hGH expression have a linear relationship with an R2 value of 0.95.

**Targeting Multiple cDNA Copies Increases Transgene Expression.** Random integration of transgenes often occurs by the multimerization of the transgene as an integrated array. The integration of the transgene can result in either decreased expression as the array is silenced or increased expression because there are multiple copies. We determined whether the controlled targeting of multiple copies of a transgene to a single genomic locus would result in increased expression of the transgene. The T2A peptide derived from the insect Thosea asigna virus was used to generate multicistronic vectors. The moiety mediates a ribosomal skipping mechanism which results in linkage and expression of multiple open reading frames (Szymczak, A.L., etal., Correction of multi-gene deficiency in vivo using a single 'selfcleaving' 2A peptide-based retroviral vector. Nat Biotechnol, 2004. 22(5): p. 589-94). Four constructs were generated, each with increasing numbers of the hGH cDNA termed hGH1x, hGH2x, hGH3x, hGH4x (Figure 27A). We found that gene addition could be successfully achieved with all four constructs at a frequency of 0.07%, 0.04%, 0.05%, 0.02% respectively (Figure 27B). Next, we sorted for GFP positive fibroblasts by FACS and analyzed hGH expression by ELISA. We found that between 1-3 copies of hGH, the copy number positively correlated with expression levels (Figure 27C). The expression of 4 repeats (4x), however, was lower than that with fewer repeats. Thus, targeting an array of transgenes linked with a 2A peptide results in a non-linear increase in transgene expression.

**TAL Effector Nucleases are more active and less toxic than Zinc Finger Nucleases.** We used previously described zinc finger nucleases (ZFNs) to target gene addition in Figures 25-27. We then compared TAL effector nucleases (TALENs) designed to target the sequence that overlaps with the sequence targeted by the ZFNs (Figure 29). Using the donor construct described in Figure 1A, we determined that the targeting frequency for TALENs was five times higher than for ZFNs (Figure 28A). In a titration experiment, we found that TALENs had higher targeting frequencies than ZFNs at every amount of nuclease expression plasmid transfected (Figures 28B and 28C). In fact, TALENs were able to stimulate substantial targeting when even very low amounts (0.1 ug) of the TALEN expression constructs were transfected. In our prior work with ZFNs we had seen a "goldilocks" effect in which an optimal amount of ZFN needed to be transfected to obtain maximal targeting frequencies but had never been able to titrate down the amount of ZFN as much as we could with the TALENs (Figures 28B and 28C and (Pruett-Miller, S.M., etal., Comparison of zinc finger nucleases for use in gene targeting in mammalian cells. Mol Ther, 2008. 16(4): p. 707-17; Pruett-Miller, S.M., etal., Attenuation of zinc finger nuclease toxicity by small-molecule regulation of protein levels. PLoS Genet, 2009. 5(2): p. e1000376)).

We compared the toxicity profiles for the ZFN and TALEN pairs using a cell based survival assay that has proven to be an accurate surrogate for nuclease specificity (Pruett-Miller, S.M., etal., Comparison of zinc finger nucleases for use in gene targeting in mammalian cells. Mol Ther, 2008. 16(4): p. 707-17). A tdTomato fluorescent plasmid was transfected with or without nucleases and tdTomato expression was analyzed by flow cytometry at days 2 and 6 post-transfection. Cell survival was calculated as a ratio of day 6:day 2 fluorescence normalized to samples transfected without nuclease. We found that cells transfected with the Ubc promoter driving one pair of ZFNs retained 96% cell survival, the CMV promoter driving a second pair of ZFNs had 83% cell survival, while the TALEN pair had 100% cell survival (Figure 28D). Thus, the TALEN pair demonstrated marked superiority compared to the ZFNs in terms of both increased gene addition frequency, even at very low transfection quantities, and decreased associated cellular toxicity.

**Gene Addition that Harnesses an Endogenous Promoter.** Finally, we determined if a transgene could be inserted in-frame with the target locus, so that the use of an exogenous promoter would not be required. We designed a donor construct in which a biologically inert surface selectable marker, ΔNGFR, would be expressed downstream from the restored GFP gene though a T2A peptide linkage (Figure 28E). We demonstrated that TALENs could induce high levels of targeting with this donor at 1.9% percent compared with 0.07% for ZFNs and that the targeted fibroblasts could be rapidly and easily purified by magnetic bead separation for ΔNGFR (Figure 28F). This data provides proof of principle for a targeting strategy in which a transgene can be targeted to any locus in a manner such that the transgene is driven by the endogenous regulatory elements of the target gene without disrupting the expression of the endogenous gene product.

### DISCUSSION

In prior work (Connelly, J.P., et al., Gene correction by homologous recombination with zinc finger nucleases in primary cells from a mouse model of a generic recessive genetic disease. Mol Ther, 2010. 18(6): p. 1103-10) we described a strategy of ex vivo nuclease mediated site-specific gene targeting in mouse adult primary fibroblasts. This current work expands on this strategy by demonstrating that fibroblasts can undergo site-specific gene addition events to secrete proteins in a manner that utilizes a gene addition specific reporter that does not require disruption of the endogenous target locus. In the literature, gene addition in fibroblasts has been used for three categories of therapy. First, fibroblasts have been modified in diseases where the fibroblast is directly related to the pathology, such as epidermolysis bullosa (Titeux, M., etal., SIN retroviral vectors expressing COL7A1 under human promoters for ex vivo gene therapy of recessive dystrophic epidermolysis bullosa. Mol Ther, 2010. 18(8): p. 1509-18). Secondly, fibroblasts have been modified to serve as vehicles for systemic protein delivery by secreting ectopic proteins such as Factor VIII and IX for the treatment of Hemophilia A and B (Palmer, T.D., A.R. Thompson, and A.D. Miller, Production of human factor IX in animals by genetically modified skin fibroblasts: potential therapy for hemophilia B. Blood, 1989. 73(2): p. 438-45; Roth, D.A., et al., Nonviral transfer of the gene encoding coagulation factor VIII in patients with severe hemophilia A. N Engl J Med, 2001. 344(23): p. 1735-42; Qiu, X., et al., Implantation of autologous skin fibroblast genetically modified to secrete clotting factor IX partially corrects the hemorrhagic tendencies in two hemophilia B patients. Chin Med J (Engl), 1996. 109(11): p. 832-9). Lastly, fibroblasts have been modified to secrete ectopic proteins, such as cytokines, to serve as enhancers of a local biologic process. This has been employed in models of wound healing, in models of tissue ischemia, and even in models of peripheral neuroregeneration through secretion of neurotrophic factors after injury (Zhang, Z., et al., Enhanced collateral growth by double transplantation of genenucleofected fibroblasts in ischemic hindlimb of rats. PLoS One, 2011. 6(4): p. e19192; Mason, M.R., et al., Gene therapy for the peripheral nervous system: a strategy to repair the injured nerve? Curr Gene Ther, 2011. 11(2): p. 75-89; Breitbart, A.S., et al., Treatment of ischemic wounds using cultured dermal fibroblasts transduced retrovirally with PDGF-B and VEGF121 genes. Ann Plast Surg, 2001. 46(5): p. 555-61; discussion 561-2). Though many variations of fibroblast modification have been described, this is the first description of creating modified fibroblasts to express surface markers or secreted proteins using the precision of homologous recombination and nuclease-mediate site-specific integration.

Previous studies, e.g. those described above, utilize gene addition strategies that apply either viral-based or plasmid-based strategies that rely on random integration of transgenes in the host cell genome (Gauglitz, G.G., etal., Combined gene and stem cell therapy for cutaneous wound healing. Mol Pharm, 2011. 8(5): p. 1471-9). At the genome level, this strategy carries inherent limitations that include unpredictable gene expression, silencing of gene expression and also a risk of insertional oncogenesis. The development of leukemia and myelodysplasia in several different clinical gene therapy trials highlights the real rather than theoretic risk of insertional oncogenesis (Boztug, K., etal., Stem-cell gene therapy for the Wiskott-Aldrich syndrome. N Engl J Med, 2010. 363(20): p. 1918-27; Hacein-Bey-Abina, S., etal., Insertional oncogenesis in 4 patients after retrovirusmediated gene therapy of SCID-X1. J Clin Invest, 2008. 118(9): p. 3132-42; Stein, S., et al., Genomic instability and myelodysplasia with monosomy 7 consequent to EVI1 activation after gene therapy for chronic granulomatous disease. Nat Med, 2010. 16(2): p. 198-204). Homologous recombination, in contrast, provides a safer method for synthetic biology to create fibroblasts with new, potentially therapeutic phenotypes.

Non-integrating viral vectors can be used to deliver transgenes in vivo but these approaches can result in the induction of pathologic inflammatory reactions from the recognition of viral elements and subsequent elimination of the modified cells by the host immune system (Manno, C.S., etal., Successful transduction of liver in hemophilia by AAV-Factor IX and limitations imposed by the host immune response. Nat Med, 2006. 12(3): p. 342-7). This immune response to in vivo delivered viral vectors can reduce the efficacy and safety, although the recent success of the gene therapy clinical trials for LCA and hemophilia B suggest that the approach may not be fatally flawed when designed correctly. In the present working example, we combined engineered fibroblasts, a less inflammatory gene delivery vehicle, with the technology of controlled, site-specific nuclease-mediated gene addition, a strategy that circumvents the lack of precision of random integration and the need for viral delivery systems.

Current literature suggests that "safe harbors" should be loci that can be disrupted without physiologic consequence and that carry no oncogenic potential when disrupted. These requirements limit the loci available for targeting and increase the difficulty of designing effective targeting strategies. Moreover, this requirement may also result in the targeting of safe harbor loci that are essentially physiologically disconnected from active cellular processes. This may mean that this category of safe-harbor does not a) provide accessible target sites for nucleases in certain cell types because of closed chromatin status or b) result in sufficient protein expression in certain cell types for the same reason, which may limit therapeutic efficacy (van Rensburg, R., etal., Chromatin structure of two genomic sites for targeted transgene integration in induced pluripotent stem cells and hematopoietic stem cells. Gene Ther, 2012). Further, these requirements remain theoretical, as little evidence has been provided that insertion of transgenes in human cells at the currently studied safe-harbor loci (such as AAVS1) are truly safe for transplantation in human patients. For example, AAVS1 is located within the PPP1R12C gene on human chromosome 19. PPP1R12C encodes the regulatory subunit of a phosphatase downstream of the AMP activated protein kinase (AMPK) pathway involved with proper completion of mitosis (Banko, M.R., etal., Chemical genetic screen for AMPKalpha2 substrates uncovers a network of proteins involved in mitosis. Mol Cell, 2011. 44(6): p. 878-92). Nuclease-mediated targeting at this locus is based on the assumption of safety because adeno-associated virus integrates at this locus at a low frequency in certain cell types and does not appear to result in a disease state. Here, we describe a novel alternative strategy to the disruption of a safe harbor locus that may provide inherent flexibility in selecting and targeting the most robustly expressed locus for each cell type and does not rely on the assumption that disruption of any locus would be implicitly safe.

We demonstrated proof of principle for this strategy by targeting a non-homologous sequence of DNA that encodes for and completes the C-terminal amino acid sequence of the target locus. Altering the nucleotide sequence so that it is not recognized as homology is critical because this prevents the homologous recombination event from excluding the transgene. We demonstrated that altering 16.5% of the nucleotides, or roughly every sixth nucleotide at the wobble position was sufficient to prevent recognition as homology, yet was capable of sustaining expression from the safe harbor. This improved strategy provides an advantage above current safe harbor targeting strategies because we are no longer limited to only the loci that can be disrupted without physiologic consequence and in proving whether the harbor is actually safe. These limitations have led to the selection of safe harbors that do not have the optimal capabilities for targeting or for therapeutic levels of expression. For this reason, we have demonstrated that we can target a locus (with the ΔNGFR transgene), in-frame with the endogenous gene product and this allows for expression from the endogenous promoter without disrupting the endogenous locus. The use of making synonymous mutations in the donor/targeting vector is a useful strategy when targeting an exon of a gene. If one used genome editing to target a transgene to either the 3' or 5' end of the gene and expression of the transgene was driven by the endogenous regulatory elements through a 2A peptide linkage, one might not have to introduce such synonymous mutations into the donor/targeting vector. In some instances, such as those with fibroblast engineering, hepatocyte engineering or within the hematopoietic system, harnessing the robust, tissue-specific expression of endogenous loci through targeted gene addition without safe harbor gene disruption may prove to be a powerful gene therapy strategy.

In conventional transgenesis by random integration, transgenes often integrate in multicopy tandem arrays. This can have consequences ranging from higher levels of transgene expression to silencing of the transgene because of cellular recognition of the array (Henikoff, S., et al., Conspiracy of silence among repeated transgenes. Bioessays, 1998. 20(7): p. 532-5; Mutskov, V., et al., Silencing of transgene transcription precedes methylation of promoter DNA and histone H3 lysine 9. EMBO J, 2004. 23(1): p. 138-49; Rosser, J.M., et al., Repeat-induced gene silencing of L1 transgenes is correlated with differential promoter methylation. Gene, 2010. 456(1-2): p. 15-23). We hypothesized that targeting multiple copies of a cDNA in our safe harbor locus might result in higher levels of transgene expression, but at a certain copy number threshold, expression might decrease, possibly because of silencing or locus instability. We targeted the human growth hormone cDNA at 1, 2, 3, or 4 copies and observed that up to 3 copies provided increased expression over 1-2 copies but that there was no further increase with 4 copies. These results demonstrate that creating targeted multi-copy arrays is feasible, does increase expression but that the optimal copy number needs to be determined experimentally.

The newly discovered TALENs have shown promise as a next generation genome engineering tool. One major reason TALENs are preferable to ZFNs is that they can be rapidly assembled to target virtually any locus with a modular assembly approach in contrast to high quality ZFNs which usually require laborious and high levels of technical expertise to engineer. Our results are consistent with the published results of others by providing another example that TALENs can give both increased targeting frequencies with reduced cellular toxicity. Thus, our results, combined with the rapid, modular assembly design strategy for TALENs supports the continued development of TALENs for gene therapy purposes.

In summary, we have used a mouse model to study a number of new approaches to nuclease-mediated genome editing by homologous recombination. These studies have shown that TALENs have improved properties relative to ZFNs, that one can target gene integration to specific genomic loci without disrupting the target locus and even utilize the endogenous locus to drive expression, that multi-copy transgene arrays to increase transgene expression can be integrated using this approach, and that fibroblasts can be engineered to secrete biologically relevant proteins in this way. All of these findings are important in using synthetic biology combined with gene and cell therapy to develop novel therapeutics for a wide variety of human diseases.

The preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of the present invention is embodied by the appended claims.

### EMBODIMENTS

1. A donor polynucleotide composition for expressing a gene of interest from a target locus in a cell without disrupting the expression of the gene at the target locus, the donor polynucleotide comprising:
   a nucleic acid cassette comprising:
      the gene of interest; and
      at least one element selected from the group consisting of:
         a) a 2A peptide;
         b) an internal ribosome entry site (IRES);
         c) an N-terminal intein splicing region and a C-terminal intein splicing region;
         d) a splice donor and a splice acceptor; and
         e) a coding sequence for the gene at the target locus; and
   sequences flanking the cassette that are homologous to sequences flanking an integration site in the target locus.
2. The method according to embodiment 1, wherein the cassette is configured such that the gene of interest is operably linked to the promoter at the target locus upon insertion into the target locus.
3. The method according to embodiment 1, wherein the cassette comprises a promoter operably linked to the gene of interest.
4. The method according to embodiment 1, wherein the cassette comprises two or more genes of interest.
5. A method for expressing a gene of interest from a target locus in a cell without disrupting the expression of the gene at the target locus, the method comprising:
   contacting the cell with an effective amount of the donor polynucleotide according to any of embodiments 1-4.
6. The method according to embodiment 5, wherein the contacting occurs in the presence of one or more targeted nucleases.
7 The method according to embodiment 6, wherein the cell stably expresses the one or more targeted nucleases.
8. The method according to embodiment 6, wherein the method further comprises contacting the cell with the one or more targeted nucleases.
9. The method according to embodiment 6, wherein the one or more targeted nucleases is selected from the group consisting of a zinc finger nuclease, a TALEN, a homing endonuclease, or a targeted SPO11 nuclease.
10. The method according to embodiment 5, wherein the target locus is selected from the group consisting of actin, ADA, albumin, α-globin, β-globin, CD2, CD3, CD5, CD7, E1α, IL2RG, Ins1, Ins2, NCF1, p50, p65, PF4, PGC-γ, PTEN, TERT, UBC, and VWF.
11. The method according to embodiment 5, wherein the gene of interest is a therapeutic peptide or polypeptide, a selectable marker, or an imaging marker.
12. The method according to embodiment 5, wherein the cell is a mitotic cell.
13. The method according to embodiment 5, wherein the cell is a post-mitotic cell.
14. The method according to embodiment 5, wherein the cell is in vitro.
15. The method according to embodiment 5, wherein the cell is in vivo.
16. A method of producing a gene modification in a cell in a subject, the gene modification comprising an insertion in a target DNA locus that does not disrupt the expression of the gene at the target locus, the method comprising:
   contacting a cell ex vivo with an effective amount of a donor polynucleotide according to embodiment 1-4, wherein the contacting occurs under conditions that are permissive for nonhomologous end joining or homologous recombination; and
   transplanting the cell into the subject.
17. The method according to embodiment 16, further comprising contacting the cells with a first targeted nuclease that is specific for a first nucleotide sequence within the target locus, and a second targeted nuclease that is specific for a second nucleotide sequence within the target locus.
18. The method according to embodiment 15, wherein the cell to be contacted is harvested from the subject.
19. The method according to embodiment 15, further comprising selecting for the cells comprising the insertion prior to transplanting.
20. The method according to embodiment 15, further comprising expanding the cells comprising the insertion prior to transplanting.
21. A method of treating a wound in an individual, the method comprising:
   contacting a cell with an effective amount of donor polynucleotide comprising at least one wound healing growth factor gene, wherein the donor polynucleotide is configured to promote the integration of the wound healing growth factor into a target locus in the cell without disrupting the expression of the gene at the target locus, and
   transplanting the cell into the subject.
22. The method according to embodiment 21, wherein the cell is a fibroblast.
23. The method according to embodiment 22, wherein the fibroblast is autologous.
24. The method according to embodiment 23, wherein the fibroblast is induced from a pluripotent stem cell.
25. The method according to embodiment 22, wherein the fibroblast is a universal fibroblast.
26. The method according to embodiment 21, wherein the wound healing growth factor gene is selected from the group consisting of PDGF, VEGF, EGF, TGFα, TGBβ, FGF, TNF, IL-1, IL-2, IL-6, IL-8, and endothelium derived growth factor.
27. The method according to embodiment 21, wherein the target locus is the adenosine deaminase gene (ADA) locus.
28. The method according to embodiment 27, wherein the donor polynucleotide promotes the integration into the ADA locus at exon 1.
29. The method according to embodiment 27, wherein the cells are contacted with a first targeted nuclease that is specific for a first nucleotide sequence within the ADA locus, and a second targeted nuclease that is specific for a second nucleotide sequence within the ADA locus.
30. The method according to embodiment 29, wherein the first targeted nuclease and the second targeted nuclease are TALENs.
31. The method according to embodiment 21, wherein the donor polynucleotide further comprises a suicide gene.
32. The method according to embodiment 31, wherein the suicide gene is the TK gene, inducible caspase 9, or CD20.
33. The method according to embodiment 31, wherein the suicide gene is under the control of a constitutively acting promoter.
34. The method according to embodiment 31, wherein the suicide gene is under the control of an inducible promoter.
35. A method of treating a nervous system condition in an individual, the method comprising:
   contacting a cell with an effective amount of donor polynucleotide comprising at least one neuroprotective factor, wherein the donor polynucleotide is configured to promote the integration of the neuroprotection factor into a target locus in the cell without disrupting the expression of the gene at the target locus, and
   transplanting the cell into the subject.
36. The method according to embodiment 35, wherein the cell is an astrocyte, an oligodendrocyte, a Schwann cell, or a neuron.
37. The method according to embodiment 36, wherein the cell is autologous.
38. The method according to embodiment 36, wherein the cell is induced from a pluripotent stem cell.
39. The method according to embodiment 35, wherein the neuroprotective factor is selected from the group consisting of a neurotrophin, Kifap3, Bcl-xl, Crmp1, Chkβ, CALM2, Caly, NPG11, NPT1, Eef1a1, Dhps, Cd151, Morf412, CTGF, LDH-A, Atl1, NPT2, Ehd3, Cox5b, Tuba1a, γ-actin, Rpsa, NPG3, NPG4, NPG5, NPG6, NPG7, NPG8, NPG9, and NPG10.

## Claims

1. A method for expressing a gene of interest from a target locus in a cell without disrupting the expression of the gene at the target locus, the method comprising:
contacting the cell with an effective amount of a donor polynucleotide composition, wherein the contacting occurs in the presence of one or more targeted nucleases.

2. The method according to claim 1, wherein:
(i) the cell transiently expresses the one or more targeted nucleases or stably expresses the one or more targeted nucleases,
(ii) the method further comprises contacting the cell with the one or more targeted nucleases,
(iii) the one or more targeted nucleases is selected from the group consisting of a zinc finger nuclease, a TALEN, a homing endonuclease, or a targeted SPO11 nuclease,
(iv) the target locus is selected from the group consisting of actin, ADA, albumin, α-globin, β-globin, CD2, CD3, CD5, CD7, E1α, IL2RG, Ins1, Ins2, NCF1, p50, p65, PF4, PGC-γ, PTEN, TERT, UBC, and VWF, or
(v) the gene of interest is a therapeutic peptide or polypeptide, a selectable marker, or an imaging marker.

3. The method according to claim 1, wherein:
(i) the cell is a mitotic cell,
(ii) the cell is a post-mitotic cell,
(iii) the cell is in vitro, or
(iv) the cell is in vivo.

4. The method of any preceding claim, wherein the donor polynucleotide composition comprises:
a nucleic acid cassette comprising:
the gene of interest; and
at least one element selected from the group consisting of:
a) a 2A peptide;
b) an internal ribosome entry site (IRES);
c) an N-terminal intein splicing region and a C-terminal intein splicing region;
d) a splice donor and a splice acceptor; and
e) a coding sequence for the gene at the target locus; and
sequences flanking the cassette that are homologous to sequences flanking an integration site in the target locus.

5. The method according to claim 4, wherein:
(i) the cassette is configured such that the gene of interest is operably linked to the promoter at the target locus upon insertion into the target locus,
(ii) the cassette comprises a promoter operably linked to the gene of interest, or
(iii) the cassette comprises two or more genes of interest.

6. A donor polynucleotide for use in a method of producing a gene modification in a cell in a subject, the gene modification comprising an insertion in a target DNA locus that does not disrupt the expression of the gene at the target locus, the method comprising:
contacting a cell ex vivo with an effective amount of the donor polynucleotide, wherein the contacting occurs under conditions that are permissive for nonhomologous end joining or homologous recombination; and
transplanting the cell into the subject.

7. The donor polynucleotide for use according to claim 6, wherein:
(i) the method further comprises contacting the cells with a first targeted nuclease that is specific for a first nucleotide sequence within the target locus, and a second targeted nuclease that is specific for a second nucleotide sequence within the target locus,
(ii) the cell to be contacted is harvested from the subject,
(iii) the method further comprises selecting for the cells comprising the insertion prior to transplanting, or
(iv) the method further comprises expanding the cells comprising the insertion prior to transplanting.

8. A donor polynucleotide for use in a method of treating a wound in an individual, wherein the method comprises contacting a cell with an effective amount of the donor polynucleotide and transplanting the cell into the subject, and wherein the donor polynucleotide comprises at least one wound healing growth factor gene, wherein the donor polynucleotide is configured to promote the integration of the wound healing growth factor into a target locus in the cell without disrupting the expression of the gene at the target locus.

9. The donor polynucleotide for use according to claim 8, wherein the cell is a fibroblast, optionally wherein:
(i) the fibroblast is autologous, optionally wherein the fibroblast is induced from a pluripotent stem cell, or
(ii) the fibroblast is a universal fibroblast.

10. The donor polynucleotide for use according to claim 8, wherein the wound healing growth factor gene is selected from the group consisting of PDGF, VEGF, EGF, TGFα, TGBβ, FGF, TNF, IL-1, IL-2, IL-6, IL-8, and endothelium derived growth factor.

11. The donor polynucleotide for use according to claim 8, wherein the target locus is the adenosine deaminase gene (ADA) locus, optionally wherein:
(i) the donor polynucleotide promotes the integration into the ADA locus at exon 1, or
(ii) the cells are contacted with a first targeted nuclease that is specific for a first nucleotide sequence within the ADA locus, and a second targeted nuclease that is specific for a second nucleotide sequence within the ADA locus, optionally wherein the first targeted nuclease and the second targeted nuclease are TALENs.

12. The donor polynucleotide for use according to claim 8, wherein the donor polynucleotide further comprises a suicide gene, optionally wherein:
(i) the suicide gene is the TK gene, inducible caspase 9, or CD20,
(ii) the suicide gene is under the control of a constitutively acting promoter, or
(iii) the suicide gene is under the control of an inducible promoter.

13. A donor polynucleotide for use in a method of treating a nervous system condition in an individual, the method comprising:
contacting a cell with an effective amount of the donor polynucleotide comprising at least one neuroprotective factor, and transplanting the cell into the subject,
and wherein the donor polynucleotide is configured to promote the integration of the neuroprotection factor into a target locus in the cell without disrupting the expression of the gene at the target locus.

14. The donor polynucleotide for use according to claim 13, wherein the cell is an astrocyte, an oligodendrocyte, a Schwann cell, or a neuron, optionally wherein:
(i) the cell is autologous, or
(ii) the cell is induced from a pluripotent stem cell.

15. The donor polynucleotide for use according to claim 13, wherein the neuroprotective factor is selected from the group consisting of a neurotrophin, Kifap3, Bcl-xl, Crmp1, Chkβ, CALM2, Caly, NPG11, NPT1, Eef1a1, Dhps, Cd151, Morf412, CTGF, LDH-A, Atl1, NPT2, Ehd3, Cox5b, Tubala, γ-actin, Rpsa, NPG3, NPG4, NPG5, NPG6, NPG7, NPG8, NPG9, and NPG10.


SEQUENCE LISTING

<110> Kay, Mark A.
      Porteus, Matthew
      Barker, Jenny
      Chekcetts, Josh
      Voit, Richard
      Barzel, Adi

<120> Non-Disruptive Gene Targeting


<130> STAN-898WO

<140> US2013/032443
<141> 2013-03-15

<150> 61/635,203
<151> 2012-04-18

<150> 61/654,645
<151> 2012-06-01

<160> 26

<170> FastSEQ for Windows Version 4.0

<210> 1
<211> 18
<212> DNA
<213> Artificial Sequence

<220>
<223> Oligonucleotide

<400> 1
aaggacgacg gcaactac                                                    18

<210> 2
<211> 20
<212> DNA
<213> Artificial Sequence

<220>
<223> Oligonucleotide

<400> 2
gacgtgcgct tttgaagcgt                                                  20

<210> 3
<211> 78
<212> DNA
<213> Artificial Sequence

<220>
<223> Oligonucleotide

<400> 3
cgcaagcgcc gcagcggcag cggcgagggc cgcggcagcc tgctgacctg cggcgacgtg 60
gaggagaacc ccggcccc                                                    78

<210> 4

<211> 16
<212> DNA
<213> Artificial Sequence

<220>
<223> Oligonucleotide

<400> 4
tgcccgaagg ctacgt                                                    16

<210> 5
<211> 18
<212> DNA
<213> Artificial Sequence

<220>
<223> Oligonucleotide

<400> 5
ttgccgtcgt ccttgaag                                                  18

<210> 6
<211> 20
<212> DNA
<213> Artificial Sequence

<220>
<223> Oligonucleotide

<400> 6
atggtgagca agggcgagga                                                20

<210> 7
<211> 25
<212> DNA
<213> Artificial Sequence

<220>
<223> Oligonucleotide

<400> 7
ttacttgtac agctcgtcca tgccg                                          25

<210> 8
<211> 29
<212> DNA
<213> Artificial Sequence

<220>
<223> Oligonucleotide

<400> 8
ttatttgtag agctcatcca ttccgaggg                                      29

<210> 9
<211> 1001
<212> PRT
<213> Artificial Sequence

<220>
<223> Synthetic Polypeptide

<400> 9

Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser His Asp Gly Gly Lys
            180                 185                 190
Gln Ala Leu Glu Thr Val Gln Arg Leu Leu Pro Val Leu Cys Gln Asp
        195                 200                 205
His Gly Leu Thr Pro Glu Gln Val Val Ala Ile Ala Ser His Asp Gly
    210                 215                 220
Gly Lys Gln Ala Leu Glu Thr Val Gln Arg Leu Leu Pro Val Leu Cys
225                 230                 235                 240
Gln Ala His Gly Leu Thr Pro Asp Gln Val Val Ala Ile Ala Ser Asn
                245                 250                 255
Gly Gly Gly Lys Gln Ala Leu Glu Thr Val Gln Arg Leu Leu Pro Val
            260                 265                 270
Leu Cys Gln Ala His Gly Leu Thr Pro Ala Gln Val Val Ala Ile Ala
        275                 280                 285
Asn Asn Asn Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser Asn Ile Gly Gly Lys Gln Ala Leu Glu Thr Val Gln Arg
                325                 330                 335
Leu Leu Pro Val Leu Cys Gln Asp His Gly Leu Thr Pro Glu Gln Val
            340                 345                 350
Val Ala Ile Ala Ser His Asp Gly Gly Lys Gln Ala Leu Glu Thr Val
        355                 360                 365
Gln Arg Leu Leu Pro Val Leu Cys Gln Ala His Gly Leu Thr Pro Asp
    370                 375                 380
Gln Val Val Ala Ile Ala Ser Asn Ile Gly Gly Lys Gln Ala Leu Glu
385                 390                 395                 400
Thr Val Gln Arg Leu Leu Pro Val Leu Cys Gln Ala His Gly Leu Thr
                405                 410                 415
Pro Ala Gln Val Val Ala Ile Ala Ser His Asp Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser Asn Ile Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Glu Gln Val Val Ala Ile Ala Asn Asn Asn Gly
                485                 490                 495
Gly Lys Gln Ala Leu Glu Thr Val Gln Arg Leu Leu Pro Val Leu Cys

                    500                     505                     510
Gln Ala His Gly Leu Thr Pro Asp Gln Val Val Ala Ile Ala Ser Asn
                515                     520                     525
Ile Gly Gly Lys Gln Ala Leu Glu Thr Val Gln Arg Leu Leu Pro Val
            530                     535                     540
Leu Cys Gln Ala His Gly Leu Thr Pro Ala Gln Val Val Ala Ile Ala
545                     550                     555                     560
Ser His Asp Gly Gly Lys Gln Ala Leu Glu Thr Val Gln Arg Leu Leu
                565                     570                     575
Pro Val Leu Cys Gln Asp His Gly Leu Thr Pro Asp Gln Val Val Ala
            580                     585                     590
Ile Ala Ser Asn Ile Gly Gly Lys Gln Ala Leu Glu Thr Val Gln Arg
        595                     600                     605
Leu Leu Pro Val Leu Cys Gln Asp His Gly Leu Thr Pro Glu Gln Val
    610                     615                     620
Val Ala Ile Ala Asn Asn Asn Gly Gly Lys Gln Ala Leu Glu Thr Val
625                     630                     635                     640
Gln Arg Leu Leu Pro Val Leu Cys Gln Ala His Gly Leu Thr Pro Asp
                645                     650                     655
Gln Val Val Ala Ile Ala Ser Asn Ile Gly Gly Lys Gln Ala Leu Glu
            660                     665                     670
Thr Val Gln Arg Leu Leu Pro Val Leu Cys Gln Ala His Gly Leu Thr
        675                     680                     685
Pro Ala Gln Val Val Ala Ile Ala Ser His Asp Gly Gly Lys Gln Ala
    690                     695                     700
Leu Glu Thr Val Gln Arg Leu Leu Pro Val Leu Cys Gln Asp His Gly
705                     710                     715                     720
Leu Thr Pro Glu Gln Val Val Ala Ile Ala Ser Asn Gly Gly Gly Arg
                725                     730                     735
Pro Ala Leu Glu Ser Ile Val Ala Gln Leu Ser Arg Pro Asp Pro Ala
            740                     745                     750
Leu Ala Ala Leu Thr Asn Asp His Leu Val Ala Leu Ala Cys Leu Gly
        755                     760                     765
Gly Arg Pro Ala Leu Asp Ala Val Lys Lys Gly Leu Pro His Ala Pro
    770                     775                     780
Ala Leu Ile Lys Arg Thr Asn Arg Arg Ile Pro Glu Arg Thr Ser His
785                     790                     795                     800
Arg Val Ala Gly Ser Gln Leu Val Lys Ser Glu Leu Glu Glu Lys Lys
                805                     810                     815
Ser Glu Leu Arg His Lys Leu Lys Tyr Val Pro His Glu Tyr Ile Glu
            820                     825                     830
Leu Ile Glu Ile Ala Arg Asn Ser Thr Gln Asp Arg Ile Leu Glu Met
        835                     840                     845
Lys Val Met Glu Phe Phe Met Lys Val Tyr Gly Tyr Arg Gly Lys His
    850                     855                     860
Leu Gly Gly Ser Arg Lys Pro Asp Gly Ala Ile Tyr Thr Val Gly Ser
865                     870                     875                     880
Pro Ile Asp Tyr Gly Val Ile Val Asp Thr Lys Ala Tyr Ser Gly Gly
                885                     890                     895
Tyr Asn Leu Pro Ile Gly Gln Ala Asp Glu Met Gln Arg Tyr Val Glu
            900                     905                     910
Glu Asn Gln Thr Arg Asn Lys His Ile Asn Pro Asn Glu Trp Trp Lys
        915                     920                     925
Val Tyr Pro Ser Ser Val Thr Glu Phe Lys Phe Leu Phe Val Ser Gly
    930                     935                     940
His Phe Lys Gly Asn Tyr Lys Ala Gln Leu Thr Arg Leu Asn His Ile
945                     950                     955                     960
Thr Asn Arg Asn Gly Ala Val Leu Ser Val Glu Glu Leu Leu Ile Gly
                965                     970                     975
Gly Glu Met Ile Lys Ala Gly Thr Leu Thr Leu Glu Glu Val Arg Arg
            980                     985                     990
Lys Phe Asn Asn Gly Glu Ile Asn Phe
        995                     1000

<210> 10
<211> 898
<212> PRT
<213> Artificial Sequence

<220>
<223> Synthetic Polypeptide

<400> 10
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Ala
    50                  55                  60
Gln His His Glu Ala Leu Val Gly His Gly Phe Thr His Ala His Ile
65                  70                  75                  80
Val Ala Leu Ser Gln His Pro Ala Ala Leu Gly Thr Val Ala Val Lys
                85                  90                  95
Tyr Gln Asp Met Ile Ala Ala Leu Pro Glu Ala Thr His Glu Ala Ile
            100                 105                 110
Val Gly Val Gly Lys Gln Trp Ser Gly Ala Arg Ala Leu Glu Ala Leu
        115                 120                 125
Leu Thr Val Ala Gly Glu Leu Arg Gly Pro Pro Leu Gln Leu Asp Thr
    130                 135                 140
Gly Gln Leu Leu Lys Ile Ala Lys Arg Gly Gly Val Thr Ala Val Glu
145                 150                 155                 160
Ala Val His Ala Trp Arg Asn Ala Leu Thr Gly Ala Pro Leu Asn Leu
                165                 170                 175
Thr Pro Asp Gln Val Val Ala Ile Ala Asn Asn Asn Gly Gly Lys Gln
            180                 185                 190
Ala Leu Glu Thr Val Gln Arg Leu Leu Pro Val Leu Cys Gln Asp His
        195                 200                 205
Gly Leu Thr Pro Glu Gln Val Val Ala Ile Ala Ser Asn Ile Gly Gly
    210                 215                 220
Lys Gln Ala Leu Glu Thr Val Gln Arg Leu Leu Pro Val Leu Cys Gln
225                 230                 235                 240
Ala His Gly Leu Thr Pro Asp Gln Val Val Ala Ile Ala Ser His Asp
                245                 250                 255
Gly Gly Lys Gln Ala Leu Glu Thr Val Gln Arg Leu Leu Pro Val Leu
            260                 265                 270
Cys Gln Ala His Gly Leu Thr Pro Ala Gln Val Val Ala Ile Ala Ser
        275                 280                 285
Asn Ile Gly Gly Lys Gln Ala Leu Glu Thr Val Gln Arg Leu Leu Pro
    290                 295                 300
Val Leu Cys Gln Asp His Gly Leu Thr Pro Asp Gln Val Val Ala Ile
305                 310                 315                 320
Ala Ser His Asp Gly Gly Lys Gln Ala Leu Glu Thr Val Gln Arg Leu
                325                 330                 335
Leu Pro Val Leu Cys Gln Asp His Gly Leu Thr Pro Glu Gln Val Val
            340                 345                 350
Ala Ile Ala Ser Asn Ile Gly Gly Lys Gln Ala Leu Glu Thr Val Gln
        355                 360                 365
Arg Leu Leu Pro Val Leu Cys Gln Ala His Gly Leu Thr Pro Asp Gln
    370                 375                 380
Val Val Ala Ile Ala Asn Asn Asn Gly Gly Lys Gln Ala Leu Glu Thr
385                 390                 395                 400
Val Gln Arg Leu Leu Pro Val Leu Cys Gln Ala His Gly Leu Thr Pro
                405                 410                 415

Ala Gln Val Val Ala Ile Ala Ser Asn Ile Gly Gly Lys Gln Ala Leu
            420                 425                 430
Glu Thr Val Gln Arg Leu Leu Pro Val Leu Cys Gln Asp His Gly Leu
        435                 440                 445
Thr Pro Asp Gln Val Val Ala Ile Ala Ser His Asp Gly Gly Lys Gln
    450                 455                 460
Ala Leu Glu Thr Val Gln Arg Leu Leu Pro Val Leu Cys Gln Asp His
465                 470                 475                 480
Gly Leu Thr Pro Glu Gln Val Val Ala Ile Ala Ser Asn Ile Gly Gly
                485                 490                 495
Lys Gln Ala Leu Glu Thr Val Gln Arg Leu Leu Pro Val Leu Cys Gln
            500                 505                 510
Ala His Gly Leu Thr Pro Asp Gln Val Val Ala Ile Ala Asn Asn Asn
        515                 520                 525
Gly Gly Lys Gln Ala Leu Glu Thr Val Gln Arg Leu Leu Pro Val Leu
    530                 535                 540
Cys Gln Ala His Gly Leu Thr Pro Ala Gln Val Val Ala Ile Ala Ser
545                 550                 555                 560
Asn Ile Gly Gly Lys Gln Ala Leu Glu Thr Val Gln Arg Leu Leu Pro
                565                 570                 575
Val Leu Cys Gln Asp His Gly Leu Thr Pro Asp Gln Val Val Ala Ile
            580                 585                 590
Ala Ser His Asp Gly Gly Lys Gln Ala Leu Glu Thr Val Gln Arg Leu
        595                 600                 605
Leu Pro Val Leu Cys Gln Asp His Gly Leu Thr Pro Glu Gln Val Val
    610                 615                 620
Ala Ile Ala Ser Asn Gly Gly Gly Arg Pro Ala Leu Glu Ser Ile Val
625                 630                 635                 640
Ala Gln Leu Ser Arg Pro Asp Pro Ala Leu Ala Ala Leu Thr Asn Asp
                645                 650                 655
His Leu Val Ala Leu Ala Cys Leu Gly Gly Arg Pro Ala Leu Asp Ala
            660                 665                 670
Val Lys Lys Gly Leu Pro His Ala Pro Ala Leu Ile Lys Arg Thr Asn
        675                 680                 685
Arg Arg Ile Pro Glu Arg Thr Ser His Arg Val Ala Gly Ser Gln Leu
    690                 695                 700
Val Lys Ser Glu Leu Glu Glu Lys Lys Ser Glu Leu Arg His Lys Leu
705                 710                 715                 720
Lys Tyr Val Pro His Glu Tyr Ile Glu Leu Ile Glu Ile Ala Arg Asn
                725                 730                 735
Ser Thr Gln Asp Arg Ile Leu Glu Met Lys Val Met Glu Phe Phe Met
            740                 745                 750
Lys Val Tyr Gly Tyr Arg Gly Lys His Leu Gly Gly Ser Arg Lys Pro
        755                 760                 765
Asp Gly Ala Ile Tyr Thr Val Gly Ser Pro Ile Asp Tyr Gly Val Ile
    770                 775                 780
Val Asp Thr Lys Ala Tyr Ser Gly Gly Tyr Asn Leu Pro Ile Gly Gln
785                 790                 795                 800
Ala Asp Glu Met Gln Arg Tyr Val Glu Glu Asn Gln Thr Arg Asn Lys
                805                 810                 815
His Ile Asn Pro Asn Glu Trp Trp Lys Val Tyr Pro Ser Ser Val Thr
            820                 825                 830
Glu Phe Lys Phe Leu Phe Val Ser Gly His Phe Lys Gly Asn Tyr Lys
        835                 840                 845
Ala Gln Leu Thr Arg Leu Asn His Ile Thr Asn Arg Asn Gly Ala Val
    850                 855                 860
Leu Ser Val Glu Glu Leu Leu Ile Gly Gly Glu Met Ile Lys Ala Gly
865                 870                 875                 880
Thr Leu Thr Leu Glu Glu Val Arg Arg Lys Phe Asn Asn Gly Glu Ile
                885                 890                 895
Asn Phe

<210> 11
<211> 865
<212> PRT
<213> Artificial Sequence

<220>
<223> Synthetic Polypeptide

<400> 11
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Arg Gly Val Pro Met Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                   165                 170                 175
   Leu Thr Pro Asp Gln Val Val Ala Ile Ala Ser Asn Ile Gly Gly Lys
               180                 185                 190
   Gln Ala Leu Glu Thr Val Gln Arg Leu Leu Pro Val Leu Cys Gln Asp
           195                 200                 205
   His Gly Leu Thr Pro Glu Gln Val Val Ala Ile Ala Ser His Asp Gly
       210                 215                 220
   Gly Lys Gln Ala Leu Glu Thr Val Gln Arg Leu Leu Pro Val Leu Cys
   225                 230                 235                 240
   Gln Ala His Gly Leu Thr Pro Asp Gln Val Val Ala Ile Ala Ser Asn
                   245                 250                 255
   Ile Gly Gly Lys Gln Ala Leu Glu Thr Val Gln Arg Leu Leu Pro Val
               260                 265                 270
   Leu Cys Gln Ala His Gly Leu Thr Pro Ala Gln Val Val Ala Ile Ala
           275                 280                 285
   Ser His Asp Gly Gly Lys Gln Ala Leu Glu Thr Val Gln Arg Leu Leu
       290                 295                 300
   Pro Val Leu Cys Gln Asp His Gly Leu Thr Pro Asp Gln Val Val Ala
   305                 310                 315                 320
   Ile Ala Ser His Asp Gly Gly Lys Gln Ala Leu Glu Thr Val Gln Arg
                   325                 330                 335
   Leu Leu Pro Val Leu Cys Gln Asp His Gly Leu Thr Pro Glu Gln Val
               340                 345                 350
   Val Ala Ile Ala Ser His Asp Gly Gly Lys Gln Ala Leu Glu Thr Val
           355                 360                 365
   Gln Arg Leu Leu Pro Val Leu Cys Gln Ala His Gly Leu Thr Pro Asp
       370                 375                 380
   Gln Val Val Ala Ile Ala Ser Asn Ile Gly Gly Lys Gln Ala Leu Glu
   385                 390                 395                 400
   Thr Val Gln Arg Leu Leu Pro Val Leu Cys Gln Ala His Gly Leu Thr
                   405                 410                 415
   Pro Ala Gln Val Val Ala Ile Ala Asn Asn Asn Gly Gly Lys Gln Ala
                420                 425                 430
   Leu Glu Thr Val Gln Arg Leu Leu Pro Val Leu Cys Gln Asp His Gly
           435                 440                 445
   Leu Thr Pro Asp Gln Val Val Ala Ile Ala Asn Asn Asn Gly Gly Lys
       450                 455                 460
   Gln Ala Leu Glu Thr Val Gln Arg Leu Leu Pro Val Leu Cys Gln Asp
   465                 470                 475                 480
   His Gly Leu Thr Pro Glu Gln Val Val Ala Ile Ala Asn Asn Asn Gly
                   485                 490                 495
   Gly Lys Gln Ala Leu Glu Thr Val Gln Arg Leu Leu Pro Val Leu Cys
               500                 505                 510
   Gln Ala His Gly Leu Thr Pro Asp Gln Val Val Ala Ile Ala Ser Asn
           515                 520                 525
   Ile Gly Gly Lys Gln Ala Leu Glu Thr Val Gln Arg Leu Leu Pro Val
       530                 535                 540
   Leu Cys Gln Ala His Gly Leu Thr Pro Ala Gln Val Val Ala Ile Ala
   545                 550                 555                 560
   Ser Asn Ile Gly Gly Lys Gln Ala Leu Glu Thr Val Gln Arg Leu Leu
                   565                 570                 575
   Pro Val Leu Cys Gln Asp His Gly Leu Thr Pro Glu Gln Val Val Ala
               580                 585                 590
   Ile Ala Ser Asn Gly Gly Gly Arg Pro Ala Leu Glu Ser Ile Val Ala
           595                 600                 605
   Gln Leu Ser Arg Pro Asp Pro Ala Leu Ala Ala Leu Thr Asn Asp His
       610                 615                 620
   Leu Val Ala Leu Ala Cys Leu Gly Gly Arg Pro Ala Leu Asp Ala Val
   625                 630                 635                 640
   Lys Lys Gly Leu Pro His Ala Pro Ala Leu Ile Lys Arg Thr Asn Arg
                   645                 650                 655
   Arg Ile Pro Glu Arg Thr Ser His Arg Val Ala Gly Ser Gln Leu Val
               660                 665                 670
   Lys Ser Glu Leu Glu Glu Lys Lys Ser Glu Leu Arg His Lys Leu Lys
           675                 680                 685
   Tyr Val Pro His Glu Tyr Ile Glu Leu Ile Glu Ile Ala Arg Asn Ser
       690                 695                 700
   Thr Gln Asp Arg Ile Leu Glu Met Lys Val Met Glu Phe Phe Met Lys
   705                 710                 715                 720
   Val Tyr Gly Tyr Arg Gly Lys His Leu Gly Gly Ser Arg Lys Pro Asp
                   725                 730                 735
   Gly Ala Ile Tyr Thr Val Gly Ser Pro Ile Asp Tyr Gly Val Ile Val
               740                 745                 750
   Asp Thr Lys Ala Tyr Ser Gly Gly Tyr Asn Leu Pro Ile Gly Gln Ala
           755                 760                 765
   Asp Glu Met Gln Arg Tyr Val Glu Glu Asn Gln Thr Arg Asn Lys His
       770                 775                 780
   Ile Asn Pro Asn Glu Trp Trp Lys Val Tyr Pro Ser Ser Val Thr Glu
   785                 790                 795                 800
   Phe Lys Phe Leu Phe Val Ser Gly His Phe Lys Gly Asn Tyr Lys Ala
                   805                 810                 815
   Gln Leu Thr Arg Leu Asn His Ile Thr Asn Arg Asn Gly Ala Val Leu
               820                 825                 830
   Ser Val Glu Glu Leu Leu Ile Gly Gly Glu Met Ile Lys Ala Gly Thr
           835                 840                 845
   Leu Thr Leu Glu Glu Val Arg Arg Lys Phe Asn Asn Gly Glu Ile Asn
       850                 855                 860
   Phe
   865

<210> 12
<211> 966
<212> PRT
<213> Artificial Sequence

<220>
<223> Synthetic Polypeptide

<400> 12
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala His His Glu Ala Leu Val Gly His Gly Phe Thr His Ala His Ile
65                  70                  75                  80
Val Ala Leu Ser Gln His Pro Ala Ala Leu Gly Thr Val Ala Val Lys
                85                  90                  95
Tyr Gln Asp Met Ile Ala Ala Leu Pro Glu Ala Thr His Glu Ala Ile
            100                 105                 110
Val Gly Val Gly Lys Gln Trp Ser Gly Ala Arg Ala Leu Glu Ala Leu
        115                 120                 125
Leu Thr Val Ala Gly Glu Leu Arg Gly Pro Pro Leu Gln Leu Asp Thr
    130                 135                 140
Gly Gln Leu Leu Lys Ile Ala Lys Arg Gly Gly Val Thr Ala Val Glu
145                 150                 155                 160
Ala Val His Ala Trp Arg Asn Ala Leu Thr Gly Ala Pro Leu Asn Leu
                165                 170                 175
Thr Pro Asp Gln Val Val Ala Ile Ala Ser His Asp Gly Gly Lys Gln
            180                 185                 190
Ala Leu Glu Thr Val Gln Arg Leu Leu Pro Val Leu Cys Gln Asp His
        195                 200                 205
Gly Leu Thr Pro Glu Gln Val Val Ala Ile Ala Ser Asn Ile Gly Gly
    210                 215                 220
Lys Gln Ala Leu Glu Thr Val Gln Arg Leu Leu Pro Val Leu Cys Gln
225                 230                 235                 240
Ala His Gly Leu Thr Pro Asp Gln Val Val Ala Ile Ala Ser Asn Ile
                245                 250                 255
Gly Gly Lys Gln Ala Leu Glu Thr Val Gln Arg Leu Leu Pro Val Leu
            260                 265                 270
Cys Gln Ala His Gly Leu Thr Pro Ala Gln Val Val Ala Ile Ala Ser
        275                 280                 285
His Asp Gly Gly Lys Gln Ala Leu Glu Thr Val Gln Arg Leu Leu Pro
    290                 295                 300
Val Leu Cys Gln Asp His Gly Leu Thr Pro Asp Gln Val Val Ala Ile
305                 310                 315                 320
Ala Ser Asn Ile Gly Gly Lys Gln Ala Leu Glu Thr Val Gln Arg Leu
                325                 330                 335
Leu Pro Val Leu Cys Gln Asp His Gly Leu Thr Pro Glu Gln Val Val
            340                 345                 350
Ala Ile Ala Ser Asn Gly Gly Gly Lys Gln Ala Leu Glu Thr Val Gln
        355                 360                 365
Arg Leu Leu Pro Val Leu Cys Gln Ala His Gly Leu Thr Pro Asp Gln
    370                 375                 380
Val Val Ala Ile Ala Asn Asn Asn Gly Gly Lys Gln Ala Leu Glu Thr
385                 390                 395                 400
Val Gln Arg Leu Leu Pro Val Leu Cys Gln Ala His Gly Leu Thr Pro
                405                 410                 415
Ala Gln Val Val Ala Ile Ala Asn Asn Asn Gly Gly Lys Gln Ala Leu
            420                 425                 430
Glu Thr Val Gln Arg Leu Leu Pro Val Leu Cys Gln Asp His Gly Leu
        435                 440                 445
Thr Pro Asp Gln Val Val Ala Ile Ala Ser His Asp Gly Gly Lys Gln
    450                 455                 460

Ala Leu Glu Thr Val Gln Arg Leu Leu Pro Val Leu Cys Gln Asp His
465                 470                 475                 480
Gly Leu Thr Pro Glu Gln Val Val Ala Ile Ala Asn Asn Asn Gly Gly
                485                 490                 495
Lys Gln Ala Leu Glu Thr Val Gln Arg Leu Leu Pro Val Leu Cys Gln
            500                 505                 510
Ala His Gly Leu Thr Pro Asp Gln Val Val Ala Ile Ala Ser His Asp
        515                 520                 525
Gly Gly Lys Gln Ala Leu Glu Thr Val Gln Arg Leu Leu Pro Val Leu
    530                 535                 540
Cys Gln Ala His Gly Leu Thr Pro Ala Gln Val Val Ala Ile Ala Ser
545                 550                 555                 560
Asn Gly Gly Gly Lys Gln Ala Leu Glu Thr Val Gln Arg Leu Leu Pro
                565                 570                 575
Val Leu Cys Gln Asp His Gly Leu Thr Pro Asp Gln Val Val Ala Ile
            580                 585                 590
Ala Ser Asn Gly Gly Gly Lys Gln Ala Leu Glu Thr Val Gln Arg Leu
        595                 600                 605
Leu Pro Val Leu Cys Gln Asp His Gly Leu Thr Pro Glu Gln Val Val
    610                 615                 620
Ala Ile Ala Asn Asn Asn Gly Gly Lys Gln Ala Leu Glu Thr Val Gln
625                 630                 635                 640
Arg Leu Leu Pro Val Leu Cys Gln Ala His Gly Leu Thr Pro Asp Gln
                645                 650                 655
Val Val Ala Ile Ala Ser His Asp Gly Gly Lys Gln Ala Leu Glu Thr
            660                 665                 670
Val Gln Arg Leu Leu Pro Val Leu Cys Gln Asp His Gly Leu Thr Pro
        675                 680                 685
Glu Gln Val Val Ala Ile Ala Ser Asn Gly Gly Gly Arg Pro Ala Leu
    690                 695                 700
Glu Ser Ile Val Ala Gln Leu Ser Arg Pro Asp Pro Ala Leu Ala Ala
705                 710                 715                 720
Leu Thr Asn Asp His Leu Val Ala Leu Ala Cys Leu Gly Gly Arg Pro
                725                 730                 735
Ala Leu Asp Ala Val Lys Lys Gly Leu Pro His Ala Pro Ala Leu Ile
            740                 745                 750
Lys Arg Thr Asn Arg Arg Ile Pro Glu Arg Thr Ser His Arg Val Ala
        755                 760                 765
Gly Ser Gln Leu Val Lys Ser Glu Leu Glu Glu Lys Lys Ser Glu Leu
    770                 775                 780
Arg His Lys Leu Lys Tyr Val Pro His Glu Tyr Ile Glu Leu Ile Glu
785                 790                 795                 800
Ile Ala Arg Asn Ser Thr Gln Asp Arg Ile Leu Glu Met Lys Val Met
                805                 810                 815
Glu Phe Phe Met Lys Val Tyr Gly Tyr Arg Gly Lys His Leu Gly Gly
            820                 825                 830
Ser Arg Lys Pro Asp Gly Ala Ile Tyr Thr Val Gly Ser Pro Ile Asp
        835                 840                 845
Tyr Gly Val Ile Val Asp Thr Lys Ala Tyr Ser Gly Gly Tyr Asn Leu
    850                 855                 860
Pro Ile Gly Gln Ala Asp Glu Met Gln Arg Tyr Val Glu Glu Asn Gln
865                 870                 875                 880
Thr Arg Asn Lys His Ile Asn Pro Asn Glu Trp Trp Lys Val Tyr Pro
                885                 890                 895
Ser Ser Val Thr Glu Phe Lys Phe Leu Phe Val Ser Gly His Phe Lys
            900                 905                 910
Gly Asn Tyr Lys Ala Gln Leu Thr Arg Leu Asn His Ile Thr Asn Arg
        915                 920                 925
Asn Gly Ala Val Leu Ser Val Glu Glu Leu Leu Ile Gly Gly Glu Met
    930                 935                 940
Ile Lys Ala Gly Thr Leu Thr Leu Glu Glu Val Arg Arg Lys Phe Asn
945                 950                 955                 960
Asn Gly Glu Ile Asn Phe

                965

<210> 13
<211> 899
<212> PRT
<213> Artificial Sequence

<220>
<223> Synthetic Polypeptide

<400> 13
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Arg Gly Val Pro Met Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                   165                 170                 175
   Leu Thr Pro Asp Gln Val Val Ala Ile Ala Ser Asn Gly Gly Gly Lys
               180                 185                 190
   Gln Ala Leu Glu Thr Val Gln Arg Leu Leu Pro Val Leu Cys Gln Asp
           195                 200                 205
   His Gly Leu Thr Pro Glu Gln Val Val Ala Ile Ala Ser His Asp Gly
       210                 215                 220
   Gly Lys Gln Ala Leu Glu Thr Val Gln Arg Leu Leu Pro Val Leu Cys
   225                 230                 235                 240
   Gln Ala His Gly Leu Thr Pro Asp Gln Val Val Ala Ile Ala Ser Asn
                   245                 250                 255
   Ile Gly Gly Lys Gln Ala Leu Glu Thr Val Gln Arg Leu Leu Pro Val
               260                 265                 270
   Leu Cys Gln Ala His Gly Leu Thr Pro Ala Gln Val Val Ala Ile Ala
           275                 280                 285
   Ser Asn Ile Gly Gly Lys Gln Ala Leu Glu Thr Val Gln Arg Leu Leu
       290                 295                 300
   Pro Val Leu Cys Gln Asp His Gly Leu Thr Pro Asp Gln Val Val Ala
   305                 310                 315                 320
   Ile Ala Ser His Asp Gly Gly Lys Gln Ala Leu Glu Thr Val Gln Arg
                   325                 330                 335
   Leu Leu Pro Val Leu Cys Gln Asp His Gly Leu Thr Pro Glu Gln Val
               340                 345                 350
   Val Ala Ile Ala Ser Asn Ile Gly Gly Lys Gln Ala Leu Glu Thr Val
           355                 360                 365
   Gln Arg Leu Leu Pro Val Leu Cys Gln Ala His Gly Leu Thr Pro Asp
       370                 375                 380
   Gln Val Val Ala Ile Ala Ser Asn Gly Gly Gly Lys Gln Ala Leu Glu
   385                 390                 395                 400
   Thr Val Gln Arg Leu Leu Pro Val Leu Cys Gln Ala His Gly Leu Thr
   405                 410                 415
   Pro Ala Gln Val Val Ala Ile Ala Asn Asn Asn Gly Gly Lys Gln Ala
               420                 425                 430
   Leu Glu Thr Val Gln Arg Leu Leu Pro Val Leu Cys Gln Asp His Gly
           435                 440                 445
   Leu Thr Pro Asp Gln Val Val Ala Ile Ala Asn Asn Asn Gly Gly Lys
       450                 455                 460
   Gln Ala Leu Glu Thr Val Gln Arg Leu Leu Pro Val Leu Cys Gln Asp
   465                 470                 475                 480
   His Gly Leu Thr Pro Glu Gln Val Val Ala Ile Ala Ser His Asp Gly
                   485                 490                 495
   Gly Lys Gln Ala Leu Glu Thr Val Gln Arg Leu Leu Pro Val Leu Cys
               500                 505                 510
   Gln Ala His Gly Leu Thr Pro Asp Gln Val Val Ala Ile Ala Asn Asn
           515                 520                 525
   Asn Gly Gly Lys Gln Ala Leu Glu Thr Val Gln Arg Leu Leu Pro Val
       530                 535                 540
   Leu Cys Gln Ala His Gly Leu Thr Pro Ala Gln Val Val Ala Ile Ala
   545                 550                 555                 560
   Ser His Asp Gly Gly Lys Gln Ala Leu Glu Thr Val Gln Arg Leu Leu
                   565                 570                 575
   Pro Val Leu Cys Gln Asp His Gly Leu Thr Pro Asp Gln Val Val Ala
               580                 585                 590
   Ile Ala Ser Asn Gly Gly Gly Lys Gln Ala Leu Glu Thr Val Gln Arg
           595                 600                 605
   Leu Leu Pro Val Leu Cys Gln Asp His Gly Leu Thr Pro Glu Gln Val
       610                 615                 620
   Val Ala Ile Ala Ser Asn Gly Gly Gly Arg Pro Ala Leu Glu Ser Ile
   625                 630                 635                 640
   Val Ala Gln Leu Ser Arg Pro Asp Pro Ala Leu Ala Ala Leu Thr Asn
                   645                 650                 655
   Asp His Leu Val Ala Leu Ala Cys Leu Gly Gly Arg Pro Ala Leu Asp
               660                 665                 670
   Ala Val Lys Lys Gly Leu Pro His Ala Pro Ala Leu Ile Lys Arg Thr
           675                 680                 685
   Asn Arg Arg Ile Pro Glu Arg Thr Ser His Arg Val Ala Gly Ser Gln
       690                 695                 700
   Leu Val Lys Ser Glu Leu Glu Glu Lys Lys Ser Glu Leu Arg His Lys
   705                 710                 715                 720
   Leu Lys Tyr Val Pro His Glu Tyr Ile Glu Leu Ile Glu Ile Ala Arg
                   725                 730                 735
   Asn Ser Thr Gln Asp Arg Ile Leu Glu Met Lys Val Met Glu Phe Phe
               740                 745                 750
   Met Lys Val Tyr Gly Tyr Arg Gly Lys His Leu Gly Gly Ser Arg Lys
           755                 760                 765
   Pro Asp Gly Ala Ile Tyr Thr Val Gly Ser Pro Ile Asp Tyr Gly Val
       770                 775                 780
   Ile Val Asp Thr Lys Ala Tyr Ser Gly Gly Tyr Asn Leu Pro Ile Gly
   785                 790                 795                 800
   Gln Ala Asp Glu Met Gln Arg Tyr Val Glu Glu Asn Gln Thr Arg Asn
                   805                 810                 815
   Lys His Ile Asn Pro Asn Glu Trp Trp Lys Val Tyr Pro Ser Ser Val
               820                 825                 830
   Thr Glu Phe Lys Phe Leu Phe Val Ser Gly His Phe Lys Gly Asn Tyr
           835                 840                 845
   Lys Ala Gln Leu Thr Arg Leu Asn His Ile Thr Asn Arg Asn Gly Ala
       850                 855                 860
   Val Leu Ser Val Glu Glu Leu Leu Ile Gly Gly Glu Met Ile Lys Ala
   865                 870                 875                 880
   Gly Thr Leu Thr Leu Glu Glu Val Arg Arg Lys Phe Asn Asn Gly Glu
                   885                 890                 895
   Ile Asn Phe

<210> 14
<211> 1001
<212> PRT
<213> Artificial Sequence

<220>
<223> Synthetic Polypeptide

<400> 14
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser Asn Ile Gly Gly Lys
            180                 185                 190
Gln Ala Leu Glu Thr Val Gln Arg Leu Leu Pro Val Leu Cys Gln Asp
        195                 200                 205
His Gly Leu Thr Pro Glu Gln Val Val Ala Ile Ala Ser Asn Ile Gly
    210                 215                 220
Gly Lys Gln Ala Leu Glu Thr Val Gln Arg Leu Leu Pro Val Leu Cys
225                 230                 235                 240
Gln Ala His Gly Leu Thr Pro Asp Gln Val Val Ala Ile Ala Ser Asn
                245                 250                 255
Gly Gly Gly Lys Gln Ala Leu Glu Thr Val Gln Arg Leu Leu Pro Val
            260                 265                 270
Leu Cys Gln Ala His Gly Leu Thr Pro Ala Gln Val Val Ala Ile Ala
        275                 280                 285
Asn Asn Asn Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser Asn Ile Gly Gly Lys Gln Ala Leu Glu Thr Val Gln Arg
                325                 330                 335
Leu Leu Pro Val Leu Cys Gln Asp His Gly Leu Thr Pro Glu Gln Val
            340                 345                 350
Val Ala Ile Ala Ser Asn Gly Gly Gly Lys Gln Ala Leu Glu Thr Val
        355                 360                 365
Gln Arg Leu Leu Pro Val Leu Cys Gln Ala His Gly Leu Thr Pro Asp
    370                 375                 380
Gln Val Val Ala Ile Ala Asn Asn Asn Gly Gly Lys Gln Ala Leu Glu
385                 390                 395                 400
Thr Val Gln Arg Leu Leu Pro Val Leu Cys Gln Ala His Gly Leu Thr
                405                 410                 415

Pro Ala Gln Val Val Ala Ile Ala Asn Asn Asn Gly Gly Lys Gln Ala
                420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser His Asp Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Glu Gln Val Val Ala Ile Ala Ser Asn Gly Gly
                485                 490                 495
Gly Lys Gln Ala Leu Glu Thr Val Gln Arg Leu Leu Pro Val Leu Cys
            500                 505                 510
Gln Ala His Gly Leu Thr Pro Asp Gln Val Val Ala Ile Ala Ser Asn
        515                 520                 525
Gly Gly Gly Lys Gln Ala Leu Glu Thr Val Gln Arg Leu Leu Pro Val
    530                 535                 540
Leu Cys Gln Ala His Gly Leu Thr Pro Ala Gln Val Val Ala Ile Ala
545                 550                 555                 560
Ser His Asp Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser Asn Ile Gly Gly Lys Gln Ala Leu Glu Thr Val Gln Arg
        595                 600                 605
Leu Leu Pro Val Leu Cys Gln Asp His Gly Leu Thr Pro Glu Gln Val
    610                 615                 620
Val Ala Ile Ala Ser Asn Ile Gly Gly Lys Gln Ala Leu Glu Thr Val
625                 630                 635                 640
Gln Arg Leu Leu Pro Val Leu Cys Gln Ala His Gly Leu Thr Pro Asp
                645                 650                 655
Gln Val Val Ala Ile Ala Ser His Asp Gly Gly Lys Gln Ala Leu Glu
            660                 665                 670
Thr Val Gln Arg Leu Leu Pro Val Leu Cys Gln Ala His Gly Leu Thr
        675                 680                 685
Pro Ala Gln Val Val Ala Ile Ala Ser Asn Ile Gly Gly Lys Gln Ala
    690                 695                 700
Leu Glu Thr Val Gln Arg Leu Leu Pro Val Leu Cys Gln Asp His Gly
705                 710                 715                 720
Leu Thr Pro Glu Gln Val Val Ala Ile Ala Ser Asn Gly Gly Gly Arg
                725                 730                 735
Pro Ala Leu Glu Ser Ile Val Ala Gln Leu Ser Arg Pro Asp Pro Ala
            740                 745                 750
Leu Ala Ala Leu Thr Asn Asp His Leu Val Ala Leu Ala Cys Leu Gly
        755                 760                 765
Gly Arg Pro Ala Leu Asp Ala Val Lys Lys Gly Leu Pro His Ala Pro
    770                 775                 780
Ala Leu Ile Lys Arg Thr Asn Arg Arg Ile Pro Glu Arg Thr Ser His
785                 790                 795                 800
Arg Val Ala Gly Ser Gln Leu Val Lys Ser Glu Leu Glu Glu Lys Lys
                805                 810                 815
Ser Glu Leu Arg His Lys Leu Lys Tyr Val Pro His Glu Tyr Ile Glu
            820                 825                 830
Leu Ile Glu Ile Ala Arg Asn Ser Thr Gln Asp Arg Ile Leu Glu Met
        835                 840                 845
Lys Val Met Glu Phe Phe Met Lys Val Tyr Gly Tyr Arg Gly Lys His
    850                 855                 860
Leu Gly Gly Ser Arg Lys Pro Asp Gly Ala Ile Tyr Thr Val Gly Ser
865                 870                 875                 880
Pro Ile Asp Tyr Gly Val Ile Val Asp Thr Lys Ala Tyr Ser Gly Gly
                885                 890                 895
Tyr Asn Leu Pro Ile Gly Gln Ala Asp Glu Met Gln Arg Tyr Val Glu
            900                 905                 910
Glu Asn Gln Thr Arg Asn Lys His Ile Asn Pro Asn Glu Trp Trp Lys

915                 920                 925
Val Tyr Pro Ser Ser Val Thr Glu Phe Lys Phe Leu Phe Val Ser Gly
    930                 935                 940
His Phe Lys Gly Asn Tyr Lys Ala Gln Leu Thr Arg Leu Asn His Ile
945                 950                 955                 960
Thr Asn Arg Asn Gly Ala Val Leu Ser Val Glu Glu Leu Leu Ile Gly
                965                 970                 975
Gly Glu Met Ile Lys Ala Gly Thr Leu Thr Leu Glu Glu Val Arg Arg
            980                 985                 990
Lys Phe Asn Asn Gly Glu Ile Asn Phe
        995                 1000

<210> 15
<211> 933
<212> PRT
<213> Artificial Sequence

<220>
<223> Synthetic Polypeptide

<400> 15
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser Asn Asn Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser His Asp Gly
    210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser Asn
                245                 250                 255
Ile Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser His Asp Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser His Asp Gly Gly Lys Gln Ala Leu Glu Thr Val Gln Arg

                325                 330                 335
Leu Leu Pro Val Leu Cys Gln Asp His Gly Leu Thr Pro Asp Gln Val
            340                 345                 350
Val Ala Ile Ala Ser Asn Gly Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser Asn Asn Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser Asn Ile Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser His Asp Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser Asn Gly Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser His
        515                 520                 525
Asp Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser His Asp Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser Asn Gly Gly Gly Lys Gln Ala Leu Glu Thr Val Gln Arg
        595                 600                 605
Leu Leu Pro Val Leu Cys Gln Asp His Gly Leu Thr Pro Asp Gln Val
    610                 615                 620
Val Ala Ile Ala Ser Asn Asn Gly Gly Lys Gln Ala Leu Glu Thr Val
625                 630                 635                 640
Gln Arg Leu Leu Pro Val Leu Cys Gln Asp His Gly Leu Thr Pro Asp
                645                 650                 655
Gln Val Val Ala Ile Ala Ser Asn Gly Gly Gly Lys Gln Ala Leu Glu
            660                 665                 670
Ser Ile Val Ala Gln Leu Ser Arg Pro Asp Pro Ala Leu Ala Ala Leu
        675                 680                 685
Thr Asn Asp His Leu Val Ala Leu Ala Cys Leu Gly Gly Arg Pro Ala
    690                 695                 700
Leu Asp Ala Val Lys Lys Gly Leu Pro His Ala Pro Ala Leu Ile Lys
705                 710                 715                 720
Arg Thr Asn Arg Arg Ile Pro Glu Arg Thr Ser His Arg Val Ala Gly
                725                 730                 735
Ser Gln Leu Val Lys Ser Glu Leu Glu Glu Lys Lys Ser Glu Leu Arg
            740                 745                 750
His Lys Leu Lys Tyr Val Pro His Glu Tyr Ile Glu Leu Ile Glu Ile
        755                 760                 765
Ala Arg Asn Ser Thr Gln Asp Arg Ile Leu Glu Met Lys Val Met Glu
    770                 775                 780
Phe Phe Met Lys Val Tyr Gly Tyr Arg Gly Lys His Leu Gly Gly Ser
785                 790                 795                 800
Arg Lys Pro Asp Gly Ala Ile Tyr Thr Val Gly Ser Pro Ile Asp Tyr
                805                 810                 815
Gly Val Ile Val Asp Thr Lys Ala Tyr Ser Gly Gly Tyr Asn Leu Pro
            820                 825                 830

Ile Gly Gln Ala Asp Glu Met Gln Arg Tyr Val Glu Glu Asn Gln Thr
        835                 840                 845
Arg Asn Lys His Ile Asn Pro Asn Glu Trp Trp Lys Val Tyr Pro Ser
    850                 855                 860
Ser Val Thr Glu Phe Lys Phe Leu Phe Val Ser Gly His Phe Lys Gly
865                 870                 875                 880
Asn Tyr Lys Ala Gln Leu Thr Arg Leu Asn His Ile Thr Asn Cys Asn
                885                 890                 895
Gly Ala Val Leu Ser Val Glu Glu Leu Leu Ile Gly Gly Glu Met Ile
            900                 905                 910
Lys Ala Gly Thr Leu Thr Leu Glu Glu Val Arg Arg Lys Phe Asn Asn
        915                 920                 925
Gly Glu Ile Asn Phe
    930

<210> 16
<211> 1001
<212> PRT
<213> Artificial Sequence

<220>
<223> Synthetic Polypeptide

<400> 16
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser Asn Gly Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser Asn Asn Gly
    210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser His
                245                 250                 255
Asp Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser His Asp Gly Gly Lys Gln Ala Leu Glu Thr Val Gln Arg Leu Leu
    290                 295                 300

Pro Val Leu Cys Gln Asp His Gly Leu Thr Pro Asp Gln Val Val Ala
305                 310                 315                 320
Ile Ala Ser His Asp Gly Gly Lys Gln Ala Leu Glu Thr Val Gln Arg
                325                 330                 335
Leu Leu Pro Val Leu Cys Gln Asp His Gly Leu Thr Pro Asp Gln Val
            340                 345                 350
Val Ala Ile Ala Ser His Asp Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser Asn Ile Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser His Asp Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser Asn Ile Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser Asn Asn Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser Asn
        515                 520                 525
Asn Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser Asn Asn Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser His Asp Gly Gly Lys Gln Ala Leu Glu Thr Val Gln Arg
        595                 600                 605
Leu Leu Pro Val Leu Cys Gln Asp His Gly Leu Thr Pro Asp Gln Val
    610                 615                 620
Val Ala Ile Ala Ser Asn Ile Gly Gly Lys Gln Ala Leu Glu Thr Val
625                 630                 635                 640
Gln Arg Leu Leu Pro Val Leu Cys Gln Asp His Gly Leu Thr Pro Asp
                645                 650                 655
Gln Val Val Ala Ile Ala Ser Asn Asn Gly Gly Lys Gln Ala Leu Glu
            660                 665                 670
Thr Val Gln Arg Leu Leu Pro Val Leu Cys Gln Asp His Gly Leu Thr
        675                 680                 685
Pro Asp Gln Val Val Ala Ile Ala Ser Asn Gly Gly Gly Lys Gln Ala
    690                 695                 700
Leu Glu Thr Val Gln Arg Leu Leu Pro Val Leu Cys Gln Asp His Gly
705                 710                 715                 720
Leu Thr Pro Asp Gln Val Val Ala Ile Ala Ser Asn Ile Gly Gly Lys
                725                 730                 735
Gln Ala Leu Glu Ser Ile Val Ala Gln Leu Ser Arg Pro Asp Pro Ala
            740                 745                 750
Leu Ala Ala Leu Thr Asn Asp His Leu Val Ala Leu Ala Cys Leu Gly
        755                 760                 765
Gly Arg Pro Ala Leu Asp Ala Val Lys Lys Gly Leu Pro His Ala Pro
    770                 775                 780
Ala Leu Ile Lys Arg Thr Asn Arg Arg Ile Pro Glu Arg Thr Ser His
785                 790                 795                 800
Arg Val Ala Gly Ser Gln Leu Val Lys Ser Glu Leu Glu Glu Lys Lys

805                 810                 815
Ser Glu Leu Arg His Lys Leu Lys Tyr Val Pro His Glu Tyr Ile Glu
            820                 825                 830
Leu Ile Glu Ile Ala Arg Asn Ser Thr Gln Asp Arg Ile Leu Glu Met
        835                 840                 845
Lys Val Met Glu Phe Phe Met Lys Val Tyr Gly Tyr Arg Gly Lys His
    850                 855                 860
Leu Gly Gly Ser Arg Lys Pro Asp Gly Ala Ile Tyr Thr Val Gly Ser
865                 870                 875                 880
Pro Ile Asp Tyr Gly Val Ile Val Asp Thr Lys Ala Tyr Ser Gly Gly
                885                 890                 895
Tyr Asn Leu Pro Ile Gly Gln Ala Asp Glu Met Gln Arg Tyr Val Glu
            900                 905                 910
Glu Asn Gln Thr Arg Asn Lys His Ile Asn Pro Asn Glu Trp Trp Lys
        915                 920                 925
Val Tyr Pro Ser Ser Val Thr Glu Phe Lys Phe Leu Phe Val Ser Gly
    930                 935                 940
His Phe Lys Gly Asn Tyr Lys Ala Gln Leu Thr Arg Leu Asn His Ile
945                 950                 955                 960
Thr Asn Cys Asn Gly Ala Val Leu Ser Val Glu Glu Leu Leu Ile Gly
                965                 970                 975
Gly Glu Met Ile Lys Ala Gly Thr Leu Thr Leu Glu Glu Val Arg Arg
            980                 985                 990
Lys Phe Asn Asn Gly Glu Ile Asn Phe
        995                 1000

<210> 17
<211> 1001
<212> PRT
<213> Artificial Sequence

<220>
<223> Synthetic Polypeptide

<400> 17
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser Asn Ile Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser Asn Gly Gly

210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser His
                245                 250                 255
Asp Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser Asn Ile Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser Asn Ile Gly Gly Lys Gln Ala Leu Glu Thr Val Gln Arg
                325                 330                 335
Leu Leu Pro Val Leu Cys Gln Asp His Gly Leu Thr Pro Asp Gln Val
            340                 345                 350
Val Ala Ile Ala Ser Asn Gly Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser Asn Ile Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser Asn Ile Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser Asn Asn Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser His Asp Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser Asn
        515                 520                 525
Gly Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser His Asp Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser His Asp Gly Gly Lys Gln Ala Leu Glu Thr Val Gln Arg
        595                 600                 605
Leu Leu Pro Val Leu Cys Gln Asp His Gly Leu Thr Pro Asp Gln Val
    610                 615                 620
Val Ala Ile Ala Ser Asn Gly Gly Gly Lys Gln Ala Leu Glu Thr Val
625                 630                 635                 640
Gln Arg Leu Leu Pro Val Leu Cys Gln Asp His Gly Leu Thr Pro Asp
                645                 650                 655
Gln Val Val Ala Ile Ala Ser Asn Ile Gly Gly Lys Gln Ala Leu Glu
            660                 665                 670
Thr Val Gln Arg Leu Leu Pro Val Leu Cys Gln Asp His Gly Leu Thr
        675                 680                 685
Pro Asp Gln Val Val Ala Ile Ala Ser Asn Asn Gly Gly Lys Gln Ala
    690                 695                 700
Leu Glu Thr Val Gln Arg Leu Leu Pro Val Leu Cys Gln Asp His Gly
705                 710                 715                 720

Leu Thr Pro Asp Gln Val Val Ala Ile Ala Ser Asn Gly Gly Gly Lys
                725                 730                 735
Gln Ala Leu Glu Ser Ile Val Ala Gln Leu Ser Arg Pro Asp Pro Ala
            740                 745                 750
Leu Ala Ala Leu Thr Asn Asp His Leu Val Ala Leu Ala Cys Leu Gly
        755                 760                 765
Gly Arg Pro Ala Leu Asp Ala Val Lys Lys Gly Leu Pro His Ala Pro
    770                 775                 780
Ala Leu Ile Lys Arg Thr Asn Arg Arg Ile Pro Glu Arg Thr Ser His
785                 790                 795                 800
Arg Val Ala Gly Ser Gln Leu Val Lys Ser Glu Leu Glu Glu Lys Lys
                805                 810                 815
Ser Glu Leu Arg His Lys Leu Lys Tyr Val Pro His Glu Tyr Ile Glu
            820                 825                 830
Leu Ile Glu Ile Ala Arg Asn Ser Thr Gln Asp Arg Ile Leu Glu Met
        835                 840                 845
Lys Val Met Glu Phe Phe Met Lys Val Tyr Gly Tyr Arg Gly Lys His
    850                 855                 860
Leu Gly Gly Ser Arg Lys Pro Asp Gly Ala Ile Tyr Thr Val Gly Ser
865                 870                 875                 880
Pro Ile Asp Tyr Gly Val Ile Val Asp Thr Lys Ala Tyr Ser Gly Gly
                885                 890                 895
Tyr Asn Leu Pro Ile Gly Gln Ala Asp Glu Met Gln Arg Tyr Val Glu
            900                 905                 910
Glu Asn Gln Thr Arg Asn Lys His Ile Asn Pro Asn Glu Trp Trp Lys
        915                 920                 925
Val Tyr Pro Ser Ser Val Thr Glu Phe Lys Phe Leu Phe Val Ser Gly
    930                 935                 940
His Phe Lys Gly Asn Tyr Lys Ala Gln Leu Thr Arg Leu Asn His Ile
945                 950                 955                 960
Thr Asn Cys Asn Gly Ala Val Leu Ser Val Glu Glu Leu Leu Ile Gly
                965                 970                 975
Gly Glu Met Ile Lys Ala Gly Thr Leu Thr Leu Glu Glu Val Arg Arg
            980                 985                 990
Lys Phe Asn Asn Gly Glu Ile Asn Phe
        995                 1000

<210> 18
<211> 933
<212> PRT
<213> Artificial Sequence

<220>
<223> Synthetic Polypeptide

<400> 18
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125

Leu Leu Thr Val Ala Gly Glu Leu Arg Gly Pro Pro Leu Gln Leu Asp
        130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser His Asp Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser His Asp Gly
    210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser Asn
                245                 250                 255
Gly Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser His Asp Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser His Asp Gly Gly Lys Gln Ala Leu Glu Thr Val Gln Arg
                325                 330                 335
Leu Leu Pro Val Leu Cys Gln Asp His Gly Leu Thr Pro Asp Gln Val
            340                 345                 350
Val Ala Ile Ala Ser Asn Gly Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser His Asp Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser Asn Gly Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser Asn Asn Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser Asn Gly Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser Asn
        515                 520                 525
Asn Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser Asn Ile Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser Asn Ile Gly Gly Lys Gln Ala Leu Glu Thr Val Gln Arg
        595                 600                 605
Leu Leu Pro Val Leu Cys Gln Asp His Gly Leu Thr Pro Asp Gln Val
    610                 615                 620
Val Ala Ile Ala Ser Asn Ile Gly Gly Lys Gln Ala Leu Glu Thr Val

625                 630                 635                 640
Gln Arg Leu Leu Pro Val Leu Cys Gln Asp His Gly Leu Thr Pro Asp
                645                 650                 655
Gln Val Val Ala Ile Ala Ser Asn Gly Gly Gly Lys Gln Ala Leu Glu
            660                 665                 670
Ser Ile Val Ala Gln Leu Ser Arg Pro Asp Pro Ala Leu Ala Ala Leu
        675                 680                 685
Thr Asn Asp His Leu Val Ala Leu Ala Cys Leu Gly Gly Arg Pro Ala
    690                 695                 700
Leu Asp Ala Val Lys Lys Gly Leu Pro His Ala Pro Ala Leu Ile Lys
705                 710                 715                 720
Arg Thr Asn Arg Arg Ile Pro Glu Arg Thr Ser His Arg Val Ala Gly
                725                 730                 735
Ser Gln Leu Val Lys Ser Glu Leu Glu Glu Lys Lys Ser Glu Leu Arg
            740                 745                 750
His Lys Leu Lys Tyr Val Pro His Glu Tyr Ile Glu Leu Ile Glu Ile
        755                 760                 765
Ala Arg Asn Ser Thr Gln Asp Arg Ile Leu Glu Met Lys Val Met Glu
    770                 775                 780
Phe Phe Met Lys Val Tyr Gly Tyr Arg Gly Lys His Leu Gly Gly Ser
785                 790                 795                 800
Arg Lys Pro Asp Gly Ala Ile Tyr Thr Val Gly Ser Pro Ile Asp Tyr
                805                 810                 815
Gly Val Ile Val Asp Thr Lys Ala Tyr Ser Gly Gly Tyr Asn Leu Pro
            820                 825                 830
Ile Gly Gln Ala Asp Glu Met Gln Arg Tyr Val Glu Glu Asn Gln Thr
        835                 840                 845
Arg Asn Lys His Ile Asn Pro Asn Glu Trp Trp Lys Val Tyr Pro Ser
    850                 855                 860
Ser Val Thr Glu Phe Lys Phe Leu Phe Val Ser Gly His Phe Lys Gly
865                 870                 875                 880
Asn Tyr Lys Ala Gln Leu Thr Arg Leu Asn His Ile Thr Asn Cys Asn
                885                 890                 895
Gly Ala Val Leu Ser Val Glu Glu Leu Leu Ile Gly Gly Glu Met Ile
            900                 905                 910
Lys Ala Gly Thr Leu Thr Leu Glu Glu Val Arg Arg Lys Phe Asn Asn
        915                 920                 925
Gly Glu Ile Asn Phe
    930

<210> 19
<211> 1103
<212> PRT
<213> Artificial Sequence

<220>
<223> Synthetic Polypeptide

<400> 19
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala

100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser Asn Asn Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser Asn Asn Gly
    210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser His
                245                 250                 255
Asp Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser His Asp Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser Asn Asn Gly Gly Lys Gln Ala Leu Glu Thr Val Gln Arg
                325                 330                 335
Leu Leu Pro Val Leu Cys Gln Asp His Gly Leu Thr Pro Asp Gln Val
            340                 345                 350
Val Ala Ile Ala Ser Asn Asn Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser His Asp Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser His Asp Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser Asn Asn Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser His Asp Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser Asn
        515                 520                 525
Asn Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser Asn Asn Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser His Asp Gly Gly Lys Gln Ala Leu Glu Thr Val Gln Arg
        595                 600                 605

Leu Leu Pro Val Leu Cys Gln Asp His Gly Leu Thr Pro Asp Gln Val
    610                 615                 620
Val Ala Ile Ala Ser His Asp Gly Gly Lys Gln Ala Leu Glu Thr Val
625                 630                 635                 640
Gln Arg Leu Leu Pro Val Leu Cys Gln Asp His Gly Leu Thr Pro Asp
                645                 650                 655
Gln Val Val Ala Ile Ala Ser Asn Ile Gly Gly Lys Gln Ala Leu Glu
            660                 665                 670
Thr Val Gln Arg Leu Leu Pro Val Leu Cys Gln Asp His Gly Leu Thr
        675                 680                 685
Pro Asp Gln Val Val Ala Ile Ala Ser His Asp Gly Gly Lys Gln Ala
    690                 695                 700
Leu Glu Thr Val Gln Arg Leu Leu Pro Val Leu Cys Gln Asp His Gly
705                 710                 715                 720
Leu Thr Pro Asp Gln Val Val Ala Ile Ala Ser His Asp Gly Gly Lys
                725                 730                 735
Gln Ala Leu Glu Thr Val Gln Arg Leu Leu Pro Val Leu Cys Gln Asp
            740                 745                 750
His Gly Leu Thr Pro Asp Gln Val Val Ala Ile Ala Ser Asn Asn Gly
        755                 760                 765
Gly Lys Gln Ala Leu Glu Thr Val Gln Arg Leu Leu Pro Val Leu Cys
    770                 775                 780
Gln Asp His Gly Leu Thr Pro Asp Gln Val Val Ala Ile Ala Ser His
785                 790                 795                 800
Asp Gly Gly Lys Gln Ala Leu Glu Thr Val Gln Arg Leu Leu Pro Val
                805                 810                 815
Leu Cys Gln Asp His Gly Leu Thr Pro Asp Gln Val Val Ala Ile Ala
            820                 825                 830
Ser Asn Gly Gly Gly Lys Gln Ala Leu Glu Ser Ile Val Ala Gln Leu
        835                 840                 845
Ser Arg Pro Asp Pro Ala Leu Ala Ala Leu Thr Asn Asp His Leu Val
    850                 855                 860
Ala Leu Ala Cys Leu Gly Gly Arg Pro Ala Leu Asp Ala Val Lys Lys
865                 870                 875                 880
Gly Leu Pro His Ala Pro Ala Leu Ile Lys Arg Thr Asn Arg Arg Ile
                885                 890                 895
Pro Glu Arg Thr Ser His Arg Val Ala Gly Ser Gln Leu Val Lys Ser
            900                 905                 910
Glu Leu Glu Glu Lys Lys Ser Glu Leu Arg His Lys Leu Lys Tyr Val
        915                 920                 925
Pro His Glu Tyr Ile Glu Leu Ile Glu Ile Ala Arg Asn Ser Thr Gln
    930                 935                 940
Asp Arg Ile Leu Glu Met Lys Val Met Glu Phe Phe Met Lys Val Tyr
945                 950                 955                 960
Gly Tyr Arg Gly Lys His Leu Gly Gly Ser Arg Lys Pro Asp Gly Ala
                965                 970                 975
Ile Tyr Thr Val Gly Ser Pro Ile Asp Tyr Gly Val Ile Val Asp Thr
            980                 985                 990
Lys Ala Tyr Ser Gly Gly Tyr Asn Leu Pro Ile Gly Gln Ala Asp Glu
        995                 1000                1005
Met Gln Arg Tyr Val Glu Glu Asn Gln Thr Arg Asn Lys His Ile Asn
    1010                1015                1020
Pro Asn Glu Trp Trp Lys Val Tyr Pro Ser Ser Val Thr Glu Phe Lys
1025                1030                1035                1040
Phe Leu Phe Val Ser Gly His Phe Lys Gly Asn Tyr Lys Ala Gln Leu
                1045                1050                1055
Thr Arg Leu Asn His Ile Thr Asn Cys Asn Gly Ala Val Leu Ser Val
            1060                1065                1070
Glu Glu Leu Leu Ile Gly Gly Glu Met Ile Lys Ala Gly Thr Leu Thr
        1075                1080                1085
Leu Glu Glu Val Arg Arg Lys Phe Asn Asn Gly Glu Ile Asn Phe
    1090                1095                1100

<210> 20
<211> 1001
<212> PRT
<213> Artificial Sequence

<220>
<223> Synthetic Polypeptide

<400> 20
   Met Asp Tyr Lys Asp His Asp Gly Asp Tyr Lys Asp His Asp Ile Asp
   1               5                   10                  15
   Tyr Lys Asp Asp Asp Asp Lys Met Ala Pro Lys Lys Lys Arg Lys Val
               20                  25                  30
   Gly Ile His Arg Gly Val Pro Met Val Asp Leu Arg Thr Leu Gly Tyr
           35                  40                  45
   Ser Gln Gln Gln Gln Glu Lys Ile Lys Pro Lys Val Arg Ser Thr Val
       50                  55                  60
   Ala Gln His His Glu Ala Leu Val Gly His Gly Phe Thr His Ala His
   65                  70                  75                  80
   Ile Val Ala Leu Ser Gln His Pro Ala Ala Leu Gly Thr Val Ala Val
                   85                  90                  95
   Lys Tyr Gln Asp Met Ile Ala Ala Leu Pro Glu Ala Thr His Glu Ala
               100                 105                 110
   Ile Val Gly Val Gly Lys Gln Trp Ser Gly Ala Arg Ala Leu Glu Ala
           115                 120                 125
   Leu Leu Thr Val Ala Gly Glu Leu Arg Gly Pro Pro Leu Gln Leu Asp
       130                 135                 140
   Thr Gly Gln Leu Leu Lys Ile Ala Lys Arg Gly Gly Val Thr Ala Val
   145                 150                 155                 160
   Glu Ala Val His Ala Trp Arg Asn Ala Leu Thr Gly Ala Pro Leu Asn
                   165                 170                 175
   Leu Thr Pro Asp Gln Val Val Ala Ile Ala Ser Asn Asn Gly Gly Lys
               180                 185                 190
   Gln Ala Leu Glu Thr Val Gln Arg Leu Leu Pro Val Leu Cys Gln Asp
           195                 200                 205
   His Gly Leu Thr Pro Asp Gln Val Val Ala Ile Ala Ser Asn Asn Gly
       210                 215                 220
   Gly Lys Gln Ala Leu Glu Thr Val Gln Arg Leu Leu Pro Val Leu Cys
   225                 230                 235                 240
   Gln Asp His Gly Leu Thr Pro Asp Gln Val Val Ala Ile Ala Ser His
                   245                 250                 255
   Asp Gly Gly Lys Gln Ala Leu Glu Thr Val Gln Arg Leu Leu Pro Val
               260                 265                 270
   Leu Cys Gln Asp His Gly Leu Thr Pro Asp Gln Val Val Ala Ile Ala
           275                 280                 285
   Ser His Asp Gly Gly Lys Gln Ala Leu Glu Thr Val Gln Arg Leu Leu
       290                 295                 300
   Pro Val Leu Cys Gln Asp His Gly Leu Thr Pro Asp Gln Val Val Ala
   305                 310                 315                 320
   Ile Ala Ser His Asp Gly Gly Lys Gln Ala Leu Glu Thr Val Gln Arg
                   325                 330                 335
   Leu Leu Pro Val Leu Cys Gln Asp His Gly Leu Thr Pro Asp Gln Val
               340                 345                 350
   Val Ala Ile Ala Ser Asn Ile Gly Gly Lys Gln Ala Leu Glu Thr Val
           355                 360                 365
   Gln Arg Leu Leu Pro Val Leu Cys Gln Asp His Gly Leu Thr Pro Asp
       370                 375                 380
   Gln Val Val Ala Ile Ala Ser Asn Asn Gly Gly Lys Gln Ala Leu Glu
   385                 390                 395                 400
   Thr Val Gln Arg Leu Leu Pro Val Leu Cys Gln Asp His Gly Leu Thr
                   405                 410                 415
   Pro Asp Gln Val Val Ala Ile Ala Ser Asn Ile Gly Gly Lys Gln Ala
   420 425 430
   Leu Glu Thr Val Gln Arg Leu Leu Pro Val Leu Cys Gln Asp His Gly
   435 440 445
   Leu Thr Pro Asp Gln Val Val Ala Ile Ala Ser His Asp Gly Gly Lys
   450 455 460
   Gln Ala Leu Glu Thr Val Gln Arg Leu Leu Pro Val Leu Cys Gln Asp
   465 470 475 480
   His Gly Leu Thr Pro Asp Gln Val Val Ala Ile Ala Ser Asn Asn Gly
   485 490 495
   Gly Lys Gln Ala Leu Glu Thr Val Gln Arg Leu Leu Pro Val Leu Cys
   500 505 510
   Gln Asp His Gly Leu Thr Pro Asp Gln Val Val Ala Ile Ala Ser His
   515 520 525
   Asp Gly Gly Lys Gln Ala Leu Glu Thr Val Gln Arg Leu Leu Pro Val
   530 535 540
   Leu Cys Gln Asp His Gly Leu Thr Pro Asp Gln Val Val Ala Ile Ala
   545 550 555 560
   Ser His Asp Gly Gly Lys Gln Ala Leu Glu Thr Val Gln Arg Leu Leu
   565 570 575
   Pro Val Leu Cys Gln Asp His Gly Leu Thr Pro Asp Gln Val Val Ala
   580 585 590
   Ile Ala Ser His Asp Gly Gly Lys Gln Ala Leu Glu Thr Val Gln Arg
   595 600 605
   Leu Leu Pro Val Leu Cys Gln Asp His Gly Leu Thr Pro Asp Gln Val
   610 615 620
   Val Ala Ile Ala Ser Asn Asn Gly Gly Lys Gln Ala Leu Glu Thr Val
   625 630 635 640
   Gln Arg Leu Leu Pro Val Leu Cys Gln Asp His Gly Leu Thr Pro Asp
   645 650 655
   Gln Val Val Ala Ile Ala Ser His Asp Gly Gly Lys Gln Ala Leu Glu
   660 665 670
   Thr Val Gln Arg Leu Leu Pro Val Leu Cys Gln Asp His Gly Leu Thr
   675 680 685
   Pro Asp Gln Val Val Ala Ile Ala Ser His Asp Gly Gly Lys Gln Ala
   690 695 700
   Leu Glu Thr Val Gln Arg Leu Leu Pro Val Leu Cys Gln Asp His Gly
   705 710 715 720
   Leu Thr Pro Asp Gln Val Val Ala Ile Ala Ser Asn Gly Gly Gly Lys
   725 730 735
   Gln Ala Leu Glu Ser Ile Val Ala Gln Leu Ser Arg Pro Asp Pro Ala
   740 745 750
   Leu Ala Ala Leu Thr Asn Asp His Leu Val Ala Leu Ala Cys Leu Gly
   755 760 765
   Gly Arg Pro Ala Leu Asp Ala Val Lys Lys Gly Leu Pro His Ala Pro
   770 775 780
   Ala Leu Ile Lys Arg Thr Asn Arg Arg Ile Pro Glu Arg Thr Ser His
   785 790 795 800
   Arg Val Ala Gly Ser Gln Leu Val Lys Ser Glu Leu Glu Glu Lys Lys
   805 810 815
   Ser Glu Leu Arg His Lys Leu Lys Tyr Val Pro His Glu Tyr Ile Glu
   820 825 830
   Leu Ile Glu Ile Ala Arg Asn Ser Thr Gln Asp Arg Ile Leu Glu Met
   835 840 845
   Lys Val Met Glu Phe Phe Met Lys Val Tyr Gly Tyr Arg Gly Lys His
   850 855 860
   Leu Gly Gly Ser Arg Lys Pro Asp Gly Ala Ile Tyr Thr Val Gly Ser
   865 870 875 880
   Pro Ile Asp Tyr Gly Val Ile Val Asp Thr Lys Ala Tyr Ser Gly Gly
   885 890 895
   Tyr Asn Leu Pro Ile Gly Gln Ala Asp Glu Met Gln Arg Tyr Val Glu
   900 905 910
   Glu Asn Gln Thr Arg Asn Lys His Ile Asn Pro Asn Glu Trp Trp Lys
   915 920 925
   Val Tyr Pro Ser Ser Val Thr Glu Phe Lys Phe Leu Phe Val Ser Gly
       930                 935                 940
   His Phe Lys Gly Asn Tyr Lys Ala Gln Leu Thr Arg Leu Asn His Ile
   945                 950                 955                 960
   Thr Asn Cys Asn Gly Ala Val Leu Ser Val Glu Glu Leu Leu Ile Gly
                   965                 970                 975
   Gly Glu Met Ile Lys Ala Gly Thr Leu Thr Leu Glu Glu Val Arg Arg
               980                 985                 990
   Lys Phe Asn Asn Gly Glu Ile Asn Phe
           995                 1000

<210> 21
<211> 933
<212> PRT
<213> Artificial Sequence

<220>
<223> Synthetic Polypeptide

<400> 21
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser His Asp Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser Asn Gly Gly
    210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser His
                245                 250                 255
Asp Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser Asn Gly Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala
305                 310                 315                 320
Ile Ala Ser Asn Asn Gly Gly Lys Gln Ala Leu Glu Thr Val Gln Arg
                325                 330                 335

Leu Leu Pro Val Leu Cys Gln Asp His Gly Leu Thr Pro Asp Gln Val
            340                 345                 350
Val Ala Ile Ala Ser His Asp Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser His Asp Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser Asn Asn Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser Asn Asn Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser His Asp Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser Asn
        515                 520                 525
Gly Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser His Asp Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser Asn Asn Gly Gly Lys Gln Ala Leu Glu Thr Val Gln Arg
        595                 600                 605
Leu Leu Pro Val Leu Cys Gln Asp His Gly Leu Thr Pro Asp Gln Val
    610                 615                 620
Val Ala Ile Ala Ser Asn Asn Gly Gly Lys Gln Ala Leu Glu Thr Val
625                 630                 635                 640
Gln Arg Leu Leu Pro Val Leu Cys Gln Asp His Gly Leu Thr Pro Asp
                645                 650                 655
Gln Val Val Ala Ile Ala Ser Asn Gly Gly Gly Lys Gln Ala Leu Glu
            660                 665                 670
Ser Ile Val Ala Gln Leu Ser Arg Pro Asp Pro Ala Leu Ala Ala Leu
        675                 680                 685
Thr Asn Asp His Leu Val Ala Leu Ala Cys Leu Gly Gly Arg Pro Ala
    690                 695                 700
Leu Asp Ala Val Lys Lys Gly Leu Pro His Ala Pro Ala Leu Ile Lys
705                 710                 715                 720
Arg Thr Asn Arg Arg Ile Pro Glu Arg Thr Ser His Arg Val Ala Gly
                725                 730                 735
Ser Gln Leu Val Lys Ser Glu Leu Glu Glu Lys Lys Ser Glu Leu Arg
            740                 745                 750
His Lys Leu Lys Tyr Val Pro His Glu Tyr Ile Glu Leu Ile Glu Ile
        755                 760                 765
Ala Arg Asn Ser Thr Gln Asp Arg Ile Leu Glu Met Lys Val Met Glu
    770                 775                 780
Phe Phe Met Lys Val Tyr Gly Tyr Arg Gly Lys His Leu Gly Gly Ser
785                 790                 795                 800
Arg Lys Pro Asp Gly Ala Ile Tyr Thr Val Gly Ser Pro Ile Asp Tyr
                805                 810                 815
Gly Val Ile Val Asp Thr Lys Ala Tyr Ser Gly Gly Tyr Asn Leu Pro
            820                 825                 830
Ile Gly Gln Ala Asp Glu Met Gln Arg Tyr Val Glu Glu Asn Gln Thr

835                 840                 845
Arg Asn Lys His Ile Asn Pro Asn Glu Trp Trp Lys Val Tyr Pro Ser
    850                 855                 860
Ser Val Thr Glu Phe Lys Phe Leu Phe Val Ser Gly His Phe Lys Gly
865                 870                 875                 880
Asn Tyr Lys Ala Gln Leu Thr Arg Leu Asn His Ile Thr Asn Cys Asn
                885                 890                 895
Gly Ala Val Leu Ser Val Glu Glu Leu Leu Ile Gly Gly Glu Met Ile
            900                 905                 910
Lys Ala Gly Thr Leu Thr Leu Glu Glu Val Arg Arg Lys Phe Asn Asn
        915                 920                 925
Gly Glu Ile Asn Phe
    930

<210> 22
<211> 1171
<212> PRT
<213> Artificial Sequence

<220>
<223> Synthetic Polypeptide

<400> 22
Met Asp Tyr Lys Asp His Asp Gly Asp Tyr Lys Asp His Asp Ile Asp
1               5                   10                  15
Tyr Lys Asp Asp Asp Asp Lys Met Ala Pro Lys Lys Lys Arg Lys Val
            20                  25                  30
Gly Ile His Arg Gly Val Pro Met Val Asp Leu Arg Thr Leu Gly Tyr
        35                  40                  45
Ser Gln Gln Gln Gln Glu Lys Ile Lys Pro Lys Val Arg Ser Thr Val
    50                  55                  60
Ala Gln His His Glu Ala Leu Val Gly His Gly Phe Thr His Ala His
65                  70                  75                  80
Ile Val Ala Leu Ser Gln His Pro Ala Ala Leu Gly Thr Val Ala Val
                85                  90                  95
Lys Tyr Gln Asp Met Ile Ala Ala Leu Pro Glu Ala Thr His Glu Ala
            100                 105                 110
Ile Val Gly Val Gly Lys Gln Trp Ser Gly Ala Arg Ala Leu Glu Ala
        115                 120                 125
Leu Leu Thr Val Ala Gly Glu Leu Arg Gly Pro Pro Leu Gln Leu Asp
    130                 135                 140
Thr Gly Gln Leu Leu Lys Ile Ala Lys Arg Gly Gly Val Thr Ala Val
145                 150                 155                 160
Glu Ala Val His Ala Trp Arg Asn Ala Leu Thr Gly Ala Pro Leu Asn
                165                 170                 175
Leu Thr Pro Asp Gln Val Val Ala Ile Ala Ser His Asp Gly Gly Lys
            180                 185                 190
Gln Ala Leu Glu Thr Val Gln Arg Leu Leu Pro Val Leu Cys Gln Asp
        195                 200                 205
His Gly Leu Thr Pro Asp Gln Val Val Ala Ile Ala Ser His Asp Gly
    210                 215                 220
Gly Lys Gln Ala Leu Glu Thr Val Gln Arg Leu Leu Pro Val Leu Cys
225                 230                 235                 240
Gln Asp His Gly Leu Thr Pro Asp Gln Val Val Ala Ile Ala Ser His
                245                 250                 255
Asp Gly Gly Lys Gln Ala Leu Glu Thr Val Gln Arg Leu Leu Pro Val
            260                 265                 270
Leu Cys Gln Asp His Gly Leu Thr Pro Asp Gln Val Val Ala Ile Ala
        275                 280                 285
Ser His Asp Gly Gly Lys Gln Ala Leu Glu Thr Val Gln Arg Leu Leu
    290                 295                 300
Pro Val Leu Cys Gln Asp His Gly Leu Thr Pro Asp Gln Val Val Ala

305                 310                 315                 320
Ile Ala Ser Asn Asn Gly Gly Lys Gln Ala Leu Glu Thr Val Gln Arg
                325                 330                 335
Leu Leu Pro Val Leu Cys Gln Asp His Gly Leu Thr Pro Asp Gln Val
            340                 345                 350
Val Ala Ile Ala Ser Asn Asn Gly Gly Lys Gln Ala Leu Glu Thr Val
        355                 360                 365
Gln Arg Leu Leu Pro Val Leu Cys Gln Asp His Gly Leu Thr Pro Asp
    370                 375                 380
Gln Val Val Ala Ile Ala Ser Asn Ile Gly Gly Lys Gln Ala Leu Glu
385                 390                 395                 400
Thr Val Gln Arg Leu Leu Pro Val Leu Cys Gln Asp His Gly Leu Thr
                405                 410                 415
Pro Asp Gln Val Val Ala Ile Ala Ser Asn Asn Gly Gly Lys Gln Ala
            420                 425                 430
Leu Glu Thr Val Gln Arg Leu Leu Pro Val Leu Cys Gln Asp His Gly
        435                 440                 445
Leu Thr Pro Asp Gln Val Val Ala Ile Ala Ser His Asp Gly Gly Lys
    450                 455                 460
Gln Ala Leu Glu Thr Val Gln Arg Leu Leu Pro Val Leu Cys Gln Asp
465                 470                 475                 480
His Gly Leu Thr Pro Asp Gln Val Val Ala Ile Ala Ser His Asp Gly
                485                 490                 495
Gly Lys Gln Ala Leu Glu Thr Val Gln Arg Leu Leu Pro Val Leu Cys
            500                 505                 510
Gln Asp His Gly Leu Thr Pro Asp Gln Val Val Ala Ile Ala Ser His
        515                 520                 525
Asp Gly Gly Lys Gln Ala Leu Glu Thr Val Gln Arg Leu Leu Pro Val
    530                 535                 540
Leu Cys Gln Asp His Gly Leu Thr Pro Asp Gln Val Val Ala Ile Ala
545                 550                 555                 560
Ser His Asp Gly Gly Lys Gln Ala Leu Glu Thr Val Gln Arg Leu Leu
                565                 570                 575
Pro Val Leu Cys Gln Asp His Gly Leu Thr Pro Asp Gln Val Val Ala
            580                 585                 590
Ile Ala Ser His Asp Gly Gly Lys Gln Ala Leu Glu Thr Val Gln Arg
        595                 600                 605
Leu Leu Pro Val Leu Cys Gln Asp His Gly Leu Thr Pro Asp Gln Val
    610                 615                 620
Val Ala Ile Ala Ser Asn Asn Gly Gly Lys Gln Ala Leu Glu Thr Val
625                 630                 635                 640
Gln Arg Leu Leu Pro Val Leu Cys Gln Asp His Gly Leu Thr Pro Asp
                645                 650                 655
Gln Val Val Ala Ile Ala Ser His Asp Gly Gly Lys Gln Ala Leu Glu
            660                 665                 670
Thr Val Gln Arg Leu Leu Pro Val Leu Cys Gln Asp His Gly Leu Thr
        675                 680                 685
Pro Asp Gln Val Val Ala Ile Ala Ser Asn Asn Gly Gly Lys Gln Ala
    690                 695                 700
Leu Glu Thr Val Gln Arg Leu Leu Pro Val Leu Cys Gln Asp His Gly
705                 710                 715                 720
Leu Thr Pro Asp Gln Val Val Ala Ile Ala Ser His Asp Gly Gly Lys
                725                 730                 735
Gln Ala Leu Glu Thr Val Gln Arg Leu Leu Pro Val Leu Cys Gln Asp
            740                 745                 750
His Gly Leu Thr Pro Asp Gln Val Val Ala Ile Ala Ser Asn Asn Gly
        755                 760                 765
Gly Lys Gln Ala Leu Glu Thr Val Gln Arg Leu Leu Pro Val Leu Cys
    770                 775                 780
Gln Asp His Gly Leu Thr Pro Asp Gln Val Val Ala Ile Ala Ser His
785                 790                 795                 800
Asp Gly Gly Lys Gln Ala Leu Glu Thr Val Gln Arg Leu Leu Pro Val
                805                 810                 815

Leu Cys Gln Asp His Gly Leu Thr Pro Asp Gln Val Val Ala Ile Ala
            820                 825                 830
Ser Asn Asn Gly Gly Lys Gln Ala Leu Glu Thr Val Gln Arg Leu Leu
        835                 840                 845
Pro Val Leu Cys Gln Asp His Gly Leu Thr Pro Asp Gln Val Val Ala
    850                 855                 860
Ile Ala Ser His Asp Gly Gly Lys Gln Ala Leu Glu Thr Val Gln Arg
865                 870                 875                 880
Leu Leu Pro Val Leu Cys Gln Asp His Gly Leu Thr Pro Asp Gln Val
                885                 890                 895
Val Ala Ile Ala Ser Asn Gly Gly Gly Lys Gln Ala Leu Glu Ser Ile
            900                 905                 910
Val Ala Gln Leu Ser Arg Pro Asp Pro Ala Leu Ala Ala Leu Thr Asn
        915                 920                 925
Asp His Leu Val Ala Leu Ala Cys Leu Gly Gly Arg Pro Ala Leu Asp
    930                 935                 940
Ala Val Lys Lys Gly Leu Pro His Ala Pro Ala Leu Ile Lys Arg Thr
945                 950                 955                 960
Asn Arg Arg Ile Pro Glu Arg Thr Ser His Arg Val Ala Gly Ser Gln
                965                 970                 975
Leu Val Lys Ser Glu Leu Glu Glu Lys Lys Ser Glu Leu Arg His Lys
            980                 985                 990
Leu Lys Tyr Val Pro His Glu Tyr Ile Glu Leu Ile Glu Ile Ala Arg
        995                 1000                1005
Asn Ser Thr Gln Asp Arg Ile Leu Glu Met Lys Val Met Glu Phe Phe
    1010                1015                1020
Met Lys Val Tyr Gly Tyr Arg Gly Lys His Leu Gly Gly Ser Arg Lys
1025                1030                1035                1040
Pro Asp Gly Ala Ile Tyr Thr Val Gly Ser Pro Ile Asp Tyr Gly Val
                1045                1050                1055
Ile Val Asp Thr Lys Ala Tyr Ser Gly Gly Tyr Asn Leu Pro Ile Gly
            1060                1065                1070
Gln Ala Asp Glu Met Gln Arg Tyr Val Glu Glu Asn Gln Thr Arg Asn
        1075                1080                1085
Lys His Ile Asn Pro Asn Glu Trp Trp Lys Val Tyr Pro Ser Ser Val
    1090                1095                1100
Thr Glu Phe Lys Phe Leu Phe Val Ser Gly His Phe Lys Gly Asn Tyr
1105                1110                1115                1120
Lys Ala Gln Leu Thr Arg Leu Asn His Ile Thr Asn Cys Asn Gly Ala
                1125                1130                1135
Val Leu Ser Val Glu Glu Leu Leu Ile Gly Gly Glu Met Ile Lys Ala
            1140                1145                1150
Gly Thr Leu Thr Leu Glu Glu Val Arg Arg Lys Phe Asn Asn Gly Glu
        1155                1160                1165
Ile Asn Phe
    1170

<210> 23
<211> 417
<212> DNA
<213> Artificial Sequence

<220>
<223> Synthetic Nucleic Acid

<400> 23
aaggacgacg gcaactacaa gacccgcgcc gaggtgaagt tcgagggcga caccctggtg 60
aaccgcatcg agctgaaggg catcgacttc aaggaggacg gcaacatcct ggggcacaag 120
ctggagtaca actacaacag ccacaacgtc tatatcatgg ccgacaagca gaagaacggc 180
atcaaggtga acttcaagat ccgccacaac atcgaggacg gcagcgtgca gctcgccgac 240
cactaccagc agaacacccc catcggcgac ggccccgtgc tgctgcccga caaccactac 300
ctgagcaccc agtccgccct gagcaaagac cccaacgaga agcgcgatca catggtcctg 360

ctggagttcg tgaccgccgc cgggatcact ctcggcatgg acgagctgta caagtaa 417

<210> 24
<211> 417
<212> DNA
<213> Artificial Sequence

<220>
<223> Synthetic Nucleic Acid

<400> 24
aaagatgatg gaaactataa gacacgcgct gaggtcaagt ttgagggaga cacactggtc 60
aaccggatcg aactgaaagg cattgacttt aaggaagacg gaaacattct gggccacaaa 120
ctggaataca attacaatag ccataacgtg tatattatgg ctgacaaaca gaaaaacgga 180
atcaaagtga atttcaaaat ccggcacaat atcgaagacg gaagcgtcca gctggccgat 240
cactatcagc aaaacacacc cattggcgat ggccctgtgc tcctgcctga caatcactat 300
ctgagtaccc aatccgctct gagtaaagat cccaatgaga aacgcgacca catggtcctc 360
ctggagttcg tcaccgctgc cggcatcacc ctcggaatgg atgagctcta caaataa 417

<210> 25
<211> 805
<212> DNA
<213> Artificial Sequence

<220>
<223> Synthetic Nucleic Acid

<400> 25
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccttcaccta cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacctaa gctctcgaat taccctgtta tccctactcg 360
atcgagtcta gctagaactt ccacagagtg ggttaaagcg gctccgaagc ttcgcgccga 420
ggtgaagttc gagggcgaca ccctggtgaa ccgcatcgag ctgaagggca tcgacttcaa 480
ggaggacggc aacatcctgg ggcacaagct ggagtacaac tacaacagcc acaacgtcta 540
tatcatggcc gacaagcaga agaacggcat caaggtgaac ttcaagatcc gccacaacat 600
cgaggacggc agcgtgcagc tcgccgacca ctaccagcag aacaccccca tcggcgacgg 660
ccccgtgctg ctgcccgaca accactacct gagcacccag tccgccctga gcaaagaccc 720
caacgagaag cgcgatcaca tggtcctgct ggagttcgtg accgccgccg ggatcactct 780
cggcatggac gagctgtaca agtaa 805

<210> 26
<211> 805
<212> DNA
<213> Artificial Sequence

<220>
<223> Synthetic Nucleic Acid

<400> 26
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccttcaccta cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacctaa gctctcgaat taccctgtta tccctactcg 360
atcgagtcta gctagaactt ccacagagtg ggttaaagcg gctccgaagc ttcgcgccga 420
ggtgaagttc gagggcgaca ccctggtgaa ccgcatcgag ctgaagggca tcgacttcaa 480
ggaggacggc aacatcctgg ggcacaagct ggagtacaac tacaacagcc acaacgtcta 540
tatcatggcc gacaagcaga agaacggcat caaggtgaac ttcaagatcc gccacaacat 600
cgaggacggc agcgtgcagc tcgccgacca ctaccagcag aacaccccca tcggcgacgg 660

ccccgtgctg ctgcccgaca accactacct gagcacccag tccgccctga gcaaagaccc 720
caacgagaag cgcgatcaca tggtcctgct ggagttcgtg accgccgccg ggatcactct 780
cggcatggac gagctgtaca agtaa 805